(19) Europäisches Patentamt
European Patent Office
Office européen des brevets

(11) EP 3 783 100 A1

(12) # EUROPEAN PATENT APPLICATION
published in accordance with Art. 153(4) EPC

(43) Date of publication:
**24.02.2021 Bulletin 2021/08**

(21) Application number: **19788373.9**

(22) Date of filing: **15.04.2019**

(51) Int Cl.:
***C12N 7/01*** (2006.01) ***A61P 35/00*** (2006.01)
***A61P 35/02*** (2006.01) ***A61P 35/04*** (2006.01)

(86) International application number:
**PCT/CN2019/082608**

(87) International publication number:
**WO 2019/201192 (24.10.2019 Gazette 2019/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.04.2018 CN 201810338046**

(71) Applicants:
- **Xiamen University**
  **Xiamen, Fujian Province, 361005 (CN)**
- **Yang Sheng Tang Company, Ltd.**
  **Zhejiang (CN)**

(72) Inventors:
- **CHENG, Tong**
  **Xiamen, Fujian 361005 (CN)**
- **WANG, Wei**
  **Xiamen, Fujian 361005 (CN)**
- **YE, Xiangzhong**
  **Xiamen, Fujian 361005 (CN)**
- **FU, Wenkun**
  **Xiamen, Fujian 361005 (CN)**
- **PAN, Dequan**
  **Xiamen, Fujian 361005 (CN)**
- **ZHANG, Jun**
  **Xiamen, Fujian 361005 (CN)**
- **XIA, Ningshao**
  **Xiamen, Fujian 361005 (CN)**

(74) Representative: **Held, Stephan**
**Meissner Bolte Patentanwälte**
**Rechtsanwälte Partnerschaft mbB**
**Widenmayerstraße 47**
**80538 München (DE)**

(54) **COXSACKIE VIRUS B FOR TREATING TUMORS**

(57) Provided are Coxsackie virus CVB1 or a modified form thereof, or a genomic sequence or cDNA sequence comprising CVB1 or the modified form thereof, or a nucleic acid molecule of a complement sequence of the genomic sequence or cDNA sequence, the use of same for treating tumors in subjects including humans, and the use of same in the preparation of a pharmaceutical composition for treating tumors in subjects including humans. Also provided is a method for treating tumors, comprising administering CVB1 or the modified form thereof, or the genomic sequence or cDNA sequence comprising CVB1 or the modified form thereof, or the nucleic acid molecule of the complement sequence of the genomic sequence or cDNA sequence to a subject in need thereof.

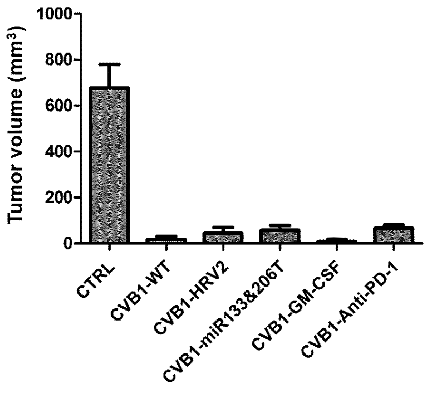


FIG. 5

EP 3 783 100 A1

SEQUENCE LISTING

<110> XIAMEN UNIVERSITY
YANG SHENG TANG COMPANY, LTD

<120> COXSACKIE VIRUS B FOR TREATING TUMORS

<130> IEC180049PEP

<140> PCT/CN2019/082608
<141> 2019-04-15

<150> CN 201810338046.2
<151> 2018-04-16

<160> 21

<170> PatentIn version 3.5

<210> 1
<211> 7371
<212> DNA
<213> Artificial Sequence

<220>
<223> cDNA sequence of CVB1-WT

<400> 1
tcctgtgggt tgatcccacc cacagggccc actgggcgct agcacactgg tattccggta 60

cctttgtgcg cctgttttat acacccttcc caagtgtaac ttagaagctt atcacacacg 120

gtcaacaggt gactcagtat gccaactggg tcttgatcaa gcacttctgt ttccccggac 180

tgagtatcaa taagctgctc acgcggctga aggagaaaac gttcgttaac cggccaatta 240

cttcgagaaa cctagtaaca ccatggaggt tgcgcagtgt ttcgctccac acaaccccag 300

tgtagatcag gccgatgagt caccgcactc ctcacgggcg accgtggcgg tggctgcgct 360

ggcggcctgc ccatgggata acccatggga cgcttcaata ctgacatggt gcgaagagtc 420

tattgagcta attggtagtc ctccggcccc tgaatgcggc taatcctaac tgcggagcag 480

atacccacac gccagtgggc agtctgtcgt aatgggcaac tctgcagcgg aaccgactac 540

tttgggtgtc cgtgtttcct tttatttta tactggctgc ttatggtgac aattgagaga 600

ttgttaccat atagctattg gattggccat ccagtgacaa acagagcgat tatttattta 660

tttgttggtt atatcccatt aagccaaaag gccctagtta ctctcaactt tatactattg 720

cttaatacaa cgaaatggga gctcaggtgt ctacacaaaa gacgggtgca catgagactg 780

gcttgaatgc cagcggcaat tctgtcatcc attacaccaa catcaattac tacaaggatg 840

ctgcatccaa ctccgcaaat aggcaagact tcacacaaga cccaggtaag ttcactgaac 900

ctgtaaagga catcatggtg aaaactatgc ctgcgttgaa ctcaccctct gctgaggaat 960

gcggctacag tgacagagtg cgttccatca cattaggtaa ttccactatc accacccaag 1020

agtgtgccaa cgtggtcgtc gggtacggag tgtggccaga gtatctaaaa gacaacgagg 1080

```
ccacagccga agatcagccc acacaacctg atgtggccac ctgccgcttc tatactttag      1140
aatcagtgca gtggatgaaa aattcggccg ggtggtggtg gaagctgcct gatgcactgt      1200
cacagatggg cttgtttggt caaaacatgc agtaccacta cttgggaaga acagggtata      1260
ctatacacgt acaatgcaat gcctcaaagt tccatcaagg atgcctgctt gtagtgtgtg      1320
tgcctgaagc tgagatgggg tgctctaact tgaacaacac gcccgaattc agtgagttat      1380
ccggaggaga cagtgccaga atgttcaccg atacacaggt cggggagtca aacgccaaga      1440
aagtacagac agcggtgtgg aacgccggaa tgggcgttgg agtgggtaat ctcactattt      1500
tccctcacca atggatcaac ctgaggacca acaacagtgc aacgctagtg atgccttaca      1560
taaacagcgt ccccatggat aacatgttca ggcacaataa cttaacacta atgattatcc      1620
catttgtacc gcttaattac agcgagggat cctcaccgta cgtgcccata acagtcacca      1680
tcgctcctat gtgtgcagag tacaacgggc tacggctggc cagcaaccag ggtctaccag      1740
ttatgacaac acctgggagc acgcaatttc taacttcaga tgacttccag tcaccgtcag      1800
cgatgccaca gtttgatgtc accccagaga tgcaaatacc aggccgcgta aataatttga      1860
tggagattgc cgaggtggac tcagtggtgc ctgtgaataa cacagaggac aacgtgagta      1920
gccttaaggc ataccagatt ccagtacagt ccaacagtga caatggtaag caggtttttg      1980
gttttccctt acaaccgggt gccaacaacg ttctgaatag aaccctcttg ggtgaaattc      2040
taaactatta cacccattgg agtggcagca taaaacttac attcatgttc tgtgggtctg      2100
ccatggccac tggcaagttc cttctggcat actcaccacc tggagcagga gtcccgaaaa      2160
accgaaaaga tgctatgctg ggcacccacg tcatatggga tgttgggtta caatcaagct      2220
gcgtgttgtg cgtaccgtgg attagccaaa cacactacag atacgtggtt gaggatgaat      2280
acacagcagc cggatatgtt acatgctggt atcaaacaaa tatcgtggtg ccagctgatg      2340
tgcaaagctc atgtgacatc ttgtgctttg tttcagcctg caacgatttc tctgtgcgga      2400
tgctgaaaga tacgcccttt ataagacagg acacatttta tcacggccca gtggaggagt      2460
ctgtggatcg tgcagtggca cgtgtggcgg acacgattag ttcacggccc actaactcgg      2520
agtcaattcc agcgctcacc gccgccgaga ctgggcatac ctcccaagtt gtgcctagcg      2580
acaccatgca aacaagacat gtgaaaaact accactcacg gtccgaatcg tccattgaaa      2640
atttcctatg ccggtctgcc tgtgtatact atgccacata caccaataac tcaaaaaaag      2700
aggggtttgc agagtgggtt atcaacacta gacaggtggc acagctaaga agaaagttgg      2760
agctcttcac atatctaagg tttgacttag aactaacatt tgttataacg agcgcacaac      2820
aacccagtac cgcctccagt gtagacgctc ccgtgcaaac ccatcagatt atgtatgtgc      2880
ctccaggtgg ccctgtacca acaaaggtta aagattatgc atggcaaact tccacaaacc      2940
```

ccagcgtttt ctggacagaa ggaaacgccc caccaaggat gtccatccca ttcattagca 3000
ttggtaatgc gtatagttgt ttttatgatg ggtggacgca gttttcaaga aacggggtct 3060
atggcatcaa taccctcaac aacatgggca cgttgtatat gaggcatgtc aatgaagcgg 3120
ggcagggtcc aatcaaaagt actgttagaa tatatttcaa acccaagcac gtgaaggcgt 3180
gggtgccacg tccgccaaga ttgtgtcaat atgagaagca gaaaaatgtc aattttagcc 3240
ctataggagt aaccacaagt agaacggaca ttataacaac aggcactttt ggtcagcagt 3300
ccggagcgat atatgtgggc aattacagga tagtcaatag gcacctggca acacactttg 3360
actggcaaaa ttgcatatgg gaggattaca acagggacct cctagtatgc actacaacgg 3420
cgcacggttg tgacaatatt gcaagatgcc agtgcacagc aggtgtgtat tattgtgctt 3480
ctaggaacaa gcattacccg gtgcattttg aaggaccagg cttggtggag gtccaggaaa 3540
gtgaatatta tccaaaaagg tatcaatcac acgtgcttct tgccgcaggg ttttctgagc 3600
cgggggattg tggtggtatt ctcaggtgtg aacatggtgt tataggcatc gtgaccatgg 3660
gtggtgaggg tgttgtcggc tttgccgatg tgcgcgatct cttatggctg gaagacgacg 3720
caatggaaca gggagtcagg gactacgtgg aacagcttgg aaacgccttt ggttctgggt 3780
ttaccaacca gatttgcgag caagtcaatc tgttaaaaga gtcactaata ggccaagact 3840
ccatcttgga aaaatcactc aaggcattag tgaaaatcat atcagcactg gttatcgtgg 3900
tgagaaacca cgatgacctc ataacagtca ctgccacctt agccctgatt ggctgtacca 3960
cttcaccgtg gcggtggcta aagaaaaaag tgactcaata ctatgggatt cccatggccg 4020
agagacagaa caatggctgg ctcaagaaat tcacagaaat gaccaatgca tgcaagggca 4080
tggaatggat cgccatcaaa atccaaaagt tcattgagtg gctcaaaatt aagatcttgc 4140
cagaagtaaa ggaaaaacac gagtttctta ccagacttaa gcaactccca ctcttagaaa 4200
gccaaattgc caccatcgag cagagtgcac catcacagag tgatcaggaa caactgttct 4260
ccaatataca gtacttcgcg cactattgca gaaagtacgc cccactgtac gcagctgagg 4320
caaaaagagt gttctctcta gaaaagaaaa tgagcaacta catacagttc aagtccaaat 4380
gccgtattga accagtctgt ttattgctcc atggtagccc tggagccgga aaatccgtgg 4440
ctaccaattt gattggacgc tcacttgcag agaagctcaa cagttcagtg tattcattgc 4500
caccagaccc agaccatttt gacggctata aacaacaagc tgtcgtaatc atggatgacc 4560
tgtgccagaa cccagatggg aaagacgtgt ccttgttctg ccagatggtt tcaagtgtag 4620
actttgttcc accaatggca gcacttgagg agaaaggcat actcttcaca tcgcctttcg 4680
tgctcgcctc taccaacgca ggttccatca acgctcccac tgtgtcagac agcagagccc 4740
ttgctagaag gttccacttc gacttgaaca ttgaggtcat ctcaatgtac agccagaatg 4800
gcaaaatcaa catgccaatg tcagtgaagg cttgtgatga tgagtgttgt ccagttaatt 4860

tcaagaggtg ttgtccatta gtgtgcggta aggccattca gttcattgac agaagaacac 4920
aaatgaggta ttcactagac atgcttgtaa ctgaaatgtt cagggagtac aaccacagac 4980
atagtgtggg ggcaacactc gaggcactct tccaaggccc acccgtgtac aaagagatca 5040
agatcagtgt tgcgccagaa acccctcccc caccggcaat agcagatctg ctgaaatctg 5100
tggacagtga agcagttaga gagtactgca aggaaaaggg atggttggtg cctgagatca 5160
attccactct acaaattgaa aagcacgtga gcagggcctt catttgcctg caagcgctca 5220
ccacgtttgt ttccgtggcg gggataattt acatcatata caaattgttt gctggcttcc 5280
agggggccta caccggtatg ccaaaccaaa aacctaaagt accaactttg aggcaggcta 5340
aggtgcaggg accggcattc gaattcgctg tggcaatgat gaaaagaaat gctagcacag 5400
tgaagaccga gtacggtgag ttcacaatgc ttggaatcta tgacaggtgg gcagtgctac 5460
ctcgtcacgc tagaccaggg cctaccatcc taatgaatga ccaggaagtg ggtgtggtgg 5520
atgccaagga gttagtggac aaggatggca ccaatttaga attaacactc ttgaagctca 5580
atagaaatga gaaattcaga gacatccgag gcttcttaac acgtgaggag gccgaagtca 5640
acgaggctgt gcttgctatt aacaccagca agttcccaaa tatgtacatt ccagttgggc 5700
aagtaacaga ttatggcttc ctcaacttgg gtggcacacc aactaagcgc atgctgatgt 5760
acaacttccc aacacgtgca ggccaatgtg ggggagttct aatgtctaca ggtaaagtgc 5820
tcggaataca cgtcggtggt aatggacacc aagggttttc ggcagccctg ttacgccact 5880
atttcaatga cgagcaggga gagattgagt tcatcgagag ctctaaggat gcaggctttc 5940
cagtcattaa cacacctagc aaaacaaagc tcgagcccag tgttttccat cacgtgtttg 6000
agggaaacaa agaaccagca gtgttgagga atggagaccc aagactgaaa gccaattttg 6060
aggaagccat tttctccaag tacatcggga atgtgaacac acatgtagac gagtacatga 6120
tggaagccgt cgaccattat gcaggacagt tagctacttt agatatcagt acagaaccta 6180
tgaagcttga ggatgctgtc tacggtactg agggcttgga agctctggac ttgaccacta 6240
gtgctgggta tccctatgtc gcccttggta taaaaaagag agacatctta tccaaaaagt 6300
ctaaggacct gtctaaactg agagagtgta tggacaagta tgggttaaac ctccctatgg 6360
tcacctatgt gaaggatgaa ttgaggtcag cagagaaagt ggctaagggc aaatccagac 6420
taatcgaggc gtccagttta aatgactcag tggcaatgag acagactttc ggtaacctgt 6480
ataagacatt ccatctgaac ccgggcatag tgactggcag tgcagttgga tgcgatcccg 6540
acttgttctg gagcaagatt ccagtcatgc tcgatggtca cctcatcgcc tttgactaca 6600
gtggatatga tgccagcctg agtccggtgt ggtttgcttg cttgaaactg ttgttggaaa 6660
agctggggta ctcacacaag gaaactaatt acatagatta cttgtgcaac tcccaccatt 6720

tgtacagaga caagcactac tttgtgaggg gaggaatgcc atccgggtgt tctggaacca 6780

gcattttcaa ttcaatgatt aataacataa tcatcagaac actgatgttg aaggtgtaca 6840

aagggataga tctagaccaa tttaggatga ttgcatatgg tgacgacgtc attgcttcgt 6900

acccgtatcc aattgatgcg tcattgctgg ctgaggctgg caaggggtat ggtctgatta 6960

tgacaccagc agacaaagga gagtgtttca atgaagtaac gtggaccaac gtcactttcc 7020

tgaaaagata cttcagagcg gatgagcaat atcccttcct cgttcaccca gtgatgccaa 7080

tgaaggacat tcatgagtca attaggtgga ctaaagaccc caaaaacacc caagatcacg 7140

tgcgatcctt gtgcttattg gcctggcaca atggagagca agaatatgag aattttgtat 7200

ccaaaatcag aagcgtccca gttgggcgct gcttgacatt gcctgcgttc tcgactctgc 7260

gcaggaagtg gttggattcc ttctaaaatt gaagtacaat gtgatttaac tcaattggct 7320

taaccctacc acacttaccg aactagataa cggtgtggta ggggtaaatt c 7371

<210> 2
<211> 507
<212> RNA
<213> Artificial Sequence

<220>
<223> RNA sequence of internal ribosome entry site sequence of HRV2

<400> 2
aacuuagaag uuuuucacaa agaccaauag ccgguaauca gccagauuac ugaaggucaa 60

gcacuucugu uuccccgguc aauguugaua ugcuccaaca gggcaaaaac aacugcgauc 120

guuaaccgca aagcgccuac gcaaagcuua guagcaucuu ugaaaucguu uggcuggucg 180

auccgccauu uccccuggua gaccuggcag augaggcuag aaauacccca cuggcgacag 240

uguucuagcc ugcguggcug ccugcacacc cuaugggugu gaagccaaac aauggacaag 300

gugugaagag ccccgugugc ucgcuuugag uccuccggcc ccugaaugug gcuaaccuua 360

acccugcagc uagagcacgu aacccaaugu guaucuaguc guaaugagca auugcgggau 420

gggaccaacu acuuugggug uccguguuuc acuuuuuccu uuauauuugc uuauggugac 480

aauauauaca auauauauau uggcacc 507

<210> 3
<211> 22
<212> RNA
<213> Artificial Sequence

<220>
<223> RNA sequence of miR-133 target sequence

<400> 3
acagcugguu gaaggggacc aa 22

<210> 4
<211> 22
<212> RNA
<213> Artificial Sequence

<220>
<223> RNA sequence of miR-206 target sequence

<400> 4
ccacacacuu ccuuacauuc ca 22

<210> 5
<211> 102
<212> RNA
<213> Artificial Sequence

<220>
<223> RNA sequence of tandem sequence of miR-133 target sequence and miR-206 target sequence

<400> 5
acagcugguu gaaggggacc aacgauacag cugguugaag gggaccaaac cgguccacac 60

acuuccuuac auuccaucac ccacacacuu ccuuacauuc ca 102

<210> 6
<211> 435
<212> DNA
<213> Artificial Sequence

<220>
<223> DNA sequence of GM-CSF gene

<400> 6
atgtggctgc agagcctgct gctcttgggc actgtggcct gcagcatctc tgcacccgcc 60

cgctcgccca gccccagcac gcagccctgg gagcatgtga atgccatcca ggaggcccgg 120

cgtctcctga acctgagtag agacactgct gctgagatga atgaaacagt agaagtcatc 180

tcagaaatgt ttgacctcca ggagccgacc tgcctacaga cccgcctgga gctgtacaag 240

cagggcctgc ggggcagcct caccaagctc aagggcccct tgaccatgat ggccagccac 300

tacaagcagc actgccctcc aaccccggaa acttcctgtg caacccagat tatcaccttt 360

gaaagtttca aagagaacct gaaggacttt ctgcttgtca tcccctttga ctgctgggag 420

ccagtccagg agtga 435

<210> 7
<211> 795
<212> DNA
<213> Artificial Sequence

<220>
<223> DNA sequence of Anti-PD-1 scFv

<400> 7
atgaagcacc tgtggttctt cctgctgctg gtggccgctc ctaggtgggt gctgtcccag 60

gtgcagctgg tgcagagcgg cgtggaggtg aagaagcccg gcgcttccgt gaaggtgtcc 120

tgcaaggcct ccggctacac cttcaccaac tactacatgt actgggtgag gcaggcccct 180

ggacagggac tggagtggat gggcggcatc aacccttcca acggcggcac caacttcaac 240

gagaagttca agaaccgggt gaccctgacc accgactcct ccaccaccac cgcctacatg 300

gagctgaagt ccctgcagtt tgacgacacc gccgtgtact actgcgccag gagggactac 360

cggttcgaca tgggcttcga ctactggggc cagggcacaa ccgtgaccgt gtccagcgga 420

ggtggcggat ctggaggggg tggtagcggt ggaggcggga gtgagatcgt gctgacccag 480

tcccctgcta cactgtccct gtcccccggc gagagggcta cactgagctg cagggcctcc 540

aagggcgtgt ccacctccgg ctactcctac ctgcactggt accagcagaa gcctggacag 600

gctcccaggc tgctgatcta cctggcctcc tacctggagt ccggcgtgcc tgctaggttt 660

tccggcagcg gcagcggcac cgatttcacc ctgaccatct cctccctgga gcccgaggac 720

ttcgccgtgt actactgcca gcactccagg gatctgcctc tgaccttcgg cggcggcacc 780

aaggtggaga tcaag 795

<210> 8
<211> 7251
<212> DNA
<213> Artificial Sequence

<220>
<223> cDNA sequence of CVB1-HRV2

<400> 8
tcctgtgggt tgatcccacc cacagggccc actgggcgct agcacactgg tattccggta 60

cctttgtgcg cctgttttat acacccttcc caagtgtaac ttagaagaac ttagaagttt 120

ttcacaaaga ccaatagccg gtaatcagcc agattactga aggtcaagca cttctgtttc 180

cccggtcaat gttgatatgc tccaacaggg caaaaacaac tgcgatcgtt aaccgcaaag 240

cgcctacgca aagcttagta gcatctttga aatcgtttgg ctggtcgatc cgccatttcc 300

cctggtagac ctggcagatg aggctagaaa taccccactg gcgacagtgt tctagcctgc 360

gtggctgcct gcacacccta tgggtgtgaa gccaaacaat ggacaaggtg tgaagagccc 420

cgtgtgctcg ctttgagtcc tccggcccct gaatgtggct aaccttaacc ctgcagctag 480

agcacgtaac ccaatgtgta tctagtcgta atgagcaatt gcgggatggg accaactact 540

ttgggtgtcc gtgtttcact ttttccttta tatttgctta tggtgacaat atatacaata 600

tatatattgg caccatggga gctcaggtgt ctacacaaaa gacgggtgca catgagactg 660

gcttgaatgc cagcggcaat tctgtcatcc attacaccaa catcaattac tacaaggatg 720

ctgcatccaa ctccgcaaat aggcaagact tcacacaaga cccaggtaag ttcactgaac 780

```
ctgtaaagga catcatggtg aaaactatgc ctgcgttgaa ctcaccctct gctgaggaat      840
gcggctacag tgacagagtg cgttccatca cattaggtaa ttccactatc accacccaag      900
agtgtgccaa cgtggtcgtc gggtacggag tgtggccaga gtatctaaaa gacaacgagg      960
ccacagccga agatcagccc acacaacctg atgtggccac ctgccgcttc tatactttag     1020
aatcagtgca gtggatgaaa aattcggccg ggtggtggtg gaagctgcct gatgcactgt     1080
cacagatggg cttgtttggt caaaacatgc agtaccacta cttgggaaga acagggtata     1140
ctatacacgt acaatgcaat gcctcaaagt tccatcaagg atgcctgctt gtagtgtgtg     1200
tgcctgaagc tgagatgggg tgctctaact tgaacaacac gcccgaattc agtgagttat     1260
ccggaggaga cagtgccaga atgttcaccg atacacaggt cggggagtca aacgccaaga     1320
aagtacagac agcggtgtgg aacgccggaa tgggcgttgg agtgggtaat ctcactattt     1380
tccctcacca atggatcaac ctgaggacca acaacagtgc aacgctagtg atgccttaca     1440
taaacagcgt ccccatggat aacatgttca ggcacaataa cttaacacta atgattatcc     1500
catttgtacc gcttaattac agcgagggat cctcaccgta cgtgcccata acagtcacca     1560
tcgctcctat gtgtgcagag tacaacgggc tacggctggc cagcaaccag ggtctaccag     1620
ttatgacaac acctgggagc acgcaatttc taacttcaga tgacttccag tcaccgtcag     1680
cgatgccaca gtttgatgtc accccagaga tgcaaatacc aggccgcgta aataatttga     1740
tggagattgc cgaggtggac tcagtggtgc ctgtgaataa cacagaggac aacgtgagta     1800
gccttaaggc ataccagatt ccagtacagt ccaacagtga caatggtaag caggtttttg     1860
gttttccctt acaaccgggt gccaacaacg ttctgaatag aaccctcttg ggtgaaattc     1920
taaactatta cacccattgg agtggcagca taaaacttac attcatgttc tgtgggtctg     1980
ccatggccac tggcaagttc cttctggcat actcaccacc tggagcagga gtcccgaaaa     2040
accgaaaaga tgctatgctg ggcacccacg tcatatggga tgttgggtta caatcaagct     2100
gcgtgttgtg cgtaccgtgg attagccaaa cacactacag atacgtggtt gaggatgaat     2160
acacagcagc cggatatgtt acatgctggt atcaaacaaa tatcgtggtg ccagctgatg     2220
tgcaaagctc atgtgacatc ttgtgctttg tttcagcctg caacgatttc tctgtgcgga     2280
tgctgaaaga tacgcccttt ataagacagg acacatttta tcacggccca gtggaggagt     2340
ctgtggatcg tgcagtggca cgtgtggcgg acacgattag ttcacggccc actaactcgg     2400
agtcaattcc agcgctcacc gccgccgaga ctgggcatac ctcccaagtt gtgcctagcg     2460
acaccatgca aacaagacat gtgaaaaact accactcacg gtccgaatcg tccattgaaa     2520
atttcctatg ccggtctgcc tgtgtatact atgccacata caccaataac tcaaaaaaag     2580
aggggtttgc agagtgggtt atcaacacta gacaggtggc acagctaaga agaaagttgg     2640
agctcttcac atatctaagg tttgacttag aactaacatt tgttataacg agcgcacaac     2700
```

aacccagtac cgcctccagt gtagacgctc ccgtgcaaac ccatcagatt atgtatgtgc 2760

ctccaggtgg ccctgtacca acaaaggtta aagattatgc atggcaaact tccacaaacc 2820

ccagcgtttt ctggacagaa ggaaacgccc caccaaggat gtccatccca ttcattagca 2880

ttggtaatgc gtatagttgt ttttatgatg ggtggacgca gttttcaaga aacggggtct 2940

atggcatcaa taccctcaac aacatgggca cgttgtatat gaggcatgtc aatgaagcgg 3000

ggcagggtcc aatcaaaagt actgttagaa tatatttcaa acccaagcac gtgaaggcgt 3060

gggtgccacg tccgccaaga ttgtgtcaat atgagaagca gaaaaatgtc aattttagcc 3120

ctataggagt aaccacaagt agaacggaca ttataacaac aggcactttt ggtcagcagt 3180

ccggagcgat atatgtgggc aattacagga tagtcaatag gcacctggca acacactttg 3240

actggcaaaa ttgcatatgg gaggattaca acagggacct cctagtatgc actacaacgg 3300

cgcacggttg tgacaatatt gcaagatgcc agtgcacagc aggtgtgtat tattgtgctt 3360

ctaggaacaa gcattacccg gtgcattttg aaggaccagg cttggtggag gtccaggaaa 3420

gtgaatatta tccaaaaagg tatcaatcac acgtgcttct tgccgcaggg ttttctgagc 3480

cgggggattg tggtggtatt ctcaggtgtg aacatggtgt tataggcatc gtgaccatgg 3540

gtggtgaggg tgttgtcggc tttgccgatg tgcgcgatct cttatggctg gaagacgacg 3600

caatggaaca gggagtcagg gactacgtgg aacagcttgg aaacgccttt ggttctgggt 3660

ttaccaacca gatttgcgag caagtcaatc tgttaaaaga gtcactaata ggccaagact 3720

ccatcttgga aaaatcactc aaggcattag tgaaaatcat atcagcactg gttatcgtgg 3780

tgagaaacca cgatgacctc ataacagtca ctgccacctt agccctgatt ggctgtacca 3840

cttcaccgtg gcggtggcta aagaaaaaag tgactcaata ctatgggatt cccatggccg 3900

agagacagaa caatggctgg ctcaagaaat tcacagaaat gaccaatgca tgcaagggca 3960

tggaatggat cgccatcaaa atccaaaagt tcattgagtg gctcaaaatt aagatcttgc 4020

cagaagtaaa ggaaaaacac gagtttctta ccagacttaa gcaactccca ctcttagaaa 4080

gccaaattgc caccatcgag cagagtgcac catcacagag tgatcaggaa caactgttct 4140

ccaatataca gtacttcgcg cactattgca gaaagtacgc cccactgtac gcagctgagg 4200

caaaaagagt gttctctcta gaaaagaaaa tgagcaacta catacagttc aagtccaaat 4260

gccgtattga accagtctgt ttattgctcc atggtagccc tggagccgga aaatccgtgg 4320

ctaccaattt gattggacgc tcacttgcag agaagctcaa cagttcagtg tattcattgc 4380

caccagaccc agaccatttt gacggctata aacaacaagc tgtcgtaatc atggatgacc 4440

tgtgccagaa cccagatggg aaagacgtgt ccttgttctg ccagatggtt tcaagtgtag 4500

actttgttcc accaatggca gcacttgagg agaaaggcat actcttcaca tcgcctttcg 4560

tgctcgcctc taccaacgca ggttccatca acgctcccac tgtgtcagac agcagagccc 4620
ttgctagaag gttccacttc gacttgaaca ttgaggtcat ctcaatgtac agccagaatg 4680
gcaaaatcaa catgccaatg tcagtgaagg cttgtgatga tgagtgttgt ccagttaatt 4740
tcaagaggtg ttgtccatta gtgtgcggta aggccattca gttcattgac agaagaacac 4800
aaatgaggta ttcactagac atgcttgtaa ctgaaatgtt cagggagtac aaccacagac 4860
atagtgtggg ggcaacactc gaggcactct tccaaggccc acccgtgtac aaagagatca 4920
agatcagtgt tgcgccagaa acccctcccc caccggcaat agcagatctg ctgaaatctg 4980
tggacagtga agcagttaga gagtactgca aggaaaaggg atggttggtg cctgagatca 5040
attccactct acaaattgaa aagcacgtga gcagggcctt catttgcctg caagcgctca 5100
ccacgtttgt ttccgtggcg gggataattt acatcatata caaattgttt gctggcttcc 5160
agggggccta caccggtatg ccaaaccaaa aacctaaagt accaactttg aggcaggcta 5220
aggtgcaggg accggcattc gaattcgctg tggcaatgat gaaaagaaat gctagcacag 5280
tgaagaccga gtacggtgag ttcacaatgc ttggaatcta tgacaggtgg gcagtgctac 5340
ctcgtcacgc tagaccaggg cctaccatcc taatgaatga ccaggaagtg ggtgtggtgg 5400
atgccaagga gttagtggac aaggatggca ccaatttaga attaacactc ttgaagctca 5460
atagaaatga gaaattcaga gacatccgag gcttcttaac acgtgaggag gccgaagtca 5520
acgaggctgt gcttgctatt aacaccagca agttcccaaa tatgtacatt ccagttgggc 5580
aagtaacaga ttatggcttc ctcaacttgg gtggcacacc aactaagcgc atgctgatgt 5640
acaacttccc aacacgtgca ggccaatgtg ggggagttct aatgtctaca ggtaaagtgc 5700
tcggaataca cgtcggtggt aatggacacc aagggttttc ggcagccctg ttacgccact 5760
atttcaatga cgagcaggga gagattgagt tcatcgagag ctctaaggat gcaggctttc 5820
cagtcattaa cacacctagc aaaacaaagc tcgagcccag tgttttccat cacgtgtttg 5880
agggaaacaa agaaccagca gtgttgagga atggagaccc aagactgaaa gccaattttg 5940
aggaagccat tttctccaag tacatcggga atgtgaacac acatgtagac gagtacatga 6000
tggaagccgt cgaccattat gcaggacagt tagctacttt agatatcagt acagaaccta 6060
tgaagcttga ggatgctgtc tacggtactg agggcttgga agctctggac ttgaccacta 6120
gtgctgggta tccctatgtc gcccttggta taaaaaagag agacatctta tccaaaaagt 6180
ctaaggacct gtctaaactg agagagtgta tggacaagta tgggttaaac ctccctatgg 6240
tcacctatgt gaaggatgaa ttgaggtcag cagagaaagt ggctaagggc aaatccagac 6300
taatcgaggc gtccagttta aatgactcag tggcaatgag acagactttc ggtaacctgt 6360
ataagacatt ccatctgaac ccgggcatag tgactggcag tgcagttgga tgcgatcccg 6420
acttgttctg gagcaagatt ccagtcatgc tcgatggtca cctcatcgcc tttgactaca 6480

gtggatatga tgccagcctg agtccggtgt ggtttgcttg cttgaaactg ttgttggaaa 6540

agctggggta ctcacacaag gaaactaatt acatagatta cttgtgcaac tcccaccatt 6600

tgtacagaga caagcactac tttgtgaggg gaggaatgcc atccgggtgt tctggaacca 6660

gcattttcaa ttcaatgatt aataacataa tcatcagaac actgatgttg aaggtgtaca 6720

aagggataga tctagaccaa tttaggatga ttgcatatgg tgacgacgtc attgcttcgt 6780

acccgtatcc aattgatgcg tcattgctgg ctgaggctgg caaggggtat ggtctgatta 6840

tgacaccagc agacaaagga gagtgtttca atgaagtaac gtggaccaac gtcactttcc 6900

tgaaaagata cttcagagcg gatgagcaat atcccttcct cgttcaccca gtgatgccaa 6960

tgaaggacat tcatgagtca attaggtgga ctaaagaccc caaaaacacc caagatcacg 7020

tgcgatcctt gtgcttattg gcctggcaca atggagagca agaatatgag aattttgtat 7080

ccaaaatcag aagcgtccca gttgggcgct gcttgacatt gcctgcgttc tcgactctgc 7140

gcaggaagtg gttggattcc ttctaaaatt gaagtacaat gtgatttaac tcaattggct 7200

taaccctacc acacttaccg aactagataa cggtgtggta ggggtaaatt c 7251

<210> 9
<211> 7473
<212> DNA
<213> Artificial Sequence

<220>
<223> cDNA sequence of CVB1-miR133&206T

<400> 9
tcctgtgggt tgatcccacc cacagggccc actgggcgct agcacactgg tattccggta 60

cctttgtgcg cctgttttat acacccttcc caagtgtaac ttagaagctt atcacacacg 120

gtcaacaggt gactcagtat gccaactggg tcttgatcaa gcacttctgt ttccccggac 180

tgagtatcaa taagctgctc acgcggctga aggagaaaac gttcgttaac cggccaatta 240

cttcgagaaa cctagtaaca ccatggaggt tgcgcagtgt ttcgctccac acaaccccag 300

tgtagatcag gccgatgagt caccgcactc ctcacgggcg accgtggcgg tggctgcgct 360

ggcggcctgc ccatgggata acccatggga cgcttcaata ctgacatggt gcgaagagtc 420

tattgagcta attggtagtc ctccggcccc tgaatgcggc taatcctaac tgcggagcag 480

atacccacac gccagtgggc agtctgtcgt aatgggcaac tctgcagcgg aaccgactac 540

tttgggtgtc cgtgtttcct tttatttta tactggctgc ttatggtgac aattgagaga 600

ttgttaccat atagctattg gattggccat ccagtgacaa acagagcgat tatttattta 660

tttgttggtt atatcccatt aagccaaaag gccctagtta ctctcaactt tatactattg 720

cttaatacaa cgaaatggga gctcaggtgt ctacacaaaa gacgggtgca catgagactg 780

gcttgaatgc cagcggcaat tctgtcatcc attacaccaa catcaattac tacaaggatg 840
ctgcatccaa ctccgcaaat aggcaagact tcacacaaga cccaggtaag ttcactgaac 900
ctgtaaagga catcatggtg aaaactatgc ctgcgttgaa ctcaccctct gctgaggaat 960
gcggctacag tgacagagtg cgttccatca cattaggtaa ttccactatc accacccaag 1020
agtgtgccaa cgtggtcgtc gggtacggag tgtggccaga gtatctaaaa gacaacgagg 1080
ccacagccga agatcagccc acacaacctg atgtggccac ctgccgcttc tatactttag 1140
aatcagtgca gtggatgaaa aattcggccg ggtggtggtg gaagctgcct gatgcactgt 1200
cacagatggg cttgtttggt caaaacatgc agtaccacta cttgggaaga acagggtata 1260
ctatacacgt acaatgcaat gcctcaaagt tccatcaagg atgcctgctt gtagtgtgtg 1320
tgcctgaagc tgagatgggg tgctctaact tgaacaacac gcccgaattc agtgagttat 1380
ccggaggaga cagtgccaga atgttcaccg atacacaggt cggggagtca aacgccaaga 1440
aagtacagac agcggtgtgg aacgccggaa tgggcgttgg agtgggtaat ctcactattt 1500
tccctcacca atggatcaac ctgaggacca acaacagtgc aacgctagtg atgccttaca 1560
taaacagcgt ccccatggat aacatgttca ggcacaataa cttaacacta atgattatcc 1620
catttgtacc gcttaattac agcgagggat cctcaccgta cgtgcccata acagtcacca 1680
tcgctcctat gtgtgcagag tacaacgggc tacggctggc cagcaaccag ggtctaccag 1740
ttatgacaac acctgggagc acgcaatttc taacttcaga tgacttccag tcaccgtcag 1800
cgatgccaca gtttgatgtc accccagaga tgcaaatacc aggccgcgta aataatttga 1860
tggagattgc cgaggtggac tcagtggtgc ctgtgaataa cacagaggac aacgtgagta 1920
gccttaaggc ataccagatt ccagtacagt ccaacagtga caatggtaag caggtttttg 1980
gttttccctt acaaccgggt gccaacaacg ttctgaatag aaccctcttg ggtgaaattc 2040
taaactatta cacccattgg agtggcagca taaaacttac attcatgttc tgtgggtctg 2100
ccatggccac tggcaagttc cttctggcat actcaccacc tggagcagga gtcccgaaaa 2160
accgaaaaga tgctatgctg ggcacccacg tcatatggga tgttgggtta caatcaagct 2220
gcgtgttgtg cgtaccgtgg attagccaaa cacactacag atacgtggtt gaggatgaat 2280
acacagcagc cggatatgtt acatgctggt atcaaacaaa tatcgtggtg ccagctgatg 2340
tgcaaagctc atgtgacatc ttgtgctttg tttcagcctg caacgatttc tctgtgcgga 2400
tgctgaaaga tacgcccttt ataagacagg acacatttta tcacggccca gtggaggagt 2460
ctgtggatcg tgcagtggca cgtgtggcgg acacgattag ttcacggccc actaactcgg 2520
agtcaattcc agcgctcacc gccgccgaga ctgggcatac ctcccaagtt gtgcctagcg 2580
acaccatgca aacaagacat gtgaaaaact accactcacg gtccgaatcg tccattgaaa 2640
atttcctatg ccggtctgcc tgtgtatact atgccacata caccaataac tcaaaaaaag 2700

aggggtttgc agagtgggtt atcaacacta gacaggtggc acagctaaga agaaagttgg 2760

agctcttcac atatctaagg tttgacttag aactaacatt tgttataacg agcgcacaac 2820

aacccagtac cgcctccagt gtagacgctc ccgtgcaaac ccatcagatt atgtatgtgc 2880

ctccaggtgg ccctgtacca acaaaggtta aagattatgc atggcaaact tccacaaacc 2940

ccagcgtttt ctggacagaa ggaaacgccc caccaaggat gtccatccca ttcattagca 3000

ttggtaatgc gtatagttgt ttttatgatg ggtggacgca gttttcaaga aacggggtct 3060

atggcatcaa taccctcaac aacatgggca cgttgtatat gaggcatgtc aatgaagcgg 3120

ggcagggtcc aatcaaaagt actgttagaa tatatttcaa acccaagcac gtgaaggcgt 3180

gggtgccacg tccgccaaga ttgtgtcaat atgagaagca gaaaaatgtc aattttagcc 3240

ctataggagt aaccacaagt agaacggaca ttataacaac aggcactttt ggtcagcagt 3300

ccggagcgat atatgtgggc aattacagga tagtcaatag gcacctggca acacactttg 3360

actggcaaaa ttgcatatgg gaggattaca acagggacct cctagtatgc actacaacgg 3420

cgcacggttg tgacaatatt gcaagatgcc agtgcacagc aggtgtgtat tattgtgctt 3480

ctaggaacaa gcattacccg gtgcattttg aaggaccagg cttggtggag gtccaggaaa 3540

gtgaatatta tccaaaaagg tatcaatcac acgtgcttct tgccgcaggg ttttctgagc 3600

cgggggattg tggtggtatt ctcaggtgtg aacatggtgt tataggcatc gtgaccatgg 3660

gtggtgaggg tgttgtcggc tttgccgatg tgcgcgatct cttatggctg gaagacgacg 3720

caatggaaca gggagtcagg gactacgtgg aacagcttgg aaacgccttt ggttctgggt 3780

ttaccaacca gatttgcgag caagtcaatc tgttaaaaga gtcactaata ggccaagact 3840

ccatcttgga aaaatcactc aaggcattag tgaaaatcat atcagcactg gttatcgtgg 3900

tgagaaacca cgatgacctc ataacagtca ctgccacctt agccctgatt ggctgtacca 3960

cttcaccgtg gcggtggcta aagaaaaaag tgactcaata ctatgggatt cccatggccg 4020

agagacagaa caatggctgg ctcaagaaat tcacagaaat gaccaatgca tgcaagggca 4080

tggaatggat cgccatcaaa atccaaaagt tcattgagtg gctcaaaatt aagatcttgc 4140

cagaagtaaa ggaaaaacac gagtttctta ccagacttaa gcaactccca ctcttagaaa 4200

gccaaattgc caccatcgag cagagtgcac catcacagag tgatcaggaa caactgttct 4260

ccaatataca gtacttcgcg cactattgca gaaagtacgc cccactgtac gcagctgagg 4320

caaaaagagt gttctctcta gaaaagaaaa tgagcaacta catacagttc aagtccaaat 4380

gccgtattga accagtctgt ttattgctcc atggtagccc tggagccgga aaatccgtgg 4440

ctaccaattt gattggacgc tcacttgcag agaagctcaa cagttcagtg tattcattgc 4500

caccagaccc agaccatttt gacggctata aacaacaagc tgtcgtaatc atggatgacc 4560

tgtgccagaa cccagatggg aaagacgtgt ccttgttctg ccagatggtt tcaagtgtag 4620
actttgttcc accaatggca gcacttgagg agaaaggcat actcttcaca tcgcctttcg 4680
tgctcgcctc taccaacgca ggttccatca acgctcccac tgtgtcagac agcagagccc 4740
ttgctagaag gttccacttc gacttgaaca ttgaggtcat ctcaatgtac agccagaatg 4800
gcaaaatcaa catgccaatg tcagtgaagg cttgtgatga tgagtgttgt ccagttaatt 4860
tcaagaggtg ttgtccatta gtgtgcggta aggccattca gttcattgac agaagaacac 4920
aaatgaggta ttcactagac atgcttgtaa ctgaaatgtt cagggagtac aaccacagac 4980
atagtgtggg ggcaacactc gaggcactct tccaaggccc acccgtgtac aaagagatca 5040
agatcagtgt tgcgccagaa acccctcccc caccggcaat agcagatctg ctgaaatctg 5100
tggacagtga agcagttaga gagtactgca aggaaaaggg atggttggtg cctgagatca 5160
attccactct acaaattgaa aagcacgtga gcagggcctt catttgcctg caagcgctca 5220
ccacgtttgt ttccgtggcg gggataattt acatcatata caaattgttt gctggcttcc 5280
aggggggccta caccggtatg ccaaaccaaa aacctaaagt accaactttg aggcaggcta 5340
aggtgcaggg accggcattc gaattcgctg tggcaatgat gaaaagaaat gctagcacag 5400
tgaagaccga gtacggtgag ttcacaatgc ttggaatcta tgacaggtgg gcagtgctac 5460
ctcgtcacgc tagaccaggg cctaccatcc taatgaatga ccaggaagtg ggtgtggtgg 5520
atgccaagga gttagtggac aaggatggca ccaatttaga attaacactc ttgaagctca 5580
atagaaatga gaaattcaga gacatccgag gcttcttaac acgtgaggag gccgaagtca 5640
acgaggctgt gcttgctatt aacaccagca agttcccaaa tatgtacatt ccagttgggc 5700
aagtaacaga ttatggcttc ctcaacttgg gtggcacacc aactaagcgc atgctgatgt 5760
acaacttccc aacacgtgca ggccaatgtg ggggagttct aatgtctaca ggtaaagtgc 5820
tcggaataca cgtcggtggt aatggacacc aagggttttc ggcagccctg ttacgccact 5880
atttcaatga cgagcaggga gagattgagt tcatcgagag ctctaaggat gcaggctttc 5940
cagtcattaa cacacctagc aaaacaaagc tcgagcccag tgttttccat cacgtgtttg 6000
agggaaacaa agaaccagca gtgttgagga atggagaccc aagactgaaa gccaattttg 6060
aggaagccat tttctccaag tacatcggga atgtgaacac acatgtagac gagtacatga 6120
tggaagccgt cgaccattat gcaggacagt tagctacttt agatatcagt acagaaccta 6180
tgaagcttga ggatgctgtc tacggtactg agggcttgga agctctggac ttgaccacta 6240
gtgctgggta tccctatgtc gcccttggta taaaaaagag agacatctta tccaaaaagt 6300
ctaaggacct gtctaaactg agagagtgta tggacaagta tgggttaaac ctccctatgg 6360
tcacctatgt gaaggatgaa ttgaggtcag cagagaaagt ggctaagggc aaatccagac 6420
taatcgaggc gtccagttta aatgactcag tggcaatgag acagactttc ggtaacctgt 6480

ataagacatt ccatctgaac ccgggcatag tgactggcag tgcagttgga tgcgatcccg 6540

acttgttctg gagcaagatt ccagtcatgc tcgatggtca cctcatcgcc tttgactaca 6600

gtggatatga tgccagcctg agtccggtgt ggtttgcttg cttgaaactg ttgttggaaa 6660

agctggggta ctcacacaag gaaactaatt acatagatta cttgtgcaac tcccaccatt 6720

tgtacagaga caagcactac tttgtgaggg gaggaatgcc atccgggtgt tctggaacca 6780

gcattttcaa ttcaatgatt aataacataa tcatcagaac actgatgttg aaggtgtaca 6840

aagggataga tctagaccaa tttaggatga ttgcatatgg tgacgacgtc attgcttcgt 6900

acccgtatcc aattgatgcg tcattgctgg ctgaggctgg caaggggtat ggtctgatta 6960

tgacaccagc agacaaagga gagtgtttca atgaagtaac gtggaccaac gtcactttcc 7020

tgaaaagata cttcagagcg gatgagcaat atcccttcct cgttcaccca gtgatgccaa 7080

tgaaggacat tcatgagtca attaggtgga ctaaagaccc caaaaacacc caagatcacg 7140

tgcgatcctt gtgcttattg gcctggcaca atggagagca agaatatgag aattttgtat 7200

ccaaaatcag aagcgtccca gttgggcgct gcttgacatt gcctgcgttc tcgactctgc 7260

gcaggaagtg gttggattcc ttctaaaata cagctggttg aaggggacca acgatacagc 7320

tggttgaagg ggaccaaacc ggtccacaca cttccttaca ttccatcacc cacacacttc 7380

cttacattcc atgaagtaca atgtgattta actcaattgg cttaacccta ccacacttac 7440

cgaactagat aacggtgtgg taggggtaaa ttc 7473

<210> 10
<211> 7848
<212> DNA
<213> Artificial Sequence

<220>
<223> cDNA sequence of CVB1-GM-CSF

<400> 10
tcctgtgggt tgatcccacc cacagggccc actgggcgct agcacactgg tattccggta 60

cctttgtgcg cctgttttat acacccttcc caagtgtaac ttagaagctt atcacacacg 120

gtcaacaggt gactcagtat gccaactggg tcttgatcaa gcacttctgt ttccccggac 180

tgagtatcaa taagctgctc acgcggctga aggagaaaac gttcgttaac cggccaatta 240

cttcgagaaa cctagtaaca ccatggaggt tgcgcagtgt ttcgctccac acaaccccag 300

tgtagatcag gccgatgagt caccgcactc ctcacgggcg accgtggcgg tggctgcgct 360

ggcggcctgc ccatgggata acccatggga cgcttcaata ctgacatggt gcgaagagtc 420

tattgagcta attggtagtc ctccggcccc tgaatgcggc taatcctaac tgcggagcag 480

atacccacac gccagtgggc agtctgtcgt aatgggcaac tctgcagcgg aaccgactac 540

tttgggtgtc cgtgtttcct tttattttta tactggctgc ttatggtgac aattgagaga 600

ttgttaccat atagctattg gattggccat ccagtgacaa acagagcgat tatttattta 660

tttgttggtt atatcccatt aagccaaaag gccctagtta ctctcaactt tatactattg 720

cttaatacaa cgaaatggga gctcaggtgt ctacacaaaa gacgggtgca catgagactg 780

gcttgaatgc cagcggcaat tctgtcatcc attacaccaa catcaattac tacaaggatg 840

ctgcatccaa ctccgcaaat aggcaagact tcacacaaga cccaggtaag ttcactgaac 900

ctgtaaagga catcatggtg aaaactatgc ctgcgttgaa ctcaccctct gctgaggaat 960

gcggctacag tgacagagtg cgttccatca cattaggtaa ttccactatc accacccaag 1020

agtgtgccaa cgtggtcgtc gggtacggag tgtggccaga gtatctaaaa gacaacgagg 1080

ccacagccga agatcagccc acacaacctg atgtggccac ctgccgcttc tatactttag 1140

aatcagtgca gtggatgaaa aattcggccg ggtggtggtg gaagctgcct gatgcactgt 1200

cacagatggg cttgtttggt caaaacatgc agtaccacta cttgggaaga acagggtata 1260

ctatacacgt acaatgcaat gcctcaaagt tccatcaagg atgcctgctt gtagtgtgtg 1320

tgcctgaagc tgagatgggg tgctctaact tgaacaacac gcccgaattc agtgagttat 1380

ccggaggaga cagtgccaga atgttcaccg atacacaggt cggggagtca aacgccaaga 1440

aagtacagac agcggtgtgg aacgccggaa tgggcgttgg agtgggtaat ctcactattt 1500

tccctcacca atggatcaac ctgaggacca acaacagtgc aacgctagtg atgccttaca 1560

taaacagcgt ccccatggat aacatgttca ggcacaataa cttaacacta atgattatcc 1620

catttgtacc gcttaattac agcgagggat cctcaccgta cgtgcccata acagtcacca 1680

tcgctcctat gtgtgcagag tacaacgggc tacggctggc cagcaaccag ggtctaccag 1740

ttatgacaac acctgggagc acgcaatttc taacttcaga tgacttccag tcaccgtcag 1800

cgatgccaca gtttgatgtc accccagaga tgcaaatacc aggccgcgta aataatttga 1860

tggagattgc cgaggtggac tcagtggtgc ctgtgaataa cacagaggac aacgtgagta 1920

gccttaaggc ataccagatt ccagtacagt ccaacagtga caatggtaag caggtttttg 1980

gttttccctt acaaccgggt gccaacaacg ttctgaatag aaccctcttg ggtgaaattc 2040

taaactatta cacccattgg agtggcagca taaaacttac attcatgttc tgtgggtctg 2100

ccatggccac tggcaagttc cttctggcat actcaccacc tggagcagga gtcccgaaaa 2160

accgaaaaga tgctatgctg ggcacccacg tcatatggga tgttgggtta caatcaagct 2220

gcgtgttgtg cgtaccgtgg attagccaaa cacactacag atacgtggtt gaggatgaat 2280

acacagcagc cggatatgtt acatgctggt atcaaacaaa tatcgtggtg ccagctgatg 2340

tgcaaagctc atgtgacatc ttgtgctttg tttcagcctg caacgatttc tctgtgcgga 2400

tgctgaaaga tacgcccttt ataagacagg acacatttta tcacggccca gtggaggagt 2460

ctgtggatcg tgcagtggca cgtgtggcgg acacgattag ttcacggccc actaactcgg 2520
agtcaattcc agcgctcacc gccgccgaga ctgggcatac ctcccaagtt gtgcctagcg 2580
acaccatgca aacaagacat gtgaaaaact accactcacg gtccgaatcg tccattgaaa 2640
atttcctatg ccggtctgcc tgtgtatact atgccacata caccaataac tcaaaaaaag 2700
aggggtttgc agagtgggtt atcaacacta gacaggtggc acagctaaga agaaagttgg 2760
agctcttcac atatctaagg tttgacttag aactaacatt tgttataacg agcgcacaac 2820
aacccagtac cgcctccagt gtagacgctc ccgtgcaaac ccatcagatt atgtatgtgc 2880
ctccaggtgg ccctgtacca acaaaggtta aagattatgc atggcaaact tccacaaacc 2940
ccagcgtttt ctggacagaa ggaaacgccc caccaaggat gtccatccca ttcattagca 3000
ttggtaatgc gtatagttgt ttttatgatg ggtggacgca gttttcaaga aacggggtct 3060
atggcatcaa taccctcaac aacatgggca cgttgtatat gaggcatgtc aatgaagcgg 3120
ggcagggtcc aatcaaaagt actgttagaa tatatttcaa acccaagcac gtgaaggcgt 3180
gggtgccacg tccgccaaga ttgtgtcaat atgagaagca gaaaaatgtc aattttagcc 3240
ctataggagt aaccacaagt agaacggaca ttataacaac aggcactttt ggtcagcagt 3300
ggctgcagag cctgctgctc ttgggcactg tggcctgcag catctctgca cccgcccgct 3360
cgcccagccc cagcacgcag ccctgggagc atgtgaatgc catccaggag gcccggcgtc 3420
tcctgaacct gagtagagac actgctgctg agatgaatga aacagtagaa gtcatctcag 3480
aaatgtttga cctccaggag ccgacctgcc tacagacccg cctggagctg tacaagcagg 3540
gcctgcgggg cagcctcacc aagctcaagg gccccttgac catgatggcc agccactaca 3600
agcagcactg ccctccaacc ccggaaactt cctgtgcaac ccagattatc acctttgaaa 3660
gtttcaaaga gaacctgaag gactttctgc ttgtcatccc ctttgactgc tgggagccag 3720
tccaggagac cacaagtaga acggacatta taacaacagg cacttttggt cagcagtccg 3780
gagcgatata tgtgggcaat tacaggatag tcaataggca cctggcaaca cactttgact 3840
ggcaaaattg catatgggag gattacaaca gggacctcct agtatgcact acaacggcgc 3900
acggttgtga caatattgca agatgccagt gcacagcagg tgtgtattat tgtgcttcta 3960
ggaacaagca ttacccggtg cattttgaag gaccaggctt ggtggaggtc caggaaagtg 4020
aatattatcc aaaaaggtat caatcacacg tgcttcttgc cgcagggttt tctgagccgg 4080
gggattgtgg tggtattctc aggtgtgaac atggtgttat aggcatcgtg accatgggtg 4140
gtgagggtgt tgtcggcttt gccgatgtgc gcgatctctt atggctggaa gacgacgcaa 4200
tggaacaggg agtcagggac tacgtggaac agcttggaaa cgcctttggt tctgggttta 4260
ccaaccagat ttgcgagcaa gtcaatctgt taaaagagtc actaataggc caagactcca 4320

tcttggaaaa atcactcaag gcattagtga aaatcatatc agcactggtt atcgtggtga 4380
gaaaccacga tgacctcata acagtcactg ccaccttagc cctgattggc tgtaccactt 4440
caccgtggcg gtggctaaag aaaaaagtga ctcaatacta tgggattccc atggccgaga 4500
gacagaacaa tggctggctc aagaaattca cagaaatgac caatgcatgc aagggcatgg 4560
aatggatcgc catcaaaatc caaaagttca ttgagtggct caaaattaag atcttgccag 4620
aagtaaagga aaaacacgag tttcttacca gacttaagca actcccactc ttagaaagcc 4680
aaattgccac catcgagcag agtgcaccat cacagagtga tcaggaacaa ctgttctcca 4740
atatacagta cttcgcgcac tattgcagaa agtacgcccc actgtacgca gctgaggcaa 4800
aaagagtgtt ctctctagaa aagaaaatga gcaactacat acagttcaag tccaaatgcc 4860
gtattgaacc agtctgttta ttgctccatg gtagccctgg agccggaaaa tccgtggcta 4920
ccaatttgat tggacgctca cttgcagaga agctcaacag ttcagtgtat tcattgccac 4980
cagacccaga ccattttgac ggctataaac aacaagctgt cgtaatcatg gatgacctgt 5040
gccagaaccc agatgggaaa gacgtgtcct tgttctgcca gatggtttca agtgtagact 5100
ttgttccacc aatggcagca cttgaggaga aaggcatact cttcacatcg cctttcgtgc 5160
tcgcctctac caacgcaggt tccatcaacg ctcccactgt gtcagacagc agagcccttg 5220
ctagaaggtt ccacttcgac ttgaacattg aggtcatctc aatgtacagc cagaatggca 5280
aaatcaacat gccaatgtca gtgaaggctt gtgatgatga gtgttgtcca gttaatttca 5340
agaggtgttg tccattagtg tgcggtaagg ccattcagtt cattgacaga agaacacaaa 5400
tgaggtattc actagacatg cttgtaactg aaatgttcag ggagtacaac cacagacata 5460
gtgtgggggc aacactcgag gcactcttcc aaggcccacc cgtgtacaaa gagatcaaga 5520
tcagtgttgc gccagaaacc cctcccccac cggcaatagc agatctgctg aaatctgtgg 5580
acagtgaagc agttagagag tactgcaagg aaaagggatg gttggtgcct gagatcaatt 5640
ccactctaca aattgaaaag cacgtgagca gggccttcat ttgcctgcaa gcgctcacca 5700
cgtttgtttc cgtggcgggg ataatttaca tcatatacaa attgtttgct ggcttccagg 5760
gggcctacac cggtatgcca aaccaaaaac ctaaagtacc aactttgagg caggctaagg 5820
tgcagggacc ggcattcgaa ttcgctgtgg caatgatgaa aagaaatgct agcacagtga 5880
agaccgagta cggtgagttc acaatgcttg gaatctatga caggtgggca gtgctacctc 5940
gtcacgctag accagggcct accatcctaa tgaatgacca ggaagtgggt gtggtggatg 6000
ccaaggagtt agtggacaag gatggcacca atttagaatt aacactcttg aagctcaata 6060
gaaatgagaa attcagagac atccgaggct tcttaacacg tgaggaggcc gaagtcaacg 6120
aggctgtgct tgctattaac accagcaagt tcccaaatat gtacattcca gttgggcaag 6180
taacagatta tggcttcctc aacttgggtg gcacaccaac taagcgcatg ctgatgtaca 6240

```
acttcccaac acgtgcaggc caatgtgggg gagttctaat gtctacaggt aaagtgctcg     6300

gaatacacgt cggtggtaat ggacaccaag ggttttcggc agccctgtta cgccactatt     6360

tcaatgacga gcagggagag attgagttca tcgagagctc taaggatgca ggctttccag     6420

tcattaacac acctagcaaa acaaagctcg agcccagtgt tttccatcac gtgtttgagg     6480

gaaacaaaga accagcagtg ttgaggaatg gagacccaag actgaaagcc aattttgagg     6540

aagccatttt ctccaagtac atcgggaatg tgaacacaca tgtagacgag tacatgatgg     6600

aagccgtcga ccattatgca ggacagttag ctactttaga tatcagtaca gaacctatga     6660

agcttgagga tgctgtctac ggtactgagg gcttggaagc tctggacttg accactagtg     6720

ctgggtatcc ctatgtcgcc cttggtataa aaaagagaga catcttatcc aaaaagtcta     6780

aggacctgtc taaactgaga gagtgtatgg acaagtatgg gttaaacctc cctatggtca     6840

cctatgtgaa ggatgaattg aggtcagcag agaaagtggc taagggcaaa tccagactaa     6900

tcgaggcgtc cagtttaaat gactcagtgg caatgagaca gactttcggt aacctgtata     6960

agacattcca tctgaacccg ggcatagtga ctggcagtgc agttggatgc gatcccgact     7020

tgttctggag caagattcca gtcatgctcg atggtcacct catcgccttt gactacagtg     7080

gatatgatgc cagcctgagt ccggtgtggt ttgcttgctt gaaactgttg ttggaaaagc     7140

tggggtactc acacaaggaa actaattaca tagattactt gtgcaactcc caccatttgt     7200

acagagacaa gcactacttt gtgaggggag gaatgccatc cgggtgttct ggaaccagca     7260

ttttcaattc aatgattaat aacataatca tcagaacact gatgttgaag gtgtacaaag     7320

ggatagatct agaccaattt aggatgattg catatggtga cgacgtcatt gcttcgtacc     7380

cgtatccaat tgatgcgtca ttgctggctg aggctggcaa ggggtatggt ctgattatga     7440

caccagcaga caaaggagag tgtttcaatg aagtaacgtg gaccaacgtc actttcctga     7500

aaagatactt cagagcggat gagcaatatc ccttcctcgt tcacccagtg atgccaatga     7560

aggacattca tgagtcaatt aggtggacta aagaccccaa aaacacccaa gatcacgtgc     7620

gatccttgtg cttattggcc tggcacaatg gagagcaaga atatgagaat tttgtatcca     7680

aaatcagaag cgtcccagtt gggcgctgct tgacattgcc tgcgttctcg actctgcgca     7740

ggaagtggtt ggattccttc taaaattgaa gtacaatgtg atttaactca attggcttaa     7800

ccctaccaca cttaccgaac tagataacgg tgtggtaggg gtaaattc                  7848
```

<210> 11
<211> 8214
<212> DNA
<213> Artificial Sequence

<220>
<223> cDNA sequence of CVB1-Anti-PD1

<400> 11
tcctgtgggt tgatcccacc cacagggccc actgggcgct agcacactgg tattccggta 60

cctttgtgcg cctgttttat acacccttcc caagtgtaac ttagaagctt atcacacacg 120

gtcaacaggt gactcagtat gccaactggg tcttgatcaa gcacttctgt ttccccggac 180

tgagtatcaa taagctgctc acgcggctga aggagaaaac gttcgttaac cggccaatta 240

cttcgagaaa cctagtaaca ccatggaggt tgcgcagtgt ttcgctccac acaaccccag 300

tgtagatcag gccgatgagt caccgcactc ctcacgggcg accgtggcgg tggctgcgct 360

ggcggcctgc ccatgggata acccatggga cgcttcaata ctgacatggt gcgaagagtc 420

tattgagcta attggtagtc ctccggcccc tgaatgcggc taatcctaac tgcggagcag 480

atacccacac gccagtgggc agtctgtcgt aatgggcaac tctgcagcgg aaccgactac 540

tttgggtgtc cgtgtttcct tttatttta tactggctgc ttatggtgac aattgagaga 600

ttgttaccat atagctattg gattggccat ccagtgacaa acagagcgat tatttattta 660

tttgttggtt atatcccatt aagccaaaag gccctagtta ctctcaactt tatactattg 720

cttaatacaa cgaaatggga gctcaggtgt ctacacaaaa gacgggtgca catgagactg 780

gcttgaatgc cagcggcaat tctgtcatcc attacaccaa catcaattac tacaaggatg 840

ctgcatccaa ctccgcaaat aggcaagact tcacacaaga cccaggtaag ttcactgaac 900

ctgtaaagga catcatggtg aaaactatgc ctgcgttgaa ctcaccctct gctgaggaat 960

gcggctacag tgacagagtg cgttccatca cattaggtaa ttccactatc accacccaag 1020

agtgtgccaa cgtggtcgtc gggtacggag tgtggccaga gtatctaaaa gacaacgagg 1080

ccacagccga agatcagccc acacaacctg atgtggccac ctgccgcttc tatactttag 1140

aatcagtgca gtggatgaaa aattcggccg ggtggtggtg gaagctgcct gatgcactgt 1200

cacagatggg cttgtttggt caaaacatgc agtaccacta cttgggaaga acagggtata 1260

ctatacacgt acaatgcaat gcctcaaagt tccatcaagg atgcctgctt gtagtgtgtg 1320

tgcctgaagc tgagatgggg tgctctaact tgaacaacac gcccgaattc agtgagttat 1380

ccggaggaga cagtgccaga atgttcaccg atacacaggt cggggagtca aacgccaaga 1440

aagtacagac agcggtgtgg aacgccggaa tgggcgttgg agtgggtaat ctcactattt 1500

tccctcacca atggatcaac ctgaggacca acaacagtgc aacgctagtg atgccttaca 1560

taaacagcgt ccccatggat aacatgttca ggcacaataa cttaacacta atgattatcc 1620

catttgtacc gcttaattac agcgagggat cctcaccgta cgtgcccata acagtcacca 1680

tcgctcctat gtgtgcagag tacaacgggc tacggctggc cagcaaccag ggtctaccag 1740

ttatgacaac acctgggagc acgcaatttc taacttcaga tgacttccag tcaccgtcag 1800

cgatgccaca gtttgatgtc accccagaga tgcaaatacc aggccgcgta aataatttga 1860

tggagattgc cgaggtggac tcagtggtgc ctgtgaataa cacagaggac aacgtgagta 1920
gccttaaggc ataccagatt ccagtacagt ccaacagtga caatggtaag caggtttttg 1980
gttttccctt acaaccgggt gccaacaacg ttctgaatag aaccctcttg ggtgaaattc 2040
taaactatta cacccattgg agtggcagca taaaacttac attcatgttc tgtgggtctg 2100
ccatggccac tggcaagttc cttctggcat actcaccacc tggagcagga gtcccgaaaa 2160
accgaaaaga tgctatgctg ggcacccacg tcatatggga tgttgggtta caatcaagct 2220
gcgtgttgtg cgtaccgtgg attagccaaa cacactacag atacgtggtt gaggatgaat 2280
acacagcagc cggatatgtt acatgctggt atcaaacaaa tatcgtggtg ccagctgatg 2340
tgcaaagctc atgtgacatc ttgtgctttg tttcagcctg caacgatttc tctgtgcgga 2400
tgctgaaaga tacgcccttt ataagacagg acacatttta tcacggccca gtggaggagt 2460
ctgtggatcg tgcagtggca cgtgtggcgg acacgattag ttcacggccc actaactcgg 2520
agtcaattcc agcgctcacc gccgccgaga ctgggcatac ctcccaagtt gtgcctagcg 2580
acaccatgca aacaagacat gtgaaaaact accactcacg gtccgaatcg tccattgaaa 2640
atttcctatg ccggtctgcc tgtgtatact atgccacata caccaataac tcaaaaaaag 2700
aggggtttgc agagtgggtt atcaacacta gacaggtggc acagctaaga agaaagttgg 2760
agctcttcac atatctaagg tttgacttag aactaacatt tgttataacg agcgcacaac 2820
aacccagtac cgcctccagt gtagacgctc ccgtgcaaac ccatcagatt atgtatgtgc 2880
ctccaggtgg ccctgtacca acaaaggtta aagattatgc atggcaaact tccacaaacc 2940
ccagcgtttt ctggacagaa ggaaacgccc caccaaggat gtccatccca ttcattagca 3000
ttggtaatgc gtatagttgt ttttatgatg ggtggacgca gttttcaaga aacggggtct 3060
atggcatcaa taccctcaac aacatgggca cgttgtatat gaggcatgtc aatgaagcgg 3120
ggcagggtcc aatcaaaagt actgttagaa tatatttcaa acccaagcac gtgaaggcgt 3180
gggtgccacg tccgccaaga ttgtgtcaat atgagaagca gaaaaatgtc aattttagcc 3240
ctataggagt aaccacaagt agaacggaca ttataacaac aggcactttt ggtcagcaga 3300
tgaagcacct gtggttcttc ctgctgctgg tggccgctcc taggtgggtg ctgtcccagg 3360
tgcagctggt gcagagcggc gtggaggtga agaagcccgg cgcttccgtg aaggtgtcct 3420
gcaaggcctc cggctacacc ttcaccaact actacatgta ctgggtgagg caggcccctg 3480
gacagggact ggagtggatg ggcggcatca acccttccaa cggcggcacc aacttcaacg 3540
agaagttcaa gaaccgggtg accctgacca ccgactcctc caccaccacc gcctacatgg 3600
agctgaagtc cctgcagttt gacgacaccg ccgtgtacta ctgcgccagg agggactacc 3660
ggttcgacat gggcttcgac tactggggcc agggcacaac cgtgaccgtg tccagcggag 3720

gtggcggatc tggagggggt ggtagcggtg gaggcgggag tgagatcgtg ctgacccagt 3780
cccctgctac actgtccctg tcccccggcg agagggctac actgagctgc agggcctcca 3840
agggcgtgtc cacctccggc tactcctacc tgcactggta ccagcagaag cctggacagg 3900
ctcccaggct gctgatctac ctggcctcct acctggagtc cggcgtgcct gctaggtttt 3960
ccggcagcgg cagcggcacc gatttcaccc tgaccatctc ctccctggag cccgaggact 4020
tcgccgtgta ctactgccag cactccaggg atctgcctct gaccttcggc ggcggcacca 4080
aggtggagat caagaccaca agtagaacgg acattataac aacaggcact tttggtcagc 4140
agtccggagc gatatatgtg ggcaattaca ggatagtcaa taggcacctg gcaacacact 4200
ttgactggca aaattgcata tgggaggatt acaacaggga cctcctagta tgcactacaa 4260
cggcgcacgg ttgtgacaat attgcaagat gccagtgcac agcaggtgtg tattattgtg 4320
cttctaggaa caagcattac ccggtgcatt ttgaaggacc aggcttggtg gaggtccagg 4380
aaagtgaata ttatccaaaa aggtatcaat cacacgtgct tcttgccgca gggttttctg 4440
agccggggga ttgtggtggt attctcaggt gtgaacatgg tgttataggc atcgtgacca 4500
tgggtggtga gggtgttgtc ggctttgccg atgtgcgcga tctcttatgg ctggaagacg 4560
acgcaatgga acagggagtc agggactacg tggaacagct tggaaacgcc tttggttctg 4620
ggtttaccaa ccagatttgc gagcaagtca atctgttaaa agagtcacta ataggccaag 4680
actccatctt ggaaaaatca ctcaaggcat tagtgaaaat catatcagca ctggttatcg 4740
tggtgagaaa ccacgatgac ctcataacag tcactgccac cttagccctg attggctgta 4800
ccacttcacc gtggcggtgg ctaaagaaaa aagtgactca atactatggg attcccatgg 4860
ccgagagaca gaacaatggc tggctcaaga aattcacaga aatgaccaat gcatgcaagg 4920
gcatggaatg gatcgccatc aaaatccaaa agttcattga gtggctcaaa attaagatct 4980
tgccagaagt aaaggaaaaa cacgagtttc ttaccagact taagcaactc ccactcttag 5040
aaagccaaat tgccaccatc gagcagagtg caccatcaca gagtgatcag gaacaactgt 5100
tctccaatat acagtacttc gcgcactatt gcagaaagta cgccccactg tacgcagctg 5160
aggcaaaaag agtgttctct ctagaaaaga aaatgagcaa ctacatacag ttcaagtcca 5220
aatgccgtat tgaaccagtc tgtttattgc tccatggtag ccctggagcc ggaaaatccg 5280
tggctaccaa tttgattgga cgctcacttg cagagaagct caacagttca gtgtattcat 5340
tgccaccaga cccagaccat tttgacggct ataaacaaca agctgtcgta atcatggatg 5400
acctgtgcca gaacccagat gggaaagacg tgtccttgtt ctgccagatg gtttcaagtg 5460
tagactttgt tccaccaatg gcagcacttg aggagaaagg catactcttc acatcgcctt 5520
tcgtgctcgc ctctaccaac gcaggttcca tcaacgctcc cactgtgtca gacagcagag 5580
cccttgctag aaggttccac ttcgacttga acattgaggt catctcaatg tacagccaga 5640

atggcaaaat caacatgcca atgtcagtga aggcttgtga tgatgagtgt tgtccagtta 5700
atttcaagag gtgttgtcca ttagtgtgcg gtaaggccat tcagttcatt gacagaagaa 5760
cacaaatgag gtattcacta gacatgcttg taactgaaat gttcagggag tacaaccaca 5820
gacatagtgt gggggcaaca ctcgaggcac tcttccaagg cccacccgtg tacaaagaga 5880
tcaagatcag tgttgcgcca gaaacccctc ccccaccggc aatagcagat ctgctgaaat 5940
ctgtggacag tgaagcagtt agagagtact gcaaggaaaa gggatggttg gtgcctgaga 6000
tcaattccac tctacaaatt gaaaagcacg tgagcagggc cttcatttgc ctgcaagcgc 6060
tcaccacgtt tgtttccgtg gcggggataa tttacatcat atacaaattg tttgctggct 6120
tccagggggc ctacaccggt atgccaaacc aaaaacctaa agtaccaact ttgaggcagg 6180
ctaaggtgca gggaccggca ttcgaattcg ctgtggcaat gatgaaaaga aatgctagca 6240
cagtgaagac cgagtacggt gagttcacaa tgcttggaat ctatgacagg tgggcagtgc 6300
tacctcgtca cgctagacca gggcctacca tcctaatgaa tgaccaggaa gtgggtgtgg 6360
tggatgccaa ggagttagtg gacaaggatg gcaccaattt agaattaaca ctcttgaagc 6420
tcaatagaaa tgagaaattc agagacatcc gaggcttctt aacacgtgag gaggccgaag 6480
tcaacgaggc tgtgcttgct attaacacca gcaagttccc aaatatgtac attccagttg 6540
ggcaagtaac agattatggc ttcctcaact tgggtggcac accaactaag cgcatgctga 6600
tgtacaactt cccaacacgt gcaggccaat gtgggggagt tctaatgtct acaggtaaag 6660
tgctcggaat acacgtcggt ggtaatggac accaagggtt ttcggcagcc ctgttacgcc 6720
actatttcaa tgacgagcag ggagagattg agttcatcga gagctctaag gatgcaggct 6780
ttccagtcat taacacacct agcaaaacaa agctcgagcc cagtgttttc catcacgtgt 6840
ttgagggaaa caaagaacca gcagtgttga ggaatggaga cccaagactg aaagccaatt 6900
ttgaggaagc cattttctcc aagtacatcg ggaatgtgaa cacacatgta gacgagtaca 6960
tgatggaagc cgtcgaccat tatgcaggac agttagctac tttagatatc agtacagaac 7020
ctatgaagct tgaggatgct gtctacggta ctgagggctt ggaagctctg gacttgacca 7080
ctagtgctgg gtatccctat gtcgcccttg gtataaaaaa gagagacatc ttatccaaaa 7140
agtctaagga cctgtctaaa ctgagagagt gtatggacaa gtatgggtta aacctcccta 7200
tggtcaccta tgtgaaggat gaattgaggt cagcagagaa agtggctaag ggcaaatcca 7260
gactaatcga ggcgtccagt ttaaatgact cagtggcaat gagacagact ttcggtaacc 7320
tgtataagac attccatctg aacccgggca tagtgactgg cagtgcagtt ggatgcgatc 7380
ccgacttgtt ctggagcaag attccagtca tgctcgatgg tcacctcatc gcctttgact 7440
acagtggata tgatgccagc ctgagtccgg tgtggtttgc ttgcttgaaa ctgttgttgg 7500

aaaagctggg gtactcacac aaggaaacta attacataga ttacttgtgc aactcccacc 7560

atttgtacag agacaagcac tactttgtga ggggaggaat gccatccggg tgttctggaa 7620

ccagcatttt caattcaatg attaataaca taatcatcag aacactgatg ttgaaggtgt 7680

acaaagggat agatctagac caatttagga tgattgcata tggtgacgac gtcattgctt 7740

cgtacccgta tccaattgat gcgtcattgc tggctgaggc tggcaagggg tatggtctga 7800

ttatgacacc agcagacaaa ggagagtgtt tcaatgaagt aacgtggacc aacgtcactt 7860

tcctgaaaag atacttcaga gcggatgagc aatatccctt cctcgttcac ccagtgatgc 7920

caatgaagga cattcatgag tcaattaggt ggactaaaga ccccaaaaac acccaagatc 7980

acgtgcgatc cttgtgctta ttggcctggc acaatggaga gcaagaatat gagaattttg 8040

tatccaaaat cagaagcgtc ccagttgggc gctgcttgac attgcctgcg ttctcgactc 8100

tgcgcaggaa gtggttggat tccttctaaa attgaagtac aatgtgattt aactcaattg 8160

gcttaaccct accacactta ccgaactaga taacggtgtg gtaggggtaa attc 8214

<210> 12
<211> 7371
<212> RNA
<213> Artificial Sequence

<220>
<223> Genomic sequence of CVB1-WT

<400> 12
uccugugggu ugaucccacc cacagggccc acugggcgcu agcacacugg uauuccggua 60

ccuuugugcg ccuguuuuau acacccuucc caaguguaac uuagaagcuu aucacacacg 120

gucaacaggu gacucaguau gccaacuggg ucuugaucaa gcacuucugu uuccccggac 180

ugaguaucaa uaagcugcuc acgcggcuga aggagaaaac guucguuaac cggccaauua 240

cuucgagaaa ccuaguaaca ccauggaggu ugcgcagugu uucgcuccac acaaccccag 300

uguagaucag gccgaugagu caccgcacuc cucacgggcg accguggcgg uggcugcgcu 360

ggcggccugc ccaugggaua acccauggga cgcuucaaua cugacauggu gcgaagaguc 420

uauugagcua auugguaguc cuccggcccc ugaaugcggc uaauccuaac ugcggagcag 480

auacccacac gccagugggc agucugucgu aaugggcaac ucugcagcgg aaccgacuac 540

uuuggguguc cguguuuccu uuuauuuuua uacuggcugc uuauggugac aauugagaga 600

uuguuaccau auagcuauug gauuggccau ccagugacaa acagagcgau uauuuauuua 660

uuuguugguu auaucccauu aagccaaaag gcccuaguua cucucaacuu uauacuauug 720

cuuaauacaa cgaaauggga gcucaggugu cuacacaaaa gacgggugca caugagacug 780

gcuugaaugc cagcggcaau ucugucaucc auuacaccaa caucaauuac uacaaggaug 840

cugcauccaa cuccgcaaau aggcaagacu ucacacaaga cccagguaag uucacugaac 900

```
cuguaaagga caucauggug aaaacuaugc cugcguugaa cucacccucu gcugaggaau      960
gcggcuacag ugacagagug cguuccauca cauuagguaa uuccacuauc accacccaag     1020
agugugccaa cguggucguc ggguacggag uguggccaga guaucuaaaa gacaacgagg     1080
ccacagccga agaucagccc acacaaccug auguggccac cugccgcuuc uauacuuuag     1140
aaucagugca guggaugaaa aauucggccg gguggguggug gaagcugccu gaugcacugu    1200
cacagauggg cuuguuuggu caaaacaugc aguaccacua cuugggaaga acaggguaua     1260
cuauacacgu acaaugcaau gccucaaagu uccaucaagg augccugcuu guagugugug     1320
ugccugaagc ugagaugggg ugcucuaacu ugaacaacac gcccgaauuc agugaguuau     1380
ccggaggaga cagugccaga auguucaccg auacacaggu cggggaguca aacgccaaga     1440
aaguacagac agcggugugg aacgccggaa ugggcguugg aguggguaau cucacuauuu     1500
ucccucacca auggaucaac cugaggacca acaacagugc aacgcuagug augccuuaca     1560
uaaacagcgu ccccauggau aacauguuca ggcacaauaa cuuaacacua augauuaucc     1620
cauuuguacc gcuuaauuac agcgagggau ccucaccgua cgugcccaua acagucacca     1680
ucgcuccuau gugugcagag uacaacgggc uacggcuggc cagcaaccag ggucuaccag     1740
uuaugacaac accugggagc acgcaauuuc uaacuucaga ugacuuccag ucaccgucag     1800
cgaugccaca guuugauguc accccagaga ugcaaauacc aggccgcgua aauaauuuga     1860
uggagauugc cgagguggac ucaguggugc cugugaauaa cacagaggac aacgugagua     1920
gccuuaaggc auaccagauu ccaguacagu ccaacaguga caaugguaag cagguuuuug     1980
guuuucccuu acaaccgggu gccaacaacg uucugaauag aacccucuug ggugaaauuc     2040
uaaacuauua cacccauugg aguggcagca uaaaacuuac auucauguuc ugugggucug     2100
ccauggccac uggcaaguuc cuucuggcau acucaccacc uggagcagga gucccgaaaa     2160
accgaaaaga ugcuaugcug ggcacccacg ucauauggga uguuggguua caaucaagcu     2220
gcguguugug cguaccgugg auuagccaaa cacacuacag auacgugguu gaggaugaau     2280
acacagcagc cggauauguu acaugcuggu aucaaacaaa uaucguggug ccagcugaug     2340
ugcaaagcuc augugacauc uugugcuuug uuucagccug caacgauuuc ucugugcgga     2400
ugcugaaaga uacgcccuuu auaagacagg acacauuuua ucacggccca guggaggagu     2460
cuguggaucg ugcaguggca cguguggcgg acacgauuag uucacggccc acuaacucgg     2520
agucaauucc agcgcucacc gccgccgaga cugggcauac cucccaaguu gugccuagcg     2580
acaccaugca aacaagacau gugaaaaacu accacucacg guccgaaucg uccauugaaa     2640
auuuccuaug ccggucugcc uguguauacu augccacaua caccaauaac ucaaaaaaag     2700
agggguuugc agaguggguu aucaacacua gacagguggc acagcuaaga agaaaguugg     2760
```

agcucuucac auaucuaagg uuugacuuag aacuaacauu uguuauaacg agcgcacaac 2820
aacccaguac cgccuccagu guagacgcuc ccgugcaaac ccaucagauu auguaugugc 2880
cuccaggugg cccuguacca acaaagguua aagauuaugc auggcaaacu uccacaaacc 2940
ccagcguuuu cuggacagaa ggaaacgccc caccaaggau guccauccca uucauuagca 3000
uugguaaugc guauaguugu uuuuaugaug gguggacgca guuuucaaga aacgggucu 3060
auggcaucaa uacccucaac aacaugggca cguuguauau gaggcauguc aaugaagcgg 3120
ggcaggguccc aaucaaaagu acuguuagaa uauauuucaa acccaagcac gugaaggcgu 3180
gggugccacg uccgccaaga uugugucaau augagaagca gaaaaauguc aauuuuagcc 3240
cuauaggagu aaccacaagu agaacggaca uuauaacaac aggcacuuuu ggucagcagu 3300
ccggagcgau auaugugggc aauuacagga uagucaauag gcaccuggca acacacuuug 3360
acuggcaaaa uugcauaugg gaggauuaca acagggaccu ccuaguaugc acuacaacgg 3420
cgcacgguug ugacaauauu gcaagaugcc agugcacagc agguguguau uauugugcuu 3480
cuaggaacaa gcauuacccg gugcauuuug aaggaccagg cuugguggag guccaggaaa 3540
gugaauauua uccaaaaagg uaucaaucac acgugcuucu ugccgcaggg uuuucugagc 3600
cgggggauug ugguggauuu cucaggugug aacauggugu uauaggcauc gugaccaugg 3660
gugguggaggg uguugucggc uuugccgaug ugcgcgaucu cuuauggcug gaagacgacg 3720
caauggaaca gggagucagg gacuacgugg aacagcuugg aaacgccuuu gguucugggu 3780
uuaccaacca gauuugcgag caagucaauc uguuaaaaga gucacuaaua ggccaagacu 3840
ccaucuugga aaaaucacuc aaggcauuag ugaaaaucau aucagcacug guuaucgugg 3900
ugagaaacca cgaugaccuc auaacaguca cugccaccuu agcccugauu ggcuguacca 3960
cuucaccgug gcgguggcua aagaaaaaag ugacucaaua cuaugggauu cccauggccg 4020
agagacagaa caauggcugg cucaagaaau ucacagaaau gaccaaugca ugcaagggca 4080
uggaauggau cgccaucaaa auccaaaagu ucauugagug gcucaaaauu aagaucuugc 4140
cagaaguaaa ggaaaaacac gaguuucuua ccagacuuaa gcaacuccca cucuuagaaa 4200
gccaaauugc caccaucgag cagagugcac caucacagag ugaucaggaa caacuguucu 4260
ccaauauaca guacuucgcg cacuauugca gaaaguacgc cccacuguac gcagcugagg 4320
caaaaagagu guucucucua gaaaagaaaa ugagcaacua cauacaguuc aaguccaaau 4380
gccguauuga accagucugu uuauugcucc augguagccc uggagccgga aaauccgugg 4440
cuaccaauuu gauuggacgc ucacuugcag agaagcucaa caguucagug uauucauugc 4500
caccagaccc agaccauuuu gacggcuaua aacaacaagc ugucguaauc auggaugacc 4560
ugugccagaa cccagauggg aaagacgugu ccuuguucug ccagaugguu ucaaguguag 4620
acuuuguucc accaauggca gcacuugagg agaaaggcau acucuucaca ucgccuuucg 4680

ugcucgccuc uaccaacgca gguuccauca acgcucccac ugugucagac agcagagccc 4740
uugcuagaag guuccacuuc gacuugaaca uugaggucau cucaauguac agccagaaug 4800
gcaaaaucaa caugccaaug ucagugaagg cuugugauga ugaguguugu ccaguuaauu 4860
ucaagaggug uuguccauua gugugcggua aggccauuca guucauugac agaagaacac 4920
aaaugaggua uucacuagac augcuuguaa cugaaauguu cagggaguac aaccacagac 4980
auaguguggg ggcaacacuc gaggcacucu uccaaggccc acccguguac aaagagauca 5040
agaucagugu ugcgccagaa accccucccc caccggcaau agcagaucug cugaaaucug 5100
uggacaguga agcaguuaga gaguacugca aggaaaaggg augguuggug ccugagauca 5160
auuccacucu acaaauugaa aagcacguga gcagggccuu cauuugccug caagcgcuca 5220
ccacguuugu uuccguggcg gggauaauuu acaucauaua caaauuguuu gcuggcuucc 5280
agggggccua caccgguaug ccaaaccaaa aaccuaaagu accaacuuug aggcaggcua 5340
aggugcaggg accggcauuc gaauucgcug uggcaaugau gaaaagaaau gcuagcacag 5400
ugaagaccga guacggugag uucacaaugc uuggaaucua ugacaggugg gcagugcuac 5460
cucgucacgc uagaccaggg ccuaccaucc uaaugaauga ccaggaagug gguguggugg 5520
augccaagga guuaguggac aaggauggca ccaauuuaga auuaacacuc uugaagcuca 5580
auagaaauga gaaauucaga gacauccgag gcuucuuaac acgugaggag gccgaaguca 5640
acgaggcugu gcuugcuauu aacaccagca aguucccaaa uauguacauu ccaguugggc 5700
aaguaacaga uuauggcuuc cucaacuugg guggcacacc aacuaagcgc augcugaugu 5760
acaacuuccc aacacgugca ggccaaugug gggaguucu aaugucuaca gguaaagugc 5820
ucggaauaca cgucggugcu aauggacacc aaggguuuuc ggcagcccug uuacgccacu 5880
auuucaauga cgagcaggga gagauugagu ucaucgagag cucuaaggau gcaggcuuuc 5940
cagucauuaa cacaccuagc aaaacaaagc ucgagcccag uguuuuccau cacguguuug 6000
agggaaacaa agaaccagca guguugagga auggagaccc aagacugaaa gccaauuuug 6060
aggaagccau uuucuccaag uacaucggga augugaacac acauguagac gaguacauga 6120
uggaagccgu cgaccauuau gcaggacagu uagcuacuuu agauaucagu acagaaccua 6180
ugaagcuuga ggaugcuguc uacgguacug agggcuugga agcucuggac uugaccacua 6240
gugcugggua ucccuauguc gcccuuggua uaaaaaagag agacaucuua uccaaaaagu 6300
cuaaggaccu gucuaaacug agagagugua uggacaagua uggguuaaac cucccuaugg 6360
ucaccuaugu gaaggaugaa uugaggucag cagagaaagu ggcuaagggc aaauccagac 6420
uaaucgaggc guccaguuua aaugacucag uggcaaugag acagacuuuc gguaaccugu 6480
auaagacauu ccaucugaac ccgggcauag ugacuggcag ugcaguugga ugcgaucccg 6540

acuuguucug gagcaagauu ccagucaugc ucgaugguca ccucaucgcc uuugacuaca 6600

guggauauga ugccagccug aguccggugu gguuugcuug cuugaaacug uuguuggaaa 6660

agcuggggua cucacacaag gaaacuaauu acauagauua cuugugcaac ucccaccauu 6720

uguacagaga caagcacuac uuugugaggg gaggaaugcc auccgggugu ucuggaacca 6780

gcauuuucaa uucaaugauu aauaacauaa ucaucagaac acugauguug aagguguaca 6840

aagggauaga ucuagaccaa uuuaggauga uugcauaugg ugacgacguc auugcuucgu 6900

acccguaucc aauugaugcg ucauugcugg cugaggcugg caagggguau ggucugauua 6960

ugacaccagc agacaaagga gaguguuuca augaaguaac guggaccaac gucacuuucc 7020

ugaaaagaua cuucagagcg gaugagcaau aucccuuccu cguucaccca gugaugccaa 7080

ugaaggacau ucaugaguca auuaggugga cuaaagaccc caaaaacacc caagaucacg 7140

ugcgauccuu gugcuuauug gccuggcaca auggagagca agaauaugag aauuuuguau 7200

ccaaaaucag aagcguccca guugggcgcu gcuugacauu gccugcguuc ucgacucugc 7260

gcaggaagug guuggauucc uucuaaaauu gaaguacaau gugauuuaac ucaauuggcu 7320

uaacccuacc acacuuaccg aacuagauaa cggugugua gggguaaauu c 7371

<210> 13
<211> 7251
<212> RNA
<213> Artificial Sequence

<220>
<223> Genomic sequence of CVB1-HRV2

<400> 13
uccugugggu ugaucccacc cacagggccc acugggcgcu agcacacugg uauuccggua 60

ccuuugugcg ccuguuuuau acacccuucc caaguguaac uuagaagaac uuagaaguuu 120

uucacaaaga ccaauagccg guaaucagcc agauuacuga aggucaagca cuucuguuuc 180

cccggucaau guugauaugc uccaacaggg caaaaacaac ugcgaucguu aaccgcaaag 240

cgccuacgca aagcuuagua gcaucuuuga aaucguuugg cuggucgauc cgccauuucc 300

ccugguagac cuggcagaug aggcuagaaa uaccccacug gcgacagugu ucuagccugc 360

guggcugccu gcacacccua uggguguguaa gccaaacaau ggacaaggug ugaagagccc 420

cgugugcucg cuuugagucc uccggccccu gaauguggcu aaccuuaacc cugcagcuag 480

agcacguaac ccaaugugua ucuagucgua augagcaauu gcgggauggg accaacuacu 540

uugggugucc guguuucacu uuuuccuuua uauuugcuua uggugacaau auauacaaua 600

uauauauugg caccauggga gcucaggugu cuacacaaaa gacgggugca caugagacug 660

gcuugaaugc cagcggcaau ucugucaucc auuacaccaa caucaauuac uacaaggaug 720

cugcauccaa cuccgcaaau aggcaagacu ucacacaaga cccagguaag uucacugaac 780

cuguaaagga caucauggug aaaacuaugc cugcguugaa cucacccucu gcugaggaau 840
gcggcuacag ugacagagug cguuccauca cauuagguaa uuccacuauc accacccaag 900
agugugccaa cguggucguc ggguacggag uguggccaga guaucuaaaa gacaacgagg 960
ccacagccga agaucagccc acacaaccug auguggccac cugccgcuuc uauacuuuag 1020
aaucagugca guggaugaaa aauucggccg gguggugug gaagcugccu gaugcacugu 1080
cacagauggg cuuguuuggu caaaacaugc aguaccacua cuugggaaga acaggguaua 1140
cuauacacgu acaaugcaau gccucaaagu uccaucaagg augccugcuu guagugugug 1200
ugccugaagc ugagaugggg ugcucuaacu ugaacaacac gcccgaauuc agugaguuau 1260
ccggaggaga cagugccaga auguucaccg auacacaggu cggggaguca aacgccaaga 1320
aaguacagac agcggugugg aacgccggaa ugggcguugg aguggguaau cucacuauuu 1380
ucccucacca auggaucaac cugaggacca acaacagugc aacgcuagug augccuuaca 1440
uaaacagcgu ccccauggau aacauguuca ggcacaauaa cuuaacacua augauuaucc 1500
cauuuguacc gcuuaauuac agcgagggau ccucaccgua cgugcccaua acagucacca 1560
ucgcuccuau gugugcagag uacaacgggc uacggcuggc cagcaaccag ggucuaccag 1620
uuaugacaac accugggagc acgcaauuuc uaacuucaga ugacuuccag ucaccgucag 1680
cgaugccaca guuugauguc accccagaga ugcaaauacc aggccgcgua aauaauuuga 1740
uggagauugc cgagguggac ucaguggugc cugugaauaa cacagaggac aacgugagua 1800
gccuuaaggc auaccagauu ccaguacagu ccaacaguga caaugguaag cagguuuuug 1860
guuuucccuu acaaccgggu gccaacaacg uucugaauag aacccucuug ggugaaauuc 1920
uaaacuauua cacccauugg aguggcagca uaaaacuuac auucauguuc ugugggucug 1980
ccauggccac uggcaaguuc cuucuggcau acucaccacc uggagcagga gucccgaaaa 2040
accgaaaaga ugcuaugcug ggcacccacg ucauauggga uguuggguua caaucaagcu 2100
gcguguugug cguaccgugg auuagccaaa cacacuacag auacgugguu gaggaugaau 2160
acacagcagc cggauauguu acaugcuggu aucaaacaaa uaucguggug ccagcugaug 2220
ugcaaagcuc augugacauc uugugcuuug uuucagccug caacgauuuc ucugugcgga 2280
ugcugaaaga uacgcccuuu auaagacagg acacauuuua ucacggccca guggaggagu 2340
cuguggaucg ugcaguggca cguguggcgg acacgauuag uucacggccc acuaacucgg 2400
agucaauucc agcgcucacc gccgccgaga cugggcauac cucccaaguu gugccuagcg 2460
acaccaugca aacaagacau gugaaaaacu accacucacg guccgaaucg uccauugaaa 2520
auuuccuaug ccggucugcc uguguauacu augccacaua caccaauaac ucaaaaaaag 2580
aggguuugc agagugguu aucaacacua gacagguggc acagcuaaga agaaaguugg 2640

agcucuucac auaucuaagg uuugacuuag aacuaacauu uguuauaacg agcgcacaac 2700
aacccaguac cgccuccagu guagacgcuc ccgugcaaac ccaucagauu auguaugugc 2760
cuccaggugg cccuguacca acaaagguua aagauuaugc auggcaaacu uccacaaacc 2820
ccagcguuuu cuggacagaa ggaaacgccc caccaaggau guccauccca uucauuagca 2880
uugguaaugc guauaguugu uuuuaugaug gguggacgca guuuucaaga aacggggucu 2940
auggcaucaa uacccucaac aacaugggca cguuguauau gaggcauguc aaugaagcgg 3000
ggcaggguccc aaucaaaagu acuguuagaa uauauuucaa acccaagcac gugaaggcgu 3060
gggugccacg uccgccaaga uugugucaau augagaagca gaaaaauguc aauuuuagcc 3120
cuauaggagu aaccacaagu agaacggaca uuauaacaac aggcacuuuu ggucagcagu 3180
ccggagcgau auaugugggc aauuacagga uagucaauag gcaccuggca acacacuuug 3240
acuggcaaaa uugcauaugg gaggauuaca acagggaccu ccuaguaugc acuacaacgg 3300
cgcacgguug ugacaauauu gcaagaugcc agugcacagc aggugugua uauugugcuu 3360
cuaggaacaa gcauuacccg gugcauuuug aaggaccagg cuugguggag guccaggaaa 3420
gugaauauua uccaaaaagg uaucaaucac acgugcuucu ugccgcaggg uuuucugagc 3480
cgggggauug ugguggauu cucaggugug aacauggugu uauaggcauc gugaccaugg 3540
guggugaggg uguugucggc uuugccgaug ugcgcgaucu cuuauggcug gaagacgacg 3600
caauggaaca gggagucagg gacuacgugg aacagcuugg aaacgccuuu gguucugggu 3660
uuaccaacca gauuugcgag caagucaauc uguuaaaaga gucacuaaua ggccaagacu 3720
ccaucuugga aaaaucacuc aaggcauuag ugaaaaucau aucagcacug guuaucgugg 3780
ugagaaacca cgaugaccuc auaacaguca cugccaccuu agcccugauu ggcuguacca 3840
cuucaccgug gcgguggcua aagaaaaaag ugacucaaua cuaugggauu cccauggccg 3900
agagacagaa caauggcugg cucaagaaau ucacagaaau gaccaaugca ugcaagggca 3960
uggaauggau cgccaucaaa auccaaaagu ucauugagug gcucaaaauu aagaucuugc 4020
cagaaguaaa ggaaaaacac gaguuucuua ccagacuuaa gcaacuccca cucuuagaaa 4080
gccaaauugc caccaucgag cagagugcac caucacagag ugaucaggaa caacuguucu 4140
ccaauauaca guacuucgcg cacuauugca gaaaguacgc cccacuguac gcagcugagg 4200
caaaaagagu guucucucua gaaaagaaaa ugagcaacua cauacaguuc aaguccaaau 4260
gccguauuga accagucugu uuauugcucc augguagccc uggagccgga aaauccgugg 4320
cuaccaauuu gauuggacgc ucacuugcag agaagcucaa caguucagug uauucauugc 4380
caccagaccc agaccauuuu gacggcuaua aacaacaagc ugucguaauc auggaugacc 4440
ugugccagaa cccagauggg aaagacgugu ccuuguucug ccagaugguu ucaaguguag 4500
acuuuguucc accaauggca gcacuugagg agaaaggcau acucuucaca ucgccuuucg 4560

ugcucgccuc uaccaacgca gguuccauca acgcucccac ugugucagac agcagagccc 4620
uugcuagaag guuccacuuc gacuugaaca uugaggucau cucaauguac agccagaaug 4680
gcaaaaucaa caugccaaug ucagugaagg cuugugauga ugaguguugu ccaguuaauu 4740
ucaagaggug uuguccauua gugugcggua aggccauuca guucauugac agaagaacac 4800
aaaugaggua uucacuagac augcuuguaa cugaaauguu cagggaguac aaccacagac 4860
auagugugggg ggcaacacuc gaggcacucu uccaaggccc acccguguac aaagagauca 4920
agaucagugu ugcgccagaa accccucccc caccggcaau agcagaucug cugaaaucug 4980
uggacaguga agcaguuaga gaguacugca aggaaaaggg augguuggug ccugagauca 5040
auuccacucu acaaauugaa aagcacguga gcagggccuu cauuugccug caagcgcuca 5100
ccacguuugu uuccguggcg gggauaauuu acaucauaua caaauuguuu gcuggcuucc 5160
agggggccua caccgguaug ccaaaccaaa aaccuaaagu accaacuuug aggcaggcua 5220
aggugcaggg accggcauuc gaauucgcug uggcaaugau gaaaagaaau gcuagcacag 5280
ugaagaccga guacggugag uucacaaugc uuggaaucua ugacaggugg gcagugcuac 5340
cucgucacgc uagaccaggg ccuaccaucc uaaugaauga ccaggaagug ggugguggg 5400
augccaagga guuaguggac aaggauggca ccaauuuaga auuaacacuc uugaagcuca 5460
auagaaauga gaaauucaga gacauccgag gcuucuuaac acgugaggag gccgaaguca 5520
acgaggcugu gcuugcuauu aacaccagca aguucccaaa uauguacauu ccaguugggc 5580
aaguaacaga uuauggcuuc cucaacuugg guggcacacc aacuaagcgc augcugaugu 5640
acaacuuccc aacacgugca ggccaaugug ggggaguucu aaugucuaca gguaaagugc 5700
ucggaauaca cgucgguggu aauggacacc aaggguuuuc ggcagcccug uuacgccacu 5760
auuucaauga cgagcaggga gagauugagu ucaucgagag cucuaaggau gcaggcuuuc 5820
cagucauuaa cacaccuagc aaaacaaagc ucgagcccag uguuuuccau cacguguuug 5880
agggaaacaa agaaccagca guguugagga auggagaccc aagacugaaa gccaauuuug 5940
aggaagccau uuucuccaag uacaucggga augugaacac acauguagac gaguacauga 6000
uggaagccgu cgaccauuau gcaggacagu uagcuacuuu agauaucagu acagaaccua 6060
ugaagcuuga ggaugcuguc uacgguacug agggcuugga agcucuggac uugaccacua 6120
gugcuggggua ucccuauguc gcccuuggua uaaaaaagag agacaucuua uccaaaaagu 6180
cuaaggaccu gucuaaacug agagagugua uggacaagua uggguuaaac cucccuaugg 6240
ucaccuaugu gaaggaugaa uugaggucag cagagaaagu ggcuaagggc aaauccagac 6300
uaaucgaggc guccaguuua aaugacucag uggcaaugag acagacuuuc gguaaccugu 6360
auaagacauu ccaucugaac ccgggcauag ugacuggcag ugcaguugga ugcgaucccg 6420

acuuguucug gagcaagauu ccagucaugc ucgaugguca ccucaucgcc uuugacuaca 6480

guggauauga ugccagccug aguccggugu gguuugcuug cuugaaacug uuguuggaaa 6540

agcuggggua cucacacaag gaaacuaauu acauagauua cuugugcaac ucccaccauu 6600

uguacagaga caagcacuac uuugugaggg gaggaaugcc auccgggugu ucuggaacca 6660

gcauuuucaa uucaaugauu aauaacauaa ucaucagaac acugauguug aagguguaca 6720

aagggauaga ucuagaccaa uuuaggauga uugcauaugg ugacgacguc auugcuucgu 6780

acccguaucc aauugaugcg ucauugcugg cugaggcugg caaggggua ggucugauua 6840

ugacaccagc agacaaagga gaguguuuca augaaguaac guggaccaac gucacuuucc 6900

ugaaaagaua cuucagagcg gaugagcaau aucccuuccu cguucaccca gugaugccaa 6960

ugaaggacau ucaugaguca auuaggugga cuaaagaccc caaaaacacc caagaucacg 7020

ugcgauccuu gugcuuauug gccuggcaca auggagagca agaauaugag aauuuuguau 7080

ccaaaaucag aagcguccca guugggcgcu gcuugacauu gccugcguuc ucgacucugc 7140

gcaggaagug guuggauucc uucuaaaauu gaaguacaau gugauuuaac ucaauuggcu 7200

uaacccuacc acacuuaccg aacuagauaa cggugugua gggguaaauu c 7251

<210> 14
<211> 7473
<212> RNA
<213> Artificial Sequence

<220>
<223> Genomic sequence of CVB1-miR133&206T

<400> 14
uccugugggu ugaucccacc cacagggccc acugggcgcu agcacacugg uauuccggua 60

ccuuugugcg ccuguuuuau acacccuucc caaguguaac uuagaagcuu aucacacacg 120

gucaacaggu gacucaguau gccaacuggg ucuugaucaa gcacuucugu uuccccggac 180

ugaguaucaa uaagcugcuc acgcggcuga aggagaaaac guucguuaac cggccaauua 240

cuucgagaaa ccuaguaaca ccauggaggu ugcgcagugu uucgcuccac acaaccccag 300

uguagaucag gccgaugagu caccgcacuc cucacgggcg accguggcgg uggcugcgcu 360

ggcggccugc ccaugggaua acccauggga cgcuucaaua cugacauggu gcgaagaguc 420

uauugagcua auugguaguc cuccggcccc ugaaugcggc uaauccuaac ugcggagcag 480

auacccacac gccagugggc agucugucgu aaugggcaac ucugcagcgg aaccgacuac 540

uuugggguguc cguguuuccu uuuauuuuua uacuggcugc uuauggugac aauugagaga 600

uuguuaccau auagcuauug gauuggccau ccagugacaa acagagcgau uauuuauuua 660

uuuguugguu auaucccauu aagccaaaag gcccuaguua cucucaacuu uauacuauug 720

cuuaauacaa cgaaauggga gcucaggugu cuacacaaaa gacgggugca caugagacug 780

gcuugaaugc cagcggcaau ucugucaucc auuacaccaa caucaauuac uacaaggaug 840
cugcauccaa cuccgcaaau aggcaagacu ucacacaaga cccagguaag uucacugaac 900
cuguaaagga caucauggug aaaacuaugc cugcguugaa cucacccucu gcugaggaau 960
gcggcuacag ugacagagug cguuccauca cauuagguaa uuccacuauc accacccaag 1020
agugugccaa cguggucguc ggguacggag uguggccaga guaucuaaaa gacaacgagg 1080
ccacagccga agaucagccc acacaaccug auguggccac cugccgcuuc uauacuuuag 1140
aaucagugca guggaugaaa aauucggccg gguggguggug gaagcugccu gaugcacugu 1200
cacagauggg cuuguuuggu caaaacaugc aguaccacua cuugggaaga acaggguaua 1260
cuauacacgu acaaugcaau gccucaaagu uccaucaagg augccugcuu guagugugug 1320
ugccugaagc ugagaugggg ugcucuaacu ugaacaacac gcccgaauuc agugaguuau 1380
ccggaggaga cagugccaga auguucaccg auacacaggu cggggaguca aacgccaaga 1440
aaguacagac agcggugugg aacgccggaa ugggcguugg agugggguaau cucacuauuu 1500
ucccucacca auggaucaac cugaggacca acaacagugc aacgcuagug augccuuaca 1560
uaaacagcgu ccccauggau aacauguuca ggcacaauaa cuuaacacua augauuaucc 1620
cauuuguacc gcuuaauuac agcgagggau ccucaccgua cgugcccaua acagucacca 1680
ucgcuccuau gugugcagag uacaacgggc uacggcuggc cagcaaccag ggucuaccag 1740
uuaugacaac accugggagc acgcaauuuc uaacuucaga ugacuuccag ucaccgucag 1800
cgaugccaca guuugauguc accccagaga ugcaaauacc aggccgcgua aauaauuuga 1860
uggagauugc cgagguggac ucaguggugc cugugaauaa cacagaggac aacgugagua 1920
gccuuaaggc auaccagauu ccaguacagu ccaacaguga caaugguaag cagguuuuug 1980
guuuucccuu acaaccgggu gccaacaacg uucugaauag aacccucuug ggugaaauuc 2040
uaaacuauua cacccauugg aguggcagca uaaaacuuac auucauguuc ugugggucug 2100
ccauggccac uggcaaguuc cuucuggcau acucaccacc uggagcagga gucccgaaaa 2160
accgaaaaga ugcuaugcug ggcacccacg ucauauggga uguuggguua caaucaagcu 2220
gcguguugug cguaccgugg auuagccaaa cacacuacag auacgugguu gaggaugaau 2280
acacagcagc cggauauguu acaugcuggu aucaaacaaa uaucguggug ccagcugaug 2340
ugcaaagcuc augugacauc uugugcuuug uuucagccug caacgauuuc ucugugcgga 2400
ugcugaaaga uacgcccuuu auaagacagg acacauuuua ucacggccca guggaggagu 2460
cuguggaucg ugcaguggca cgugugggcgg acacgauuag uucacggccc acuaacucgg 2520
agucaauucc agcgcucacc gccgccgaga cugggcauac cucccaaguu gugccuagcg 2580
acaccaugca aacaagacau gugaaaaacu accacucacg guccgaaucg uccauugaaa 2640

auuuccuaug ccggucugcc uguguauacu augccacaua caccaauaac ucaaaaaaag 2700
aggggguuugc agaguggguu aucaacacua gacaggugggc acagcuaaga agaaaguugg 2760
agcucuucac auaucuaagg uuugacuuag aacuaacauu uguuauaacg agcgcacaac 2820
aacccaguac cgccuccagu guagacgcuc ccgugcaaac ccaucagauu auguaugugc 2880
cuccaggugg cccuguacca acaaagguua aagauuaugc auggcaaacu uccacaaacc 2940
ccagcguuuu cuggacagaa ggaaacgccc caccaaggau guccauccca uucauuagca 3000
uugguaaugc guauaguugu uuuuaugaug gguggacgca guuuucaaga aacggggucu 3060
auggcaucaa uacccucaac aacaugggca cguuguauau gaggcauguc aaugaagcgg 3120
ggcagggucc aaucaaaagu acuguuagaa uauauuucaa acccaagcac gugaaggcgu 3180
gggugccacg uccgccaaga uugugucaau augagaagca gaaaaauguc aauuuuagcc 3240
cuauaggagu aaccacaagu agaacggaca uuauaacaac aggcacuuuu ggucagcagu 3300
ccggagcgau auauguggggc aauuacagga uagucaauag gcaccuggca acacacuuug 3360
acuggcaaaa uugcauaugg gaggauuaca acagggaccu ccuaguaugc acuacaacgg 3420
cgcacgguug ugacaauauu gcaagaugcc agugcacagc agguguguau uauugugcuu 3480
cuaggaacaa gcauuacccg gugcauuuug aaggaccagg cuugguggag guccaggaaa 3540
gugaauauua uccaaaaagg uaucaaucac acgugcuucu ugccgcaggg uuuucugagc 3600
cgggggauug ugggugguauu cucaggugug aacauggugu uauaggcauc gugaccaugg 3660
guggugaggg uguugucggc uuugccgaug ugcgcgaucu cuuauggcug gaagacgacg 3720
caauggaaca gggagucagg gacuacgugg aacagcuugg aaacgccuuu gguucugggu 3780
uuaccaacca gauuugcgag caagucaauc uguuaaaaga gucacuaaua ggccaagacu 3840
ccaucuugga aaaaucacuc aaggcauuag ugaaaaucau aucagcacug guuaucgugg 3900
ugagaaacca cgaugaccuc auaacaguca cugccaccuu agcccugauu ggcuguacca 3960
cuucaccgug gcgguggcua aagaaaaaag ugacucaaua cuaugggauu cccauggccg 4020
agagacagaa caauggcugg cucaagaaau ucacagaaau gaccaaugca ugcaagggca 4080
uggaauggau cgccaucaaa auccaaaagu ucauugagug gcucaaaauu aagaucuugc 4140
cagaaguaaa ggaaaaacac gaguuucuua ccagacuuaa gcaacuccca cucuuagaaa 4200
gccaaauugc caccaucgag cagagugcac caucacagag ugaucaggaa caacuguucu 4260
ccaauauaca guacuucgcg cacuauugca gaaaguacgc cccacuguac gcagcugagg 4320
caaaaagagu guucucucua gaaaagaaaa ugagcaacua cauacaguuc aaguccaaau 4380
gccguauuga accagucugu uuauugcucc augguagccc uggagccgga aaauccgugg 4440
cuaccaauuu gauuggacgc ucacuugcag agaagcucaa caguucagug uauucauugc 4500
caccagaccc agaccauuuu gacggcuaua aacaacaagc ugucguaauc auggaugacc 4560

ugugccagaa cccagauggg aaagacgugu ccuuguucug ccagaugguu ucaaguguag 4620
acuuuguucc accaauggca gcacuugagg agaaaggcau acucuucaca ucgccuuucg 4680
ugcucgccuc uaccaacgca gguuccauca acgcucccac ugugucagac agcagagccc 4740
uugcuagaag guuccacuuc gacuugaaca uugaggucau cucaauguac agccagaaug 4800
gcaaaaucaa caugccaaug ucagugaagg cuugugauga ugaguguugu ccaguuaauu 4860
ucaagaggug uuguccauua gugugcggua aggccauuca guucauugac agaagaacac 4920
aaaugaggua uucacuagac augcuuguaa cugaaauguu cagggaguac aaccacagac 4980
auagugugg gcaacacuc gaggcacucu uccaaggccc acccguguac aaagagauca 5040
agaucagugu ugcgccagaa accccucccc caccggcaau agcagaucug cugaaaucug 5100
uggacaguga agcaguuaga gaguacugca aggaaaaggg augguuggug ccugagauca 5160
auuccacucu acaaauugaa aagcacguga gcagggccuu cauuugccug caagcgcuca 5220
ccacguuugu uuccguggcg gggauaauuu acaucauaua caaauuguuu gcuggcuucc 5280
agggggccua caccgguaug ccaaaccaaa aaccuaaagu accaacuuug aggcaggcua 5340
aggugcaggg accggcauuc gaauucgcug uggcaaugau gaaaagaaau gcuagcacag 5400
ugaagaccga guacggugag uucacaaugc uuggaaucua ugacaggugg gcagugcuac 5460
cucgucacgc uagaccaggg ccuaccaucc uaaugaauga ccaggaagug ggugugugg 5520
augccaagga guuaguggac aaggauggca ccaauuuaga auuaacacuc uugaagcuca 5580
auagaaauga gaaauucaga gacauccgag gcuucuuaac acgugaggag gccgaaguca 5640
acgaggcugu gcuugcuauu aacaccagca aguucccaaa uauguacauu ccaguugggc 5700
aaguaacaga uuauggcuuc cucaacuugg guggcacacc aacuaagcgc augcugaugu 5760
acaacuuccc aacacgugca ggccaaugug gggaguucu aaugucuaca gguaaagugc 5820
ucggaauaca cgucggugu aauggacacc aaggguuuuc ggcagcccug uuacgccacu 5880
auuucaauga cgagcaggga gagauugagu ucaucgagag cucuaaggau gcaggcuuuc 5940
cagucauuaa cacaccuagc aaaacaaagc ucgagcccag uguuuuccau cacguguuug 6000
agggaaacaa agaaccagca guguugagga auggagaccc aagacugaaa gccaauuuug 6060
aggaagccau uuucuccaag uacaucggga augugaacac acauguagac gaguacauga 6120
uggaagccgu cgaccauuau gcaggacagu uagcuacuuu agauaucagu acagaaccua 6180
ugaagcuuga ggaugcuguc uacgguacug agggcuugga agcucuggac uugaccacua 6240
gugcugggua ucccuauguc gcccuuggua uaaaaaagag agacaucuua uccaaaaagu 6300
cuaaggaccu gucuaaacug agagagugua uggacaagua uggguuaaac cucccuaugg 6360
ucaccuaugu gaaggaugaa uugaggucag cagagaaagu ggcuaagggc aaauccagac 6420

uaaucgaggc guccaguuua aaugacucag uggcaaugag acagacuuuc gguaaccugu 6480

auaagacauu ccaucugaac ccgggcauag ugacuggcag ugcaguugga ugcgaucccg 6540

acuuguucug gagcaagauu ccagucaugc ucgaugguca ccucaucgcc uuugacuaca 6600

guggauauga ugccagccug aguccggugu gguuugcuug cuugaaacug uuguuggaaa 6660

agcuggggua cucacacaag gaaacuaauu acauagauua cuugugcaac ucccaccauu 6720

uguacagaga caagcacuac uuugugaggg gaggaaugcc auccgggugu ucuggaacca 6780

gcauuuucaa uucaaugauu aauaacauaa ucaucagaac acugauguug aagguguaca 6840

aagggauaga ucuagaccaa uuuaggauga uugcauaugg ugacgacguc auugcuucgu 6900

acccguaucc aauugaugcg ucauugcugg cugaggcugg caagggguau ggucugauua 6960

ugacaccagc agacaaagga gaguguuuca augaaguaac guggaccaac gucacuuucc 7020

ugaaaagaua cuucagagcg gaugagcaau aucccuuccu cguucaccca gugaugccaa 7080

ugaaggacau ucaugaguca auuaggugga cuaaagaccc caaaaacacc caagaucacg 7140

ugcgauccuu gugcuuauug gccuggcaca auggagagca agaauaugag aauuuuguau 7200

ccaaaaucag aagcguccca guugggcgcu gcuugacauu gccugcguuc ucgacucugc 7260

gcaggaagug guuggauucc uucuaaaaua cagcugguug aaggggacca acgauacagc 7320

ugguugaagg ggaccaaacc gguccacaca cuuccuuaca uuccaucacc cacacacuuc 7380

cuuacauucc augaaguaca augugauuua acucaauugg cuuaacccua ccacacuuac 7440

cgaacuagau aacggugugg uaggggguaaa uuc 7473

<210> 15
<211> 7848
<212> RNA
<213> Artificial Sequence

<220>
<223> Genomic sequence of CVB1-GM-CSF

<400> 15
uccugugggu ugaucccacc cacagggccc acugggcgcu agcacacugg uauuccggua 60

ccuuugugcg ccuguuuuau acacccuucc caaguguaac uuagaagcuu aucacacacg 120

gucaacaggu gacucaguau gccaacuggg ucuugaucaa gcacuucugu uuccccggac 180

ugaguaucaa uaagcugcuc acgcggcuga aggagaaaac guucguuaac cggccaauua 240

cuucgagaaa ccuaguaaca ccauggaggu ugcgcagugu uucgcuccac acaaccccag 300

uguagaucag gccgaugagu caccgcacuc cucacgggcg accguggcgg uggcugcgcu 360

ggcggccugc ccaugggaua acccauggga cgcuucaaua cugacauggu gcgaagaguc 420

uauugagcua auugguaguc cuccggcccc ugaaugcggc uaauccuaac ugcggagcag 480

auacccacac gccagugggc agucugucgu aaugggcaac ucugcagcgg aaccgacuac 540

uuugggguc cguguuuccu uuuauuuuua uacuggcugc uuauggugac aauugagaga 600
uuguuaccau auagcuauug gauuggccau ccagugacaa acagagcgau uauuuauuua 660
uuuguugguu auaucccauu aagccaaaag gcccuaguua cucucaacuu uauacuauug 720
cuuaauacaa cgaaauggga gcucaggugu cuacacaaaa gacgggugca caugagacug 780
gcuugaaugc cagcggcaau ucugucaucc auuacaccaa caucaauuac uacaaggaug 840
cugcauccaa cuccgcaaau aggcaagacu ucacacaaga cccagguaag uucacugaac 900
cuguaaagga caucauggug aaaacuaugc cugcguugaa cucacccucu gcugaggaau 960
gcggcuacag ugacagagug cguuccauca cauuagguaa uuccacuauc accacccaag 1020
agugugccaa cgugguc guc gguacggag uguggccaga guaucuaaaa gacaacgagg 1080
ccacagccga agaucagccc acacaaccug auguggccac cugccgcuuc uauacuuuag 1140
aaucagugca guggaugaaa aauucggccg gguggugggug gaagcugccu gaugcacugu 1200
cacagauggg cuuguuuggu caaaacaugc aguaccacua cuugggaaga acaggguaua 1260
cuauacacgu acaaugcaau gccucaaagu uccaucaagg augccugcuu guagugugug 1320
ugccugaagc ugagaugggg ugcucuaacu ugaacaacac gcccgaauuc agugaguuau 1380
ccggaggaga cagugccaga auguucaccg auacacaggu cggggaguca aacgccaaga 1440
aaguacagac agcggugugg aacgccggaa ugggcguugg agugggguaau cucacuauuu 1500
ucccucacca auggaucaac cugaggacca acaacagugc aacgcuagug augccuuaca 1560
uaaacagcgu ccccauggau aacauguuca ggcacaauaa cuuaacacua augauuaucc 1620
cauuuguacc gcuuaauuac agcgagggau ccucaccgua cgugcccaua acagucacca 1680
ucgcuccuau gugugcagag uacaacgggc uacggcuggc cagcaaccag ggucuaccag 1740
uuaugacaac accugggagc acgcaauuuc uaacuucaga ugacuuccag ucaccgucag 1800
cgaugccaca guuugauguc accccagaga ugcaaauacc aggccgcgua aauaauuuga 1860
uggagauugc cgagguggac ucaguggugc cugugaauaa cacagaggac aacgugagua 1920
gccuuaaggc auaccagauu ccaguacagu ccaacaguga caaugguaag cagguuuuug 1980
guuuucccuu acaaccgggu gccaacaacg uucugaauag aacccucuug ggugaaauuc 2040
uaaacuauua cacccauugg aguggcagca uaaaacuuac auucauguuc ugugggucug 2100
ccauggccac uggcaaguuc cuucuggcau acucaccacc uggagcagga gucccgaaaa 2160
accgaaaaga ugcuaugcug ggcacccacg ucauauggga uguuggguua caaucaagcu 2220
gcguguugug cguaccgugg auuagccaaa cacacuacag auacgugguu gaggaugaau 2280
acacagcagc cggauauguu acaugcuggu aucaaacaaa uaucguggug ccagcugaug 2340
ugcaaagcuc augugacauc uugugcuuug uuucagccug caacgauuuc ucugugcgga 2400

ugcugaaaga uacgcccuuu auaagacagg acacauuuua ucacggccca guggaggagu 2460
cuguggaucg ugcaguggca cguguggcgg acacgauuag uucacggccc acuaacucgg 2520
agucaauucc agcgcucacc gccgccgaga cugggcauac cucccaaguu gugccuagcg 2580
acaccaugca aacaagacau gugaaaaacu accacucacg guccgaaucg uccauugaaa 2640
auuuccuaug ccggucugcc uguguauacu augccacaua caccaauaac ucaaaaaaag 2700
aggguuugc agagugggu aucaacacua gacagguggc acagcuaaga agaaaguugg 2760
agcucuucac auaucuaagg uuugacuuag aacuaacauu uguuauaacg agcgcacaac 2820
aacccaguac cgccuccagu guagacgcuc ccgugcaaac ccaucagauu auguaugugc 2880
cuccaggugg cccuguacca acaaagguua aagauuaugc auggcaaacu uccacaaacc 2940
ccagcguuuu cuggacagaa ggaaacgccc caccaaggau guccauccca uucauuagca 3000
uugguaaugc guauaguugu uuuuaugaug gguggacgca guuuucaaga aacgggucu 3060
auggcaucaa uacccucaac aacaugggca cguuguauau gaggcauguc aaugaagcgg 3120
ggcaggucc aaucaaaagu acuguuagaa uauauuucaa acccaagcac gugaaggcgu 3180
gggugccacg uccgccaaga uugugucaau augagaagca gaaaaauguc aauuuuagcc 3240
cuauaggagu aaccacaagu agaacggaca uuauaacaac aggcacuuuu ggucagcagu 3300
ggcugcagag ccugcugcuc uugggcacug uggccugcag caucucugca cccgcccgcu 3360
cgcccagccc cagcacgcag cccugggagc augugaaugc cauccaggag gcccggcguc 3420
uccugaaccu gaguagagac acugcugcug agaugaauga aacaguagaa gucaucucag 3480
aaauguuuga ccuccaggag ccgaccugcc uacagacccg ccuggagcug uacaagcagg 3540
gccugcgggg cagccucacc aagcucaagg gccccuugac caugauggcc agccacuaca 3600
agcagcacug cccuccaacc ccggaaacuu ccugugcaac ccagauuauc accuuugaaa 3660
guuucaaaga gaaccugaag gacuuucugc uugucauccc cuuugacugc ugggagccag 3720
uccaggagac cacaaguaga acggacauua uaacaacagg cacuuuuggu cagcaguccg 3780
gagcgauaua ugugggcaau uacaggauag ucaauaggca ccuggcaaca cacuuugacu 3840
ggcaaaauug cauaugggag gauuacaaca gggaccuccu aguaugcacu acaacggcgc 3900
acgguuguga caauauugca agaugccagu gcacagcagg uguguauuau ugugcuucua 3960
ggaacaagca uuacccggug cauuuugaag gaccaggcuu gguggagguc caggaaagug 4020
aauauuaucc aaaaagguau caaucacacg ugcuucuugc cgcaggguuu ucugagccgg 4080
gggauugugg ugguauucuc aggugugaac auggguguau aggcaucgug accaugggug 4140
gugagggugu ugucggcuuu gccgaugugc gcgaucucuu auggcuggaa gacgacgcaa 4200
uggaacaggg agucagggac uacguggaac agcuuggaaa cgccuuuggu ucuggguuua 4260
ccaaccagau uugcgagcaa gucaaucugu uaaaagaguc acuaauaggc caagacucca 4320

ucuuggaaaa aucacucaag gcauuaguga aaaucauauc agcacugguu aucguggugA 4380
gaaaccacga ugaccucaua acagucacug ccaccuuagc ccugauuggc uguaccacuu 4440
caccguggcg guggcuaaag aaaaaaguga cucaauacua ugggauuccc auggccgaga 4500
gacagaacaa uggcuggcuc aagaaauuca cagaaaugac caaugcaugc aagggcaugg 4560
aauggaucgc caucaaaauc caaaaguuca uugaguggcu caaaauuaag aucuugccag 4620
aaguaaagga aaaacacgag uuucuuacca gacuuaagca acucccacuc uuagaaagcc 4680
aaauugccac caucgagcag agugcaccau cacagaguga ucaggaacaa cuguucucca 4740
auauacagua cuucgcgcac uauugcagaa aguacgcccc acuguacgca gcugaggcaa 4800
aaagaguguu cucucuagaa aagaaaauga gcaacuacau acaguucaag uccaaaugcc 4860
guauugaacc agucuguuua uugcuccaug guagcccugg agccggaaaa uccguggcua 4920
ccaauuugau uggacgcuca cuugcagaga agcucaacag uucaguguau ucauugccac 4980
cagacccaga ccauuuugac ggcuauaaac aacaagcugu cguaaucaug gaugaccugu 5040
gccagaaccc agaugggaaa gacguguccu uguucugcca gaugguuuca aguguagacu 5100
uuguuccacc aauggcagca cuugaggaga aaggcauacu cuucacaucg ccuuucgugc 5160
ucgccucuac caacgcaggu uccaucaacg cucccacugu gucagacagc agagcccuug 5220
cuagaagguu ccacuucgac uugaacauug aggucaucuc aauguacagc cagaauggca 5280
aaaucaacau gccaauguca gugaaggcuu gugaugauga guguugucca guuaauuuca 5340
agagguguug uccauuagug ugcgguaagg ccauucaguu cauugacaga agaacacaaa 5400
ugagguauuc acuagacaug cuuguaacug aaauguucag ggaguacaac cacagacaua 5460
gugugggggc aacacucgag gcacucuucc aaggcccacc cguguacaaa gagaucaaga 5520
ucaguguugc gccagaaacc ccuccccac cggcaauagc agaucugcug aaaucugugg 5580
acagugaagc aguuagagag uacugcaagg aaaagggaug guuggugccu gagaucaauu 5640
ccacucuaca aauugaaaag cacgugagca gggccuucau uugccugcaa gcgcucacca 5700
cguuuguuuc cguggcgggg auaauuuaca ucauauacaa auuguuugcu ggcuuccagg 5760
gggccuacac cgguaugcca aaccaaaaac cuaaaguacc aacuuugagg caggcuaagg 5820
ugcagggacc ggcauucgaa uucgcugugg caaugaugaa aagaaaugcu agcacaguga 5880
agaccgagua cggugaguuc acaaugcuug gaaucuauga cagguggca gugcuaccuc 5940
gucacgcuag accagggccu accauccuaa ugaaugacca ggaagugggu gugguggaug 6000
ccaaggaguu aguggacaag gauggcacca auuuagaauu aacacucuug aagcucaaua 6060
gaaaugagaa auucagagac auccgaggcu ucuuaacacg ugaggaggcc gaagucaacg 6120
aggcugugcu ugcuauuaac accagcaagu ucccaaauau guacauucca guuggcaag 6180

uaacagauua uggcuuccuc aacuugggug gcacaccaac uaagcgcaug cugauguaca 6240

acuucccaac acgugcaggc caaugugggg gaguucuaau gucuacaggu aaagugcucg 6300

gaauacacgu cggugguaau ggacaccaag gguuuucggc agcccuguua cgccacuauu 6360

ucaaugacga gcagggagag auugaguuca ucgagagcuc uaaggaugca ggcuuuccag 6420

ucauuaacac accuagcaaa acaaagcucg agcccagugu uuuccaucac guguuugagg 6480

gaaacaaaga accagcagug uugaggaaug gagacccaag acugaaagcc aauuuugagg 6540

aagccauuuu cuccaaguac aucgggaaug ugaacacaca uguagacgag uacaugaugg 6600

aagccgucga ccauuaugca ggacaguuag cuacuuuaga uaucaguaca gaaccuauga 6660

agcuugagga ugcugucuac gguacugagg gcuuggaagc ucuggacuug accacuagug 6720

cuggguaucc cuaugucgcc cuugguauaa aaaagagaga caucuuaucc aaaaagucua 6780

aggaccuguc uaaacugaga gaguguaugg acaaguaugg guuaaaccuc ccuaugguca 6840

ccuaugugaa ggaugaauug aggucagcag agaaaguggc uaagggcaaa uccagacuaa 6900

ucgaggcguc caguuuaaau gacucagugg caaugagaca gacuuucggu aaccuguaua 6960

agacauucca ucugaacccg ggcauaguga cuggcagugc aguuggaugc gaucccgacu 7020

uguucuggag caagauucca gucaugcucg auggucaccu caucgccuuu gacuacagug 7080

gauaugaugc cagccugagu ccggugugguu uugcuugcuu gaaacuguug uuggaaaagc 7140

uggguacuc acacaaggaa acuaauuaca uagauuacuu gugcaacucc caccauuugu 7200

acagagacaa gcacuacuuu gugaggggag gaaugccauc cggguguucu ggaaccagca 7260

uuuucaauuc aaugauuaau aacauaauca ucagaacacu gauguugaag guguacaaag 7320

ggauagaucu agaccaauuu aggaugauug cauauggguga cgacgucauu gcuucguacc 7380

cguauccaau ugaugcguca uugcuggcug aggcuggcaa gggguauggu cugauuauga 7440

caccagcaga caaaggagag uguuucaaug aaguaacgug gaccaacguc acuuuccuga 7500

aaagauacuu cagagcggau gagcaauauc ccuuccucgu ucacccagug augccaauga 7560

aggacauuca ugagucaauu agguggacua aagaccccaa aaacacccaa gaucacgugc 7620

gauccuugug cuuauuggcc uggcacaaug gagagcaaga auaugagaau uuuguaucca 7680

aaaucagaag cgucccaguu gggcgcugcu ugacauugcc ugcguucucg acucugcgca 7740

ggaagugguu ggauuccuuc uaaaauugaa guacaaugug auuuaacuca auuggcuuaa 7800

cccuaccaca cuuaccgaac uagauaacgg ugugguaggg guaaauuc 7848

<210> 16
<211> 8214
<212> RNA
<213> Artificial Sequence

<220>

<223> Genomic sequence of CVB1-Anti-PD1

<400> 16
uccugugggu ugaucccacc cacagggccc acugggcgcu agcacacugg uauuccggua 60

ccuuugugcg ccuguuuuau acacccuucc caaguguaac uuagaagcuu aucacacacg 120

gucaacaggu gacucaguau gccaacuggg ucuugaucaa gcacuucugu uuccccggac 180

ugaguaucaa uaagcugcuc acgcggcuga aggagaaaac guucguuaac cggccaauua 240

cuucgagaaa ccuaguaaca ccauggaggu ugcgcagugu uucgcuccac acaaccccag 300

uguagaucag gccgaugagu caccgcacuc cucacgggcg accguggcgg uggcugcgcu 360

ggcggccugc ccaugggaua acccauggga cgcuucaaua cugacauggu gcgaagaguc 420

uauugagcua auugguaguc cuccggcccc ugaaugcggc uaauccuaac ugcggagcag 480

auacccacac gccagugggc agucugucgu aaugggcaac ucugcagcgg aaccgacuac 540

uuuggguguc cguguuuccu uuuauuuuua uacuggcugc uuauggugac aauugagaga 600

uuguuaccau auagcuauug gauuggccau ccagugacaa acagagcgau uauuuauuua 660

uuuguugguu auaucccauu aagccaaaag gcccuaguua cucucaacuu uauacuauug 720

cuuaauacaa cgaaauggga gcucaggugu cuacacaaaa gacgggugca caugagacug 780

gcuugaaugc cagcggcaau ucugucaucc auuacaccaa caucaauuac uacaaggaug 840

cugcauccaa cuccgcaaau aggcaagacu ucacacaaga cccagguaag uucacugaac 900

cuguaaagga caucauggug aaaacuaugc cugcguugaa cucacccucu gcugaggaau 960

gcggcuacag ugacagagug cguuccauca cauuagguaa uuccacuauc accacccaag 1020

agugugccaa cguggucguc ggguacggag uguggccaga guaucuaaaa gacaacgagg 1080

ccacagccga agaucagccc acacaaccug auguggccac cugccgcuuc uauacuuuag 1140

aaucagugca guggaugaaa aauucggccg gguggguggug gaagcugccu gaugcacugu 1200

cacagauggg cuuguuuggu caaaacaugc aguaccacua cuugggaaga acaggguaua 1260

cuauacacgu acaaugcaau gccucaaagu uccaucaagg augccugcuu guagugugug 1320

ugccugaagc ugagaugggg ugcucuaacu ugaacaacac gcccgaauuc agugaguuau 1380

ccggaggaga cagugccaga auguucaccg auacacaggu cggggaguca aacgccaaga 1440

aaguacagac agcggugugg aacgccggaa ugggcguugg agugggguaau cucacuauuu 1500

ucccucacca auggaucaac cugaggacca acaacagugc aacgcuagug augccuuaca 1560

uaaacagcgu ccccauggau aacauguuca ggcacaauaa cuuaacacua augauuaucc 1620

cauuuguacc gcuuaauuac agcgagggau ccucaccgua cgugcccaua acagucacca 1680

ucgcuccuau gugugcagag uacaacgggc uacggcuggc cagcaaccag ggucuaccag 1740

uuaugacaac accugggagc acgcaauuuc uaacuucaga ugacuuccag ucaccgucag 1800

cgaugccaca guuugauguc accccagaga ugcaaauacc aggccgcgua aauaauuuga 1860
uggagauugc cgagguggac ucaguggugc cugugaauaa cacagaggac aacgugagua 1920
gccuuaaggc auaccagauu ccaguacagu ccaacaguga caaugguaag cagguuuuug 1980
guuuucccuu acaaccgggu gccaacaacg uucugaauag aacccucuug ggugaaauuc 2040
uaaacuauua cacccauugg aguggcagca uaaaacuuac auucauguuc ugugggucug 2100
ccauggccac uggcaaguuc cuucuggcau acucaccacc uggagcagga gucccgaaaa 2160
accgaaaaga ugcuaugcug ggcacccacg ucauauggga uguuggguua caaucaagcu 2220
gcguguugug cguaccgugg auuagccaaa cacacuacag auacgugguu gaggaugaau 2280
acacagcagc cggauauguu acaugcuggu aucaaacaaa uaucguggug ccagcugaug 2340
ugcaaagcuc augugacauc uugugcuuug uuucagccug caacgauuuc ucugugcgga 2400
ugcugaaaga uacgcccuuu auaagacagg acacauuuua ucacggccca guggaggagu 2460
cuguggaucg ugcaguggca cguguggcgg acacgauuag uucacggccc acuaacucgg 2520
agucaauucc agcgcucacc gccgccgaga cugggcauac cucccaaguu gugccuagcg 2580
acaccaugca aacaagacau gugaaaaacu accacucacg guccgaaucg uccauugaaa 2640
auuuccuaug ccggucugcc uguguauacu augccacaua caccaauaac ucaaaaaaag 2700
aggguuugc agaguggguu aucaacacua gacagguggc acagcuaaga agaaaguugg 2760
agcucuucac auaucuaagg uuugacuuag aacuaacauu uguuauaacg agcgcacaac 2820
aacccaguac cgccuccagu guagacgcuc ccgugcaaac ccaucagauu auguaugugc 2880
cuccaggugg cccuguacca acaaagguua aagauuaugc auggcaaacu uccacaaacc 2940
ccagcguuuu cuggacagaa ggaaacgccc caccaaggau guccauccca uucauuagca 3000
uugguaaugc guauaguugu uuuuaugaug gguggacgca guuuucaaga aacgggucu 3060
auggcaucaa uacccucaac aacaugggca cguuguauau gaggcauguc aaugaagcgg 3120
ggcagggucc aaucaaaagu acuguuagaa uauauuucaa acccaagcac gugaaggcgu 3180
gggugccacg uccgccaaga uugugucaau augagaagca gaaaaauguc aauuuuagcc 3240
cuauaggagu aaccacaagu agaacggaca uuauaacaac aggcacuuuu ggucagcaga 3300
ugaagcaccu gugguucuuc cugcugcugg uggccgcucc uaggugggug cugucccagg 3360
ugcagcuggu gcagagcggc guggagguga agaagcccgg cgcuuccgug aaggugaccu 3420
gcaaggccuc cggcuacacc uucaccaacu acuacaugua cuggguagg caggccccug 3480
gacagggacu ggaguggaug ggcggcauca acccuuccaa cggcggcacc aacuucaacg 3540
agaaguucaa gaaccgggug acccugacca ccgacuccuc caccaccacc gccuacaugg 3600
agcugaaguc ccugcaguuu gacgacaccg ccguguacua cugcgccagg agggacuacc 3660
gguucgacau gggcuucgac uacuggggcc agggcacaac cgugaccgug uccagcggag 3720

guggcggauc uggaggggu gguagcggug gaggcgggag ugagaucgug cugacccagu 3780
ccccugcuac acugucccug ucccccggcg agagggcuac acugagcugc agggccucca 3840
agggcguguc caccuccggc uacuccuacc ugcacuggua ccagcagaag ccuggacagg 3900
cucccaggcu gcugaucuac cuggccuccu accuggaguc cggcgugccu gcuagguuuu 3960
ccggcagcgg cagcggcacc gauuucaccc ugaccaucuc cucccuggag cccgaggacu 4020
ucgccgugua cuacugccag cacuccaggg aucugccucu gaccuucggc ggcggcacca 4080
agguggagau caagaccaca aguagaacgg acauuauaac aacaggcacu uuuggucagc 4140
aguccggagc gauauaugug ggcaauuaca ggauagucaa uaggcaccug gcaacacacu 4200
uugacuggca aaauugcaua ugggaggauu acaacaggga ccuccuagua ugcacuacaa 4260
cggcgcacgg uugugacaau auugcaagau gccagugcac agcaggugug uauuauugug 4320
cuucuaggaa caagcauuac ccggugcauu uugaaggacc aggcuuggug gagguccagg 4380
aaagugaaua uuauccaaaa agguaucaau cacacgugcu ucuugccgca ggguuuucug 4440
agccgggga uuguggugggu auucucaggu gugaacaugg uguuauaggc aucgugacca 4500
uggguggguga ggguguuguc ggcuuugccg augugcgcga ucucuuaugg cuggaagacg 4560
acgcaaugga acagggaguc agggacuacg uggaacagcu uggaaacgcc uuugguucug 4620
gguuuaccaa ccagauuugc gagcaaguca aucuguuaaa agagucacua auaggccaag 4680
acuccaucuu ggaaaaauca cucaaggcau uagugaaaau cauaucagca cugguuaucg 4740
uggugagaaa ccacgaugac cucauaacag ucacugccac cuuagcccug auuggcugua 4800
ccacuucacc guggcggugg cuaaagaaaa aagugacuca auacuauggg auucccaugg 4860
ccgagagaca gaacaauggc uggcucaaga aauucacaga aaugaccaau gcaugcaagg 4920
gcauggaaug gaucgccauc aaaauccaaa aguucauuga guggcucaaa auuaagaucu 4980
ugccagaagu aaaggaaaaa cacgaguuuc uuaccagacu uaagcaacuc ccacucuuag 5040
aaagccaaau ugccaccauc gagcagagug caccaucaca gagugaucag gaacaacugu 5100
ucuccaauau acaguacuuc gcgcacuauu gcagaaagua cgccccacug uacgcagcug 5160
aggcaaaaag aguguucucu cuagaaaaga aaaugagcaa cuacauacag uucaagucca 5220
aaugccguau ugaaccaguc uguuuauugc uccaugguag cccuggagcc ggaaaauccg 5280
uggcuaccaa uuugauugga cgcucacuug cagagaagcu caacaguuca guguauucau 5340
ugccaccaga cccagaccau uuugacggcu auaaacaaca agcugucgua aucauggaug 5400
accugugcca gaacccagau gggaaagacg uguccuuguu cugccagaug guuucaagug 5460
uagacuuugu uccaccaaug gcagcacuug aggagaaagg cauacucuuc acaucgccuu 5520
ucgugcucgc cucuaccaac gcagguucca ucaacgcucc cacuguguca gacagcagag 5580

cccuugcuag aagguuccac uucgacuuga acauugaggu caucucaaug uacagccaga 5640
auggcaaaau caacaugcca augucaguga aggcuuguga ugaugagugu uguccaguua 5700
auuucaagag guguugucca uuagugugcg guaaggccau ucaguucauu gacagaagaa 5760
cacaaaugag guauucacua gacaugcuug uaacugaaau guucagggag uacaaccaca 5820
gacauagugu gggggcaaca cucgaggcac ucuuccaagg cccacccgug uacaaagaga 5880
ucaagaucag uguugcgcca gaaaccccuc ccccaccggc aauagcagau cugcugaaau 5940
cuguggacag ugaagcaguu agagaguacu gcaaggaaaa gggaugguug gugccugaga 6000
ucaauuccac ucuacaaauu gaaaagcacg ugagcagggc cuucauuugc cugcaagcgc 6060
ucaccacguu uguuuccgug gcggggauaa uuuacaucau auacaaauug uuugcuggcu 6120
uccagggggc cuacaccggu augccaaacc aaaaaccuaa aguaccaacu uugaggcagg 6180
cuaaggugca gggaccggca uucgaauucg cuguggcaau gaugaaaaga aaugcuagca 6240
cagugaagac cgaguacggu gaguucacaa ugcuuggaau cuaugacagg ugggcagugc 6300
uaccucguca cgcuagacca gggccuacca uccuaaugaa ugaccaggaa gugggugugg 6360
uggaugccaa ggaguuagug gacaaggaug gcaccaauuu agaauuaaca cucuugaagc 6420
ucaauagaaa ugagaaauuc agagacaucc gaggcuucuu aacacgugag gaggccgaag 6480
ucaacgaggc ugugcuugcu auuaacacca gcaaguuccc aaauauguac auuccaguug 6540
ggcaaguaac agauuauggc uuccucaacu ugggugcac accaacuaag cgcaugcuga 6600
uguacaacuu cccaacacgu gcaggccaau gugggggagu ucuaaugucu acagguaaag 6660
ugcucggaau acacgucggu gguaauggac accaaggguu uucggcagcc cuguuacgcc 6720
acuauuucaa ugacgagcag ggagagauug aguucaucga gagcucuaag gaugcaggcu 6780
uuccagucau uaacacaccu agcaaaacaa agcucgagcc caguguuuuc caucacgugu 6840
uugagggaaa caaagaacca gcaguguuga ggaauggaga cccaagacug aaagccaauu 6900
uugaggaagc cauuuucucc aaguacaucg ggaaugugaa cacacaugua gacgaguaca 6960
ugauggaagc cgucgaccau uaugcaggac aguuagcuac uuuagauauc aguacagaac 7020
cuaugaagcu ugaggaugcu gucuacggua cugagggcuu ggaagcucug gacuugacca 7080
cuagugcugg guaucccuau gucgcccuug guauaaaaaa gagagacauc uuauccaaaa 7140
agucuaagga ccugucuaaa cugagagagu guauggacaa guaugguua aaccucccua 7200
uggucaccua ugugaaggau gaauugaggu cagcagagaa aguggcuaag ggcaaaucca 7260
gacuaaucga ggcguccagu uuaaaugacu caguggcaau gagacagacu uucgguaacc 7320
uguauaagac auuccaucug aacccgggca uagugacugg cagugcaguu ggaugcgauc 7380
ccgacuuguu cuggagcaag auuccaguca ugcucgaugg ucaccucauc gccuuugacu 7440
acaguggaua ugaugccagc cugaguccgg ugugguuugc uugcuugaaa cuguuguugg 7500

aaaagcuggg guacucacac aaggaaacua auuacauaga uuacuugugc aacucccacc 7560

auuuguacag agacaagcac uacuuuguga ggggaggaau gccauccggg uguucuggaa 7620

ccagcauuuu caauucaaug auuaauaaca uaaucaucag aacacugaug uugaaggugu 7680

acaaagggau agaucuagac caauuuagga ugauugcaua uggugacgac gucauugcuu 7740

cguacccgua uccaauugau gcgucauugc uggcugaggc uggcaagggg uauggucuga 7800

uuaugacacc agcagacaaa ggagaguguu ucaaugaagu aacguggacc aacgucacuu 7860

uccugaaaag auacuucaga gcggaugagc aauaucccuu ccucguucac ccagugaugc 7920

caaugaagga cauucaugag ucaauuaggu ggacuaaaga ccccaaaaac acccaagauc 7980

acgugcgauc cuugugcuua uuggccuggc acaauggaga gcaagaauau gagaauuuug 8040

uauccaaaau cagaagcguc ccaguugggc gcugcuugac auugccugcg uucucgacuc 8100

ugcgcaggaa gugguuggau uccuucuaaa auugaaguac aaugugauuu aacucaauug 8160

gcuuaacccu accacacuua ccgaacuaga uaacggugug guaggguaa auuc 8214

<210> 17
<211> 22
<212> DNA
<213> Artificial Sequence

<220>
<223> DNA sequence of miR-133 target sequence

<400> 17
acagctggtt gaaggggacc aa 22

<210> 18
<211> 22
<212> DNA
<213> Artificial Sequence

<220>
<223> DNA sequence of miR-206 target sequence

<400> 18
ccacacactt ccttacattc ca 22

<210> 19
<211> 102
<212> DNA
<213> Artificial Sequence

<220>
<223> DNA sequence of tandem sequence of miR-133 target sequence and miR-206 target sequence

<400> 19
acagctggtt gaaggggacc aacgatacag ctggttgaag ggaccaaac cggtccacac 60

acttccttac attccatcac ccacacactt ccttacattc ca 102

<210> 20
<211> 507
<212> DNA
<213> Artificial Sequence

<220>
<223> DNA sequence of internal ribosome entry site sequence of HRV2

<400> 20
```
aacttagaag tttttcacaa agaccaatag ccggtaatca gccagattac tgaaggtcaa      60

gcacttctgt ttccccggtc aatgttgata tgctccaaca gggcaaaaac aactgcgatc     120

gttaaccgca aagcgcctac gcaaagctta gtagcatctt tgaaatcgtt tggctggtcg     180

atccgccatt tcccctggta gacctggcag atgaggctag aaatacccca ctggcgacag     240

tgttctagcc tgcgtggctg cctgcacacc ctatgggtgt gaagccaaac aatggacaag     300

gtgtgaagag ccccgtgtgc tcgctttgag tcctccggcc cctgaatgtg gctaacctta     360

accctgcagc tagagcacgt aacccaatgt gtatctagtc gtaatgagca attgcgggat     420

gggaccaact actttgggtg tccgtgtttc actttttcct ttatatttgc ttatggtgac     480

aatatataca atatatatat tggcacc                                         507
```

<210> 21
<211> 20
<212> PRT
<213> Artificial Sequence

<220>
<223> VEGF-derived polypeptide

<400> 21
```
Lys Ser Val Arg Gly Lys Gly Lys Gly Gln Lys Arg Lys Arg Lys Lys
1               5                   10                  15

Ser Arg Tyr Lys
            20
```

## Description

### Technical Field

The invention relates to the fields of virus and tumor therapy. In particular, the present invention relates to use of a CBV1 or modified form thereof, or of a nucleic acid molecule comprising a genomic nucleotide sequence of the CBV1 or modified form thereof or a complementary sequence thereof, for treating a tumor in a subject (e.g., a human), and for manufacture of a medicament for treating a tumor in a subject (e.g., a human). The present invention also relates to a method for treating a tumor, which comprises a step of administering to a subject in need thereof a CBV1 or modified form thereof, or a nucleic acid molecule comprising a genomic nucleotide sequence of the CBV1 or modified form thereof or a complementary sequence thereof.

### Background Art

The current means for treatment of malignant tumors mainly include surgical treatment, chemotherapy and radiotherapy. These traditional therapies are not satisfactory in the treatment of metastasized tumors, and may further cause great harm to the health of patients. In contrast, as a new type of treatment, the method for treating tumors by using oncolytic viruses has the characteristics of high specificity, good effect, and low side effects, and thus is currently considered as a promising method for treating tumors.

An oncolytic virus is a virus that can self-replicate in tumor cells, thereby killing or lysing tumor cells, or arresting the growth of tumor cells. When used in in vivo treatment, oncolytic viruses exhibit specific selectivity for tumor cells and can directly induce the death of tumor cells, but have little or no effect on normal cells; meanwhile, oncolytic viruses can also stimulate the response of B lymphocytes and T lymphocytes in immune system, thereby indirectly killing tumor cells.

Enteroviruses with oncolytic activity that have been reported so far include the chimeric poliovirus for treatment of human solid tumors such as malignant gliomas (Dobrikova et al., Mol Ther 2008, 16(11): 1865-1872 ); echovirus ECHO1 that kills human gastric cancer cells and ovarian cancer cells (Shafren et al., Int J Cancer 2005, 115(2): 320-328; Haley et al., J Mol Med (Berl) 2009, 87(4): 385-399); and so on. However, it is still necessary to obtain a virus with both tumor specificity and tumor killing activity.

CVB1 belongs to the Enterovirus genus in the Picomaviridae family. Studies have found that CVB1 generally only causes mild symptoms such as fever, sneezing, and coughing in infected people after infection, but it may also cause severe chronic autoimmune diseases such as viral myocarditis, pancreatitis, hepatitis, aseptic meningitis, and insulin-dependent diabetes in neonates, infants and immunodeficiency adults (Rinehart et al., J Virol 1997, 71(5): 3986-3991). At present, there are no reports on oncolytic activity of CVB1 in the art.

### Contents of the Invention

In the present invention, unless otherwise stated, the scientific and technical terms used herein have the meaning commonly understood by those skilled in the art. In addition, the operating steps for cell culture, biochemistry, cell biology, nucleic acid chemistry and so on used herein are conventional steps widely used in the corresponding fields. Meanwhile, in order to better understand the present invention, definitions and explanations of related terms are provided below.

As used herein, the term "CVB1 (Coxsackivirus B1)" refers to one species of Coxsackivirus B of Enterovirus genus of Picomaviridae family, the genome of which is a single-stranded positive-sense RNA consisting of 5' non-coding region (5' UTR), one open reading frame (ORF), 3' non-coding region (3' UTR), and poly(A) tail. The ORF encoding a precursor polyprotein, which can be hydrolyzed and cleaved by its own protease to produce structural proteins VP1 to VP4 and non-structural proteins 2A, 2B, 2C, 3A, 3B, 3C, and 3D. In order to more clearly describe the present invention, the nucleic acid sequences corresponding to the above-described proteins in the CVB 1 genome are called VP1 gene, VP2 gene, VP3 gene, VP4 gene, 2A gene, 2B gene, 2C gene, 3A gene, 3B gene, 3C gene and 3D gene. In the present invention, the expression "Coxsackivirus B1 (CVB1)" means wild-type CVB1, which can be isolated from sources in nature and has not been intentionally and artificially modified, examples of which include but are not limited to prototype strain Conn-5, and various clinical isolates (for example, the clinical isolates described in Example 1 of the present invention). The genomic sequence or cDNA sequence of wild-type CVB1 are well known in the art, and can be found in various public databases (for example, GenBank database accession number: MG780414).

As used herein, the term "modified form" of a virus refers to a modified virus obtained by modifying a wild-type virus, which retains the desired activities (e.g., oncolytic activity) of the wild-type virus. In the present invention, "modified form" of CVB1 includes, but is not limited to, a modified CVB1 virus, the genomic sequence of which has a substitution, insertion or deletion of one or more nucleotides compared to that of a wild-type CVB1, and at least retains the oncolytic activity of CVB1.

As used herein, the term "oncolytic virus" refers to a virus that can infect a tumor cell, replicate in the tumor cell, cause the death and lysis of the tumor cell, or prevent the growth of the tumor cell. Preferably, the virus has minimal toxic effects on a non-tumor cell.

As used herein, the term "tumor-specificity" refers to selectively exhibiting a biological function or activity in a tumor cell. For example, in the present invention, when the term "tumor specificity" is used to describe the killing selectivity of a virus, it means that the virus can selectively kill a tumor cell without killing or substantially not killing a non-tumor cell, or, the virus is more effective in killing a tumor cell than killing a non-tumor cell.

As used herein, the term "oncolytic activity" mainly comprises tumor-killing activity. When describing the oncolytic activity of a virus, the oncolytic activity of the virus can typically be measured by its ability to infect a tumor cell, its ability to replicate in the tumor cell, and/or its ability to kill the tumor cell. The oncolytic activity of a virus can be measured using any method known in the art. For example, the ability of a virus to infect a tumor cell can be evaluated by measuring the viral dose required to infect a given percentage of tumor cells (e.g., 50% of cells); the ability to replicate in a tumor cell can be evaluated by measuring the growth of the virus in the tumor cell; the ability to kill a tumor cell can be evaluated by monitoring cytopathic effect (CPE) or measuring tumor cell activity.

As used herein, the expression "cDNA sequence of CVB1" means that the DNA form of the viral genomic RNA sequence which differs from the RNA sequence only in that the ribonucleotides in the RNA sequence are replaced by corresponding deoxyribonucleotides, for example, uracil ribonucleotide (UMP) is replaced by thymine deoxyribonucleotide (dTMP).

As used herein, the term "exogenous nucleic acid" refers to an artificially introduced nucleotide sequence that is foreign to the original sequence. Exogenous nucleic acid includes, but is not limited to, any genes or nucleotide sequences not found in the viral genome. However, in the present invention, it is particularly preferred that the exogenous nucleic acid consists of at most 1500, for example at most 1200, at most 1000 nucleotides. In some cases, preferably, the exogenous nucleic acid encodes a protein or polypeptide having anti-tumor killing activity, such as a cytokine, or an anti-tumor protein or polypeptide; or, the exogenous nucleic acid includes a target sequence of microRNA (miRNA). In the present invention, the microRNA is preferably a microRNA having an expression level in tumor cells significantly lower than that in normal cells and/or having obvious tissue specificity, examples of which include, but are not limited to, miR-122, miR-192, miR-483, etc., which are specifically expressed in liver tissue; miR216a/b, miR217 and miR-375, which are specifically expressed in pancreatic tissue; miR-1, miR-133a/b, miR-208, etc., which are specifically expressed in heart; miR-192, miR-196a/b, miR-204, miR-215, etc., which are specifically expressed in kidney tissue; miR-133a/b, miR-206, etc., which are specifically expressed in muscle tissue; miR-124a , MiR-125a/b, miR-128a/b, miR-138, etc., which are specifically expressed in brain tissue; and miR-34, miR-122a, miR-26a, which are under-expressed in liver tumor tissue; miR-107, miR-96 and miR-196, which are under-expressed in pancreatic tumor tissue; miR-34, which is under-expressed in kidney tumor tissue; miR-143, miR-133a/b, which are under-expressed in bladder tumor tissue; miR-Let-7, miR-29, which are under-expressed in lung tumor tissue; and the like (see, for example, Ruiz AJ and Russell S J. MicroRNAs and oncolytic viruses. [J]. Curr Opin Virol, 2015, 13: 40-48; all of which are incorporated herein by reference in their entireties).

In the present invention, when the modified CVB1 contains the target sequence of the above microRNA, it is regulated by the microRNA in the cells/tissues where the microRNA is highly expressed or specifically expressed, so that the replication of the oncolytic virus is weakened and even the killing activity is lost, while it can normally replicate in the tumor cells/tissues with low or no expression of the microRNA, and thus kill the tumor cells.

As used herein, the term "cytokine" has a meaning well known to those skilled in the art. However, in the present invention, when the oncolytic virus of the present invention is used to treat a tumor, it is particularly preferred that the cytokine is a cytokine that can be used for tumor treatment. Examples of "cytokine" include, but are not limited to, interleukins (e.g, IL-2, IL-12 and IL-15), interferons (e.g., IFN$\alpha$, IFN$\beta$, IFN$\gamma$), tumor necrosis factors (e.g., TNF$\alpha$), colony stimulating factors (e.g., GM-CSF), and any combination thereof (see, for example, Ardolino M, Hsu J, Raulet D H. Cytokine treatment in cancer immunotherapy [J]. Oncotarget, 2015, 6 (23): 19346-19347).

As used herein, the term "anti-tumor protein or polypeptide" refers to a protein or polypeptide that has therapeutic activity against tumor, including but not limited to: (1) a protein or polypeptide that is toxic to a cell, or capable of inhibiting cell proliferation or inducing apoptosis, its examples include but are not limited to, thymidine kinase TK (TK/GCV), TRAIL, and FasL (see, for example, Candolfi M, King GD, Muhammad AG, et al. Evaluation of proapototic transgenes to use in combination with Flt3L in an immune-stimulatory gene therapy approach for Glioblastoma multiforme (GBM) [J]. FASEB J, 2008, 22: 1077.13); (2) a protein or polypeptide with immunotherapeutic effect, its examples include but are not limited to, single chain antibodies (scFv) against cytotoxic T lymphocyte-associated antigen 4 (anti-CTLA-4), programmed death receptor 1 (anti-PD-1) and programmed death ligand 1 (anti-PDL-1) (see, for example, Nolan E, Savas P, Policheni AN, et al. Combined immune checkpoint blockade as a therapeutic strategy for BRCA1-mutated breast cancer [J]. Science Trans Med, 2017, 9: eaal4922; all of which are incorporated herein by reference); (3) a protein or polypeptide that inhibits tumor angiogenesis, its examples include but are not limited to, single chain antibody (scFv) against vascular endothelial growth factor (anti-VEGF), VEGF-derived polypeptide (e.g., $_D$(LPR), KSVRGKGKGQKRKRKKSRYK, etc.), and ATN-161 (see, for example, Rosca EV, Koskimaki JE, Rivera CG, et al. Anti-angiogenic peptides for cancer therapeutics [J]. Curr Pharm Biotechnol, 2011, 12 (8): 1101-1116; all of which are incorporated herein by reference).

As used herein, the term "scFv" refers to a single polypeptide chain comprising a heavy chain variable region (VH) and a light chain variable region (VL), where the VL and VH are ligated by a linker ( See, for example, Bird et al., Science 242:423-426 (1988); Huston et al., Proc. Natl. Acad. Sci. USA 85:5879-5883 (1988); and Pluckthun, The Pharmacology of Monoclonal Antibodies, Volume 113, edited by Roseburg and Moore, Springer-Verlag, New York, pages 269-315 (1994)). Such scFv molecule can have a general structure: NH2-VL-linker-VH-COOH or NH2-VH-linker-VL-COOH.

As used herein, the term "identity" refers to the match degree between two proteins/polypeptides or between two nucleic acids. When two sequences for comparison have the same monomer sub-unit of base or amino acid at a certain site (e.g., each of two DNA molecules has an adenine at a certain site, or each of two proteins/polypeptides has a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by for example using a computer program such as Align program (DNAstar, Inc.) which is based on the method of Needleman, et al. (J. Mol. Biol. 48:443-453, 1970). The percentage of identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, and with a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and with a gap weight of 16, 14, 12, 10, 8, 6, or 4 and a length weight of 1, 2, 3, 4, 5, or 6.

As used herein, the term "vector" refers to a nucleic acid vehicle into which a polynucleotide can be inserted. When a vector enables expression of a protein encoded by an inserted polynucleotide, the vector is referred to as an expression vector. A vector can be introduced into a host cell by transformation, transduction, or transfection, so that the genetic material elements carried by the vector can be expressed in the host cell. The vector is well known to those skilled in the art and includes, but is not limited to: plasmids; phagemids; cosmids; artificial chromosomes, such as yeast artificial chromosomes (YAC), bacterial artificial chromosomes (BAC) or P1-derived artificial chromosomes (PAC); bacteriophages such as λ-phage or M13 phage and animal viruses. Animal viruses that can be used as vectors include, but are not limited to, retroviruses (including lentiviruses), adenoviruses, adeno-associated viruses, herpesviruses (such as herpes simplex virus), poxviruses, baculoviruses, papillomaviruses, and papovaviruses (such as SV40). A vector may contain a variety of elements that control expression, including, but not limited to, promoter sequences, transcription initiation sequences, enhancer sequences, elements for selection, and reporter genes. In addition, the vector may contain a replication initiation site.

As used herein, the term "internal ribosome entry site (IRES)" refers to a nucleotide sequence located in a messenger RNA (mRNA) sequence, which can initiate translation without relying on the 5' cap structure. IRES is usually located in the 5' untranslated region (5'UTR), but may also be located in other positions of the mRNA.

As used herein, the term "human rhinovirus 2 (HRV2)" refers to a virus in the family of picornaviridae whose genomic sequence or cDNA sequence is well known in the art, and can be found in various public databases (e.g., GenBank database accession number X02316.1).

As used herein, the expression "a nucleic acid molecule comprising a genomic sequence of CVB1 or a modified form thereof" or "a nucleic acid molecule comprises a genomic sequence of CVB1 or a modified form thereof" has the meaning commonly understood by those skilled in the art, that is, when the nucleic acid molecule is DNA, the nucleic acid molecule comprises a genomic sequence of CVB1 or a modified form thereof in form of DNA; when the nucleic acid molecule is RNA, the nucleic acid molecule comprises a genomic sequence of CVB1 or a modified form thereof.

As used herein, the term "pharmaceutically acceptable carrier and/or excipient" refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995), and includes, but is not limited to: pH adjusting agents, surfactants, ionic strength enhancers, agents to maintain osmotic pressure, agents to delay absorption, diluents, adjuvants, preservatives, stabilizers, etc. For example, pH adjusting agents include, but are not limited to, phosphate buffered saline. Surfactants include, but are not limited to, cationic, anionic or non-ionic surfactants, such as Tween-80. Ionic strength enhancers include, but are not limited to, sodium chloride. Agents that maintain osmotic pressure include, but are not limited to, sugar, NaCl, and the like. Agents that delay absorption include, but are not limited to, monostearate and gelatin. Diluents include, but are not limited to, water, aqueous buffers (such as buffered saline), alcohols and polyols (such as glycerol), and the like. Adjuvants include, but are not limited to, aluminum adjuvants (such as aluminum hydroxide), Freund's adjuvants (such as complete Freund's adjuvant), and the like. Preservatives include, but are not limited to, various antibacterial and antifungal agents, such as thimerosal, 2-phenoxyethanol, parabens, trichloro-t-butanol, phenol, sorbic acid, and the like. Stabilizers have the meaning commonly understood by those skilled in the art, which can stabilize the desired activity (such as oncolytic activity) of the active ingredients in the drug, including but not limited to sodium glutamate, gelatin, SPGA, sugars (e.g., sorbitol, mannitol, starch, sucrose, lactose, dextran, or glucose), amino acids (e.g., glutamic acid, glycine), proteins (e.g., dried whey, albumin, or casein) or their degradation products (e.g., lactalbumin hydrolysates).

As used herein, the term "treating" refers to treating or curing a disease (e.g., a tumor), delaying the onset of symptoms of a disease (e.g., a tumor), and/or delaying the development of a disease (e.g., a tumor).

As used herein, the term "effective amount" refers to an amount that can effectively achieve the intended purpose. For example, a therapeutically effective amount can be an amount effective or sufficient to treat or cure a disease (e.g., a tumor), delay the onset of symptoms of a disease (e.g., a tumor), and/or delay the development of a disease (e.g., a tumor). Such an effective amount can be easily determined by a person skilled in the art or a doctor, and can be related to the intended purpose (such as treatment), the general health condition, age, gender, weight of the subject, severity of the disease to be treated, complications, administration routes, etc. The determination of such an effective amount is well within the capabilities of those skilled in the art.

As used herein, the term "subject" refers to a mammal, such as a primate mammal, such as a human. In certain embodiments, the subject (e.g., a human) has a tumor, or is at risk for having a tumor.

After a lot of experiments and repeated explorations, the inventors of the present application have unexpectedly discovered that CVB1 has a broad-spectrum and significant tumor cell killing ability. Based on this discovery, the inventors have developed a new oncolytic virus for treating a tumor and a method for tumor treatment based on the virus.

Therefore, in a first aspect, the present invention provides use of a Coxsackievirus B1 (CVB1) or a modified form thereof or a nucleic acid molecule for treating a tumor in a subject, or for manufacture of a medicine for treating a tumor in a subject; wherein the nucleic acid molecule comprises a sequence selected from the following:

(1) a genomic sequence or cDNA sequence of CVB1 or modified form thereof; and

(2) a complementary sequence of the genomic sequence or cDNA sequence.

In certain preferred embodiments, the CVB1 is a wild-type CVB1.In certain preferred embodiments, the CVB1 may be a clinical isolate isolated from an individual infected with Coxsackievirus B1.

In certain preferred embodiments, the genomic sequence of CVB 1 or modified form thereof has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO:12. In certain preferred embodiments, the genomic sequence of CVB1 or modified form thereof is the nucleotide sequence as shown in SEQ ID NO: 12.

In certain preferred embodiments, the cDNA sequence of CVB1 or modified form thereof has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO:1. In certain preferred embodiments, the cDNA sequence of CVB1 or modified form thereof is the nucleotide sequence as shown in SEQ ID NO: 1.

In certain preferred embodiments, the modified form is a modified CVB1 that has a substitution, insertion or deletion of one or more nucleotides in the genome compared to a wild-type CVB1.

In certain preferred embodiments, as compared to the wild-type CVB1, the modified CVB 1 has one or more modifications selected from the following:

(1) one or more mutations in an untranslated region (e.g. 5'UTR or 3'UTR);

(2) an insertion of one or more exogenous nucleic acids;

(3) a deletion or mutation of one or more endogenous genes; and

(4) any combination of the above three items.

In certain preferred embodiments, the modified CVB1 comprises one or more mutations in a 5' untranslated region (5'UTR).

In certain preferred embodiments, the modified CVB1 has a substitution of all or part of the 5'UTR sequence.

In certain preferred embodiments, the internal ribosome entry site (IRES) sequence in the 5'UTR of the modified CVB1 is replaced with an exogenous IRES sequence, such as an internal ribosome entry site sequence of human rhinovirus 2 (HRV2). In certain preferred embodiments, the internal ribosome entry site sequence of human rhinovirus 2 (HRV2) is shown in SEQ ID NO:2.

The use of the internal ribosome entry site sequence of human rhinovirus 2 (HRV2) is advantageous in some cases, for example, to improve the tumor specificity of oncolytic viruses. It has been previously reported that in normal human nerve cells, the internal ribosome entry site sequence of human rhinovirus 2 is specifically bound by host RNA-binding proteins (DRBP76 and NF45), thereby preventing the recruitment of factors such as eIF4G (Merrill et al. J Virol 2006,80 (7): 3147-3156; Merrill and Gromeier, J Virol 2006,80 (14): 6936-6942; Neplioueva et al. PLoS One 2010,5(7): e11710); in the meantime, without the support of Raf/Erk1/2/MAPK and other signaling pathways, ribosomes can hardly be bound to the internal ribosome entry site sequence of human rhinovirus 2 and therefore translation of viral protein cannot be initiated (Dobrikov et al., Mol Cell Biol 2011, 31 (14): 2947-2959; Dobrikov et al., Mol Cell Biol 2013, 33 (5): 937-946). In human glioma tumor cells, the internal ribosome entry site of human rhinovirus 2 is not affected by the above two factors, and thus can normally initiate transcription and translation of viral protein. Therefore, in some cases, replacing the internal ribosome entry site sequence of CVB1 with the internal ribosome entry site sequence of human rhinovirus 2 is beneficial to avoid or reduce the toxic and side effects of the virus of the present invention on normal human nerve cells without affecting the use of the virus in the treatment of human gliomas.

In certain preferred embodiments, the modified CVB1 comprises an exogenous nucleic acid.

In certain preferred embodiments, the exogenous nucleic acid encodes a cytokine (e.g., GM-CSF, preferably human GM-CSF), or an anti-tumor protein or polypeptide (e.g., scFv against PD-1 or PD-L1, preferably, scFv against human PD-1 or PD-L1). In certain preferred embodiments, the exogenous nucleic acid is inserted between 5'UTR and VP4 gene, or between VP1 gene and 2A gene of a genome of the modified CVB1.

In certain preferred embodiments, the exogenous nucleic acid comprises a target sequence of microRNA (miRNA) (e.g., miR-133 or miR-206). In certain preferred embodiments, the target sequence of microRNA is inserted in a 3' untranslated region (3'UTR) of a genome of the modified CVB1.

It has been previously reported that the expression level of certain microRNAs in tumor cells is significantly lower than that in normal cells and/or has obvious tissue specificity. Thus, in some cases, it is advantageous that the modified CVB1 of the present invention comprises a target sequence of such microRNAs, because such microRNAs that are highly expressed in normal cells or tissues can reduce or even block the replication of the modified CVB1 in the normal cells or tissues via the corresponding target sequence, thereby reducing or even avoiding the toxic and side effects of the modified CVB1 on non-tumor cells. Such microRNAs include, but are not limited to, miR-133, miR-206, miR-1, miR-143, miR-145, miR-217, let-7, miR-15, miR-16, etc. (see, for example, PCT International Application WO2008103755A1, US patent application US20160143969A1, or Baohong Zhang et al., Developmental Biology, Volume 302, Issue 1, 1 February 2007, Pages 1-12; all of which are incorporated herein in their entirety by reference).

In certain preferred embodiments, the exogenous nucleic acid includes a target sequence of one or more (e.g., 2, 3 or 4) microRNAs as described above. In certain preferred embodiments, the exogenous nucleic acid comprises a target sequence of miR-133 and/or miR-206. In certain preferred embodiments, the target sequence of miR-133 is shown in SEQ ID NO:3. In certain preferred embodiments, the target sequence of miR-206 is shown in SEQ ID NO:4. In some cases, the insertion of the target sequence of miR-133 and/or miR-206 is advantageous. This is because miR-133 and miR-206 are specifically expressed in muscle tissue, so that the insertion of the target sequence of miR-133 and/or miR-206 into the modified CVB1 may change the tissue tropism of the oncolytic virus, thereby reducing or avoiding damage to normal muscle tissue.

In certain preferred embodiments, the modified CVB1 comprises at least one insertion of the exogenous nucleic acid as described above and/or at least one mutation in the untranslated region as described above.

In certain preferred embodiments, the genomic sequence of the modified CVB1 has a sequence identity of at least 70%, at least 80%, at least 85%, at least 90%, at least 91%, at least 92 %, at least 93%, at least 94%, at least 95%, at least 96%, at least 97%, at least 98%, at least 99%, or 100% to a nucleotide sequence selected from the following: nucleotide sequences as shown in SEQ ID NOs: 13-16. In certain preferred embodiments, the genomic sequence of the modified CVB1 is any one selected from the nucleotide sequences as shown in SEQ ID NOs: 13-16.

In certain preferred embodiments, the cDNA sequence of the modified CVB1 has a sequence identity of at least 70%, at least 80%, at least 85%, at least 90%, at least 91%, at least 92 %, at least 93%, at least 94%, at least 95%, at least 96%, at least 97%, at least 98%, at least 99%, or 100% to a nucleotide sequence selected from the following: nucleotide sequences as shown in SEQ ID NOs: 8-11. In certain preferred embodiments, the cDNA sequence of the modified CVB 1 is any one selected from the nucleotide sequences as shown in SEQ ID NOs: 8-11.

The modified CVB1 of the present invention can be obtained by reverse genetics technology, which is known in the art, for example, see Yang LS, Li SX, Liu YJ, et al. Virus Res, 2015, 210: 165-168; Hou WH, Yang LS, Li SX, et al. Virus Res, 2015, 205: 41-44; all of which are incorporated herein by reference. In such embodiments, the cDNA of wild-type CVB1 is typically subjected to modification (e.g., insertion of an exogenous nucleic acid, deletion or mutation of an endogenous gene, or mutation in an untranslated region) to obtain the modified CVB1.

The CVB1 or modified form thereof according to the present invention may be subjected to a pretreatment to reduce or eliminate an immune response against the virus in a subject, wherein the pretreatment may comprise: packaging the CVB1 in a liposome or micelle, and/or using a protease (e.g., chymotrypsin or trypsin) to remove a capsid protein of the virus to reduce a humoral and/or cellular immunity against the virus in the host.

In the present invention, the CVB1 or modified form thereof as described herein can be serially passaged for adaptation in tumor cells. In certain preferred embodiments, the tumor cells may be tumor cell lines or tumor cell strains known in the art, or tumor cells obtained by in vivo surgical resection or clinical isolation from an individual (e.g., a subject) having a tumor. In certain preferred embodiments, the CVB1 or modified form thereof is serially passaged for adaptation in tumor cells obtained from an individual (e.g., a subject) having a tumor. In certain preferred embodiments, the tumor cells are obtained by surgical resection or clinical isolation from an individual (e.g., a subject) having a tumor. In certain preferred embodiments, the method of serial passaging for adaptation comprises a plurality of (e.g., at least 5, at least 10, at least 15, at least 20) cycles that consists of the following processes: 1) infecting a target tumor cell with the virus; 2) harvesting the virus in the supernatant; and 3) reinfecting a fresh target tumor cell with the obtained virus.

In certain preferred embodiments, the CVB1 and modified form thereof as described above may be used in combination. Therefore, the medicament may comprise one or several of the CVB 1 and modified forms thereof.

In certain preferred embodiments, the nucleic acid molecule consists of a genomic sequence or cDNA sequence of the CVB1 or modified form thereof as described herein, or a complementary sequence of the genomic sequence or cDNA sequence. In certain preferred embodiments, the nucleic acid molecule has a genomic sequence of the CVB1 or modified form thereof as described herein. In certain preferred embodiments, the nucleic acid molecule is RNA. In certain preferred embodiments, the nucleic acid molecule has a nucleotide sequence as shown in any one of SEQ ID NOs: 12-16.

In certain preferred embodiments, the nucleic acid molecule is a vector (e.g., cloning vector or expression vector) comprising a genomic sequence or cDNA sequence of the CVB1 or modified form thereof as described herein, or a complementary sequence of the genomic sequence or cDNA sequence. In certain preferred embodiments, the nucleic acid molecule is a vector (e.g., cloning vector or expression vector) comprising a cDNA sequence of the CVB1 or modified form thereof as described herein, or a complementary sequence of the cDNA sequence.

In certain preferred embodiments, the nucleic acid molecule comprises a complementary sequence of the genomic sequence of the CVB1 or modified form thereof. In certain preferred embodiments, the complementary sequence is complementary to a nucleotide sequence selected from the following:

(1) a nucleotide sequence as shown in SEQ ID NO: 12;

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO:12;

(3) a nucleotide sequence as shown in any one of SEQ ID NOs: 13-16; and

(4) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in any one of SEQ ID NOs: 13-16.

In certain preferred embodiments, the nucleic acid molecule comprises a complementary sequence of the cDNA sequence of the CVB1 or modified form thereof. In certain preferred embodiments, the complementary sequence is complementary to a nucleotide sequence selected from the following:

(1) a nucleotide sequence as shown in SEQ ID NO: 1;

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO:1;

(3) a nucleotide sequence as shown in any one of SEQ ID NOs: 8-11; and

(4) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in any one of SEQ ID NOs: 8-11.

The nucleic acid molecule of the present invention can be delivered by any means known in the art, for example, a naked nucleic acid molecule (e.g., a naked RNA) can be directly injected, or a non-viral delivery system can be used. The non-viral delivery system can be obtained from a variety of materials well known in the art, including, but not limited to, the materials described in detail in "Yin H, et al. Nat Rev Genet. 2014 Aug; 15(8): 541-55." and "Riley MK, Vermerris W. Nanomaterials (Basel). 2017 Apr 28; 7(5). Pii: E94.", which are incorporated herein by reference in their entirety, such as liposomes, inorganic nanoparticles (such as gold nanoparticles), polymers (such as PEG).

In certain preferred embodiments, the medicament comprises a therapeutically effective amount of the CVB1 and/or modified form thereof, or a therapeutically effective amount of the nucleic acid molecule as described herein. In certain preferred embodiments, the medicament may be in any form known in the medical arts. For example, the medicament may be in the form of a tablet, a pill, a suspension, an emulsion, a solution, a gel, a capsule, a powder, a granule, an elixir, a lozenge, a suppository, or an injection (including injection liquid, lyophilized powder) and so on. In some embodiments, the medicament is an injection liquid or a lyophilized powder.

In certain preferred embodiments, the medicament further comprises a pharmaceutically acceptable carrier or excipient. In certain preferred embodiments, the medicament comprises a stabilizer.

In certain preferred embodiments, the medicament optionally further comprises an additional pharmaceutically active agent. In certain preferred embodiments, the CVB1 or modified form thereof according to the present invention, or the nucleic acid molecule of the present invention, is used in combination with an additional pharmaceutically active agent. In a preferred embodiment, the additional pharmaceutically active agent is a drug with anti-tumor activity, such as an additional oncolytic virus, chemotherapeutic agent or immunotherapeutic agent.

In the present invention, the additional oncolytic virus includes but is not limited to herpes virus, adenovirus, parvovirus, reovirus, Newcastle disease virus, vesicular stomatitis virus, measles virus, or any combination thereof. The chemotherapeutic agent includes but is not limited to 5-fluorouracil, mitomycin, methotrexate, hydroxyurea, cyclophosphamide, dacarbazine, mitoxantrone, anthracyclines (e.g., epirubicin or doxorubicin), etoposide, platinum compounds (e.g., carboplatin or cisplatin), taxanes (e.g., paclitaxel or docetaxel), or any combination thereof. The immunotherapeutic agent includes but is not limited to immune checkpoint inhibitor (e.g., PD-L1/PD-1 inhibitor or CTLA-4 inhibitor), tumor-specific targeting antibody (e.g., rituximab or herceptin), or any combination thereof.

In certain preferred embodiments, the medicament comprises a unit dose of the CVB1 and/or modified form thereof, for example, comprises at least $1 \times 10^2$ pfu, at least $1 \times 10^3$ pfu, at least $1 \times 10^4$ pfu, $1 \times 10^5$ pfu, $1 \times 10^6$ pfu, at least $1 \times 10^7$ pfu, at least $1 \times 10^8$ pfu, at least $1 \times 10^9$ pfu, at least $1 \times 10^{10}$ pfu, at least $1 \times 10^{11}$ pfu, at least $1 \times 10^{12}$ pfu, at least $1 \times 10^{13}$ pfu, at least $1 \times 10^{14}$ pfu or at least $1 \times 10^{16}$ pfu of the CVB1 and/or modified form thereof. In certain preferred embodiments, the medicament comprises $1 \times 10^2$ pfu to $1 \times 10^{17}$ pfu of the CVB1 and/or modified form thereof.

In certain preferred embodiments, the medicament comprises a unit dose of the nucleic acid molecule as described herein, for example, comprises $3 \times 10^{10}$ to $3 \times 10^{14}$ virus genome copies of the nucleic acid molecule.

In certain preferred embodiments, the medicament can be administered in combination with an additional therapy. This additional therapy may be any therapy known for tumors, such as surgery, chemotherapy, radiation therapy, immunotherapy, hormone therapy, or gene therapy. This additional therapy can be administered before, at the same time, or after administration of the medicament.

In certain preferred embodiments, the tumor is selected from colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal caner, thyroid cancer, thyroid ductal carcinoma and bladder cancer. In certain preferred embodiments, the tumor is selected from lung cancer, esophageal cancer, ovarian cancer, endometrial cancer, pancreatic cancer, tongue cancer, kidney cancer, prostate cancer, nasopharyngeal cancer, and bladder cancer.

In certain preferred embodiments, the subject is a mammal, such as a human.

In a second aspect, the present invention provides a method for treating a tumor, which comprises a step of administering to a subject in need thereof an effective amount of a CBV1 or modified form thereof, or an effective amount of a nucleic acid molecule; wherein, the nucleic acid molecule comprises a sequence selected from the following:

(1) a genomic sequence or cDNA sequence of the CBV1 or modified form thereof; and

(2) a complementary sequence of the genomic sequence or cDNA sequence.

In certain preferred embodiments, the subject is administered with the CBV1. In certain preferred embodiments, the CBV1 is a wild-type CBV1. In certain preferred embodiments, the CBV1 may be a clinical isolate isolated from an individual infected with a Coxsackievirus B1.

In certain preferred embodiments, the genomic sequence of the CBV1 or modified form thereof has a sequence identity of at least 70%, at least 80%, at least 85%, at least 90%, at least 91%, at least 92%, at least 93%, at least 94%, at least 95%, at least 96%, at least 97%, at least 98%, at least 99%, or 100%, to the nucleotide sequence as shown in SEQ ID NO: 12. In certain preferred embodiments, the genomic sequence of the CBV1 or modified form thereof is the nucleotide sequence as shown in SEQ ID NO: 12.

In certain preferred embodiments, the cDNA sequence of the CBV1 or modified form thereof has a sequence identity of at least 70%, at least 80%, at least 85%, at least 90%, at least 91%, at least 92%, at least 93%, at least 94%, at least 95%, at least 96%, at least 97%, at least 98%, at least 99%, or 100%, to the nucleotide sequence as shown in SEQ ID NO: 1. In certain preferred embodiments, the cDNA sequence of the CBV1 or modified form thereof is the nucleotide sequence as shown in SEQ ID NO: 1.

In certain preferred embodiments, the subject is administered with the modified form of the CBV1. In certain preferred embodiments, the modified form is a modified CBV1, which has a substitution, insertion or deletion of one or more nucleotides in the genome as compared to a wild-type CBV1.

In certain preferred embodiments, the modified CBV1 has one or more modifications selected from the following as compared to a wild-type CBV1:

(1) one or more mutations in an untranslated region (e.g. 5'UTR or 3'UTR);

(2) an insertion of one or more exogenous nucleic acids;

(3) a deletion or mutation of one or more endogenous genes; and

(4) any combination of the above three items.

In certain preferred embodiments, the modified CVB1 comprises one or more mutations in a 5' untranslated region (5'UTR).

In certain preferred embodiments, the modified CVB1 has a substitution of all or part of the 5'UTR sequence. In certain preferred embodiments, the internal ribosome entry site (IRES) sequence in the 5'UTR of the modified CVB1 is replaced with an exogenous IRES sequence, such as an internal ribosome entry site sequence of human rhinovirus 2 (HRV2). In certain preferred embodiments, the internal ribosome entry site sequence of human rhinovirus 2 (HRV2) is shown in SEQ ID NO:2.

In certain preferred embodiments, the modified CVB1 comprises an exogenous nucleic acid.

In certain preferred embodiments, the exogenous nucleic acid encodes a cytokine (e.g., GM-CSF, preferably human GM-CSF), or an anti-tumor protein or polypeptide (e.g., scFv against PD-1 or PD-L1, preferably, scFv against human PD-1 or PD-L1). In certain preferred embodiments, the exogenous nucleic acid is inserted between 5'UTR and VP4 gene, or between VP1 gene and 2A gene of a genome of the modified CVB1.

In certain preferred embodiments, the exogenous nucleic acid comprises a target sequence of microRNA (miRNA) (e.g., miR-133 or miR-206). In certain preferred embodiments, the target sequence of microRNA is inserted in a 3' untranslated region (3'UTR) of a genome of the modified CVB1.

In certain preferred embodiments, the exogenous nucleic acid includes a target sequence of one or more (e.g., 2, 3 or 4) microRNAs as described above. In certain preferred embodiments, the exogenous nucleic acid comprises the target sequence of miR-133 and/or miR-206. In certain preferred embodiments, the target sequence of miR-133 is shown in SEQ ID NO:3. In certain preferred embodiments, the target sequence of miR-206 is shown in SEQ ID NO:4.

In certain preferred embodiments, the modified CVB1 comprises at least one insertion of the exogenous nucleic acid as described above and/or at least one mutation in the untranslated region as described above.

In certain preferred embodiments, the genomic sequence of the modified CVB1 has a sequence identity of at least 70%, at least 80%, at least 85%, at least 90%, at least 91%, at least 92 %, at least 93%, at least 94%, at least 95%, at least 96%, at least 97%, at least 98%, at least 99%, or 100% to a nucleotide sequence selected from the following: nucleotide sequences as shown in SEQ ID NOs: 13-16. In certain preferred embodiments, the genomic sequence of the modified CVB1 is any one selected from the nucleotide sequences as shown in SEQ ID NOs: 13-16.

In certain preferred embodiments, the cDNA sequence of the modified CVB1 has a sequence identity of at least 70%, at least 80%, at least 85%, at least 90%, at least 91%, at least 92 %, at least 93%, at least 94%, at least 95%, at least 96%, at least 97%, at least 98%, at least 99%, or 100% to a nucleotide sequence selected from the following: nucleotide sequences as shown in SEQ ID NOs: 8-11. In certain preferred embodiments, the cDNA sequence of the modified CVB1 is any one selected from the nucleotide sequences as shown in SEQ ID NOs: 8-11.

In certain preferred embodiments, the CVB1 and modified forms thereof as described above may be used in combination. Therefore, one or more of the CVB1 and modified forms thereof can be administered to the subject.

In certain preferred embodiments, the nucleic acid molecule as described herein is administered to the subject.

In certain preferred embodiments, the nucleic acid molecule consists of a genomic sequence or cDNA sequence of the CVB1 or modified form thereof as described herein, or a complementary sequence of the genomic sequence or cDNA sequence. In certain preferred embodiments, the nucleic acid molecule has a genomic sequence of the CVB1 or modified form thereof as described herein. In certain preferred embodiments, the nucleic acid molecule is RNA. In certain preferred embodiments, the nucleic acid molecule has a nucleotide sequence as shown in any one of SEQ ID NOs: 12-16.

In certain preferred embodiments, the nucleic acid molecule is a vector (e.g., cloning vector or expression vector) comprising a genomic sequence or cDNA sequence of the CVB1 or modified form thereof as described herein, or a complementary sequence of the genomic sequence or cDNA sequence. In certain preferred embodiments, the nucleic acid molecule is a vector (e.g., cloning vector or expression vector) comprising a cDNA sequence of the CVB1 or modified form thereof as described herein, or a complementary sequence of the cDNA sequence.

In certain preferred embodiments, the nucleic acid molecule comprises a complementary sequence of the genomic sequence of the CVB1 or modified form thereof. In certain preferred embodiments, the complementary sequence is complementary to a nucleotide sequence selected from the following:

(1) a nucleotide sequence as shown in SEQ ID NO: 12;

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO:12;

(3) a nucleotide sequence as shown in any one of SEQ ID NOs: 13-16; and

(4) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in any one of SEQ ID NOs: 13-16.

In certain preferred embodiments, the nucleic acid molecule comprises a complementary sequence of the cDNA sequence of the CVB1 or modified form thereof. In certain preferred embodiments, the complementary sequence is complementary to a nucleotide sequence selected from the following:

(1) a nucleotide sequence as shown in SEQ ID NO: 1;

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO: 1;

(3) a nucleotide sequence as shown in any one of SEQ ID NOs: 8-11; and

(4) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in any one of SEQ ID NOs: 8-11.

In the present invention, the nucleic acid molecule of the present invention can be delivered by any means known in the art, for example, a naked nucleic acid molecule (e.g., naked RNA) can be directly injected, or a non-viral delivery system can be used. The non-viral delivery system can be obtained from a variety of materials well known in the art, including, but not limited to, the materials described in detail in "Yin H, et al. Nat Rev Genet. 2014 Aug; 15(8): 541-55." and "Riley MK, Vermerris W. Nanomaterials (Basel). 2017 Apr 28; 7(5). Pii: E94.", which are incorporated herein by reference in their entirety, such as liposomes, inorganic nanoparticles (such as gold nanoparticles), polymers (such as PEG).

In certain preferred embodiments, the CVB1 and/or modified form thereof, or nucleic acid molecules as described herein, can be formulated and administered as a pharmaceutical composition. Such pharmaceutical composition may comprise a therapeutically effective amount of the CVB1 and/or modified form thereof, or a therapeutically effective amount of the nucleic acid molecule as described herein. In certain preferred embodiments, the pharmaceutical composition may be in any form known in the medical arts. For example, the pharmaceutical composition may be a tablet, pill, suspension, emulsion, solution, gel, capsule, powder, granule, elixir, lozenge, suppository, injection (including injection liquid, lyophilized powder), and other forms. In some embodiments, the pharmaceutical composition is an injection liquid or a lyophilized powder.

In certain preferred embodiments, the pharmaceutical composition further comprises a pharmaceutically acceptable carrier or excipient. In certain preferred embodiments, the pharmaceutical composition comprises a stabilizer.

In the present invention, the CVB1 and/or modified forms thereof, or the nucleic acid molecules as described herein can be administered to a subject by various suitable routes. In some cases, the administration route of the CVB1 and/or modified form thereof, or the nucleic acid molecules as described herein, depends on the location and type of tumor. For example, for a solid tumor that is easily accessible, the virus or nucleic acid molecule is optionally administered by injection directly into the tumor (e.g., intratumoral injection); for a tumor of hematopoietic system, the virus or nucleic acid molecule can be administered by intravenous or other intravascular routes; for a tumor that is not easily accessible in the body (e.g., metastases), the virus or nucleic acid molecule can be administered systematically so that it can run over the whole body and thereby reaching the tumor (e.g., intravenous or intramuscular injection). Optionally, the virus or nucleic acid molecule of the present invention can be administrated via subcutaneous, intraperitoneal, intrathecal (e.g., for brain tumors), topical (e.g., for melanoma), oral (e.g., for oral or esophageal cancer), intranasal or inhalation spray (e.g., for lung cancer) routes, and the like. In certain preferred embodiments, the CVB1 and/or modified form thereof of the invention, or the nucleic acid as described herein, can be administered via intradermal, subcutaneous, intramuscular, intravenous and oral routes, and the like.

In certain preferred embodiments, the method further comprises administering an additional pharmaceutically active agent having anti-tumor activity. Such additional pharmaceutically active agent may be administered before, simultaneously or after administration of the CVB1 and/or modified form thereof, or the nucleic acid molecule as described herein.

In certain preferred embodiments, the additional pharmaceutically active agent comprises additional oncolytic virus, chemotherapeutic agent, or immunotherapeutic agent.

In the present invention, the additional oncolytic virus includes but is not limited to herpes virus, adenovirus, parvovirus, reovirus, Newcastle disease virus, vesicular stomatitis virus, measles virus, or any combination thereof. The chemotherapeutic agent includes but is not limited to 5-fluorouracil, mitomycin, methotrexate, hydroxyurea, cyclophosphamide, dacarbazine, mitoxantrone, anthracyclines (e.g., epirubicin or doxorubicin), etoposide, platinum compounds (e.g., carboplatin or cisplatin), taxanes (e.g., paclitaxel or docetaxel), or any combination thereof. The immunotherapeutic agent includes but is not limited to immune checkpoint inhibitor (e.g., PD-L1/PD-1 inhibitor or CTLA-4 inhibitor), tumor-specific targeting antibody (e.g., rituximab or herceptin), or any combination thereof.

In certain preferred embodiments, the CVB1 and/or modified form thereof can be administered in any amount from 1 to $1 \times 10^{15}$ pfu/kg of the subject's body weight, for example, the CVB1 and/or modified form thereof can be administered in an amount of at least $1 \times 10^3$ pfu/kg, at least $1 \times 10^4$ pfu/kg, $1 \times 10^5$ pfu/kg, $1 \times 10^6$ pfu/kg, at least $1 \times 10^7$ pfu/kg, at least $1 \times 10^8$ pfu/kg, at least $1 \times 10^9$ pfu/kg, at least $1 \times 10^{10}$ pfu/kg, at least $1 \times 10^{11}$ pfu/kg, or at least $1 \times 10^{12}$ pfu/kg of the subject's body weight. In certain preferred embodiments, the nucleic acid molecule as described herein can be administered in any amount from $3 \times 10^{10}$ to $3 \times 10^{14}$ virus genome copies per kg of the subject's body weight. In certain preferred embodiments, the CVB1 and/or modified form thereof or the nucleic acid molecule as described herein can be administered 3 times per day, 2 times per day, once per day, once every two days, or once per week, and the above-mentioned dosage regimen may be optionally repeated weekly or monthly as appropriate.

In certain preferred embodiments, the method further comprises administering an additional therapy. This additional therapy may be any therapy known for tumors, such as surgery, chemotherapy, radiation therapy, immunotherapy, hormone therapy, or gene therapy. This additional therapy can be administered before, at the same time, or after administration of the method as described above.

In certain preferred embodiments, the subject is a mammal, such as a human.

In certain preferred embodiments, the tumor is selected from colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal cancer, thyroid cancer, thyroid ductal carcinoma and bladder cancer. In certain preferred embodiments, the tumor is selected from lung cancer, esophageal cancer, ovarian cancer, endometrial cancer, pancreatic cancer, tongue cancer, kidney cancer, prostate cancer, nasopharyngeal cancer, and bladder cancer.

In a third aspect, the present invention also relates to a pharmaceutical composition, which comprises the CVB1 and/or modified form thereof as defined in the first or second aspect, or the nucleic acid molecule as defined in the first or second aspect.

In certain preferred embodiments, the pharmaceutical composition may be in any form known in the medical art. For example, the pharmaceutical composition may be a tablet, pill, suspension, emulsion, solution, gel, capsule, powder, granule, elixir, lozenge, suppository, injection (including injection liquid, lyophilized powder), and other forms. In some embodiments, the pharmaceutical composition is an injection liquid or a lyophilized powder.

In certain preferred embodiments, the pharmaceutical composition further comprises a pharmaceutically acceptable carrier or excipient. In certain preferred embodiments, the pharmaceutical composition comprises a stabilizer.

In certain preferred embodiments, the pharmaceutical composition optionally further comprises an additional pharmaceutically active agent. In a preferred embodiment, the additional pharmaceutically active agent is a drug with anti-tumor activity, such as an additional oncolytic virus, chemotherapeutic agent or immunotherapeutic agent.

In certain preferred embodiments, the pharmaceutical composition is used to treat a tumor in a subject.

In certain preferred embodiments, the subject is a mammal, such as a human.

In certain preferred embodiments, the tumor is selected from colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal cancer, thyroid cancer, thyroid ductal carcinoma and bladder cancer. In certain preferred embodiments, the tumor is selected from lung cancer, esophageal cancer, ovarian cancer, endometrial cancer, pancreatic cancer, tongue cancer, kidney cancer, prostate cancer, nasopharyngeal cancer, and bladder cancer.

In a fourth aspect, the invention also relates to the CVB1 and/or modified form thereof as defined in the first or second aspect, or the nucleic acid molecule as defined in the first or second aspect, for use as a medicament.

In a fifth aspect, the present invention provides a modified CVB1 which has a substitution of an internal ribosome entry site (IRES) sequence in a 5'UTR with an internal ribosome entry site sequence of human rhinovirus 2 (HRV2) as compared to a wild-type CVB1.

In certain preferred embodiments, the internal ribosome entry site sequence of human rhinovirus 2 (HRV2) is shown in SEQ ID NO:2.

In certain preferred embodiments, the genomic sequence of the wild-type CVB1 has a sequence identity of at least 70%, at least 80%, at least 85%, at least 90%, at least 91 %, at least 92%, at least 93%, at least 94%, at least 95%, at least 96%, at least 97%, at least 98%, at least 99%, or 100% to the nucleotide sequence as shown in SEQ ID NO: 12. In certain preferred embodiments, the genomic sequence of the wild-type CVB1 is the nucleotide sequence as shown in SEQ ID NO: 12.

In certain preferred embodiments, the cDNA sequence of the wild-type CVB1 has a sequence identity of at least 70%, at least 80%, at least 85%, at least 90%, at least 91 %, at least 92%, at least 93%, at least 94%, at least 95%, at least 96%, at least 97%, at least 98%, at least 99%, or 100% to the nucleotide sequence as shown in SEQ ID NO: 1. In certain preferred embodiments, the cDNA sequence of the wild-type CVB1 is the nucleotide sequence as shown in SEQ ID NO: 1.

In certain preferred embodiments, the modified CVB1 also contains an exogenous nucleic acid.

In certain preferred embodiments, the exogenous nucleic acid encodes a cytokine (e.g., GM-CSF, preferably human GM-CSF), or an anti-tumor protein or polypeptide (e.g., scFv against PD-1 or PD-L1, preferably, scFv against human PD-1 or PD-L1). In certain preferred embodiments, the exogenous nucleic acid is inserted between 5'UTR and VP4 gene, or between VP1 gene and 2A gene of a genome of the modified CVB1.

In certain preferred embodiments, the exogenous nucleic acid comprises a target sequence of microRNA (miRNA) (e.g., miR-133 or miR-206). In certain preferred embodiments, the target sequence of microRNA is inserted in a 3' untranslated region (3'UTR) of a genome of the modified CVB1.

In certain preferred embodiments, the exogenous nucleic acid includes a target sequence of one or more (e.g., 2, 3 or 4) microRNAs as described above. In certain preferred embodiments, the exogenous nucleic acid comprises the target sequence of miR-133 and/or miR-206. In certain preferred embodiments, the target sequence of miR-133 is shown in SEQ ID NO:3. In certain preferred embodiments, the target sequence of miR-206 is shown in SEQ ID NO:4.

In certain preferred embodiments, the modified CVB1 comprises at least one insertion of the exogenous nucleic acid as described above.

In certain preferred embodiments, the genomic sequence of the modified CVB1 has a sequence identity of at least 70%, at least 80%, at least 85%, at least 90%, at least 91%, at least 92 %, at least 93%, at least 94%, at least 95%, at least 96%, at least 97%, at least 98%, at least 99%, or 100% to the nucleotide sequence as shown in SEQ ID NO: 13. In certain preferred embodiments, the genomic sequence of the modified CVB1 is the nucleotide sequence as shown in SEQ ID NO: 13.

In certain preferred embodiments, the cDNA sequence of the modified CVB1 has a sequence identity of at least 70%, at least 80%, at least 85%, at least 90%, at least 91%, at least 92 %, at least 93%, at least 94%, at least 95%, at least 96%, at least 97%, at least 98%, at least 99%, or 100% to the nucleotide sequence as shown in SEQ ID NO: 8. In certain preferred embodiments, the cDNA sequence of the modified CVB1 is the nucleotide sequence as shown in SEQ ID NO: 8.

In certain preferred embodiments, the modified CVB1 is used for treating a tumor in a subject, or for manufacture of a medicament for treating a tumor in a subject.

In certain preferred embodiments, the tumor is selected from colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal cancer, thyroid cancer, thyroid ductal carcinoma and bladder cancer. In certain preferred embodiments, the tumor is selected from lung cancer, esophageal cancer, ovarian cancer, endometrial cancer, pancreatic cancer, tongue cancer, kidney cancer, prostate cancer, nasopharyngeal cancer, and bladder cancer. In certain preferred embodiments, the tumor is thyroid cancer.

In certain preferred embodiments, the subject is a mammal, such as a human.

In a sixth aspect, the present invention provides a nucleic acid molecule comprising a sequence selected from the following:

(1) a genomic sequence or cDNA sequence of the modified CVB1 as described in the fifth aspect; and

(2) a complementary sequence of the genomic sequence or cDNA sequence.

In certain preferred embodiments, the nucleic acid molecule consists of the genomic sequence or cDNA sequence of the modified CVB1 as described above, or a complementary sequence of the genomic sequence or cDNA sequence.

In certain preferred embodiments, the nucleic acid molecule has a genomic sequence of the modified CVB1 as described above. In certain preferred embodiments, the nucleic acid molecule is RNA. In certain preferred embodiments, the nucleic acid molecule has the nucleotide sequence as shown in SEQ ID NO: 13.

In certain preferred embodiments, the nucleic acid molecule is a vector (e.g., cloning vector or expression vector) comprising a genomic sequence or cDNA sequence of the modified CVB1 as described herein, or a complementary sequence of the genomic sequence or cDNA sequence. In certain preferred embodiments, the nucleic acid molecule is a vector (e.g., cloning vector or expression vector) comprising a cDNA sequence of the modified CVB1 as described herein, or a complementary sequence of the cDNA sequence.

In certain preferred embodiments, the nucleic acid molecule comprises a complementary sequence of the genomic sequence of the modified CVB1.In certain preferred embodiments, the complementary sequence is complementary to a nucleotide sequence selected from the following:

(1) a nucleotide sequence as shown in SEQ ID NO: 13; and

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO: 13.

In certain preferred embodiments, the nucleic acid molecule comprises a complementary sequence of the cDNA sequence of the modified CVB1 as described above. In certain preferred embodiments, the complementary sequence is complementary to a nucleotide sequence selected from the following:

(1) a nucleotide sequence as shown in SEQ ID NO: 8; and
(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO: 8.

In certain preferred embodiments, the nucleic acid molecule is used for treating a tumor in a subject, or for manufacture of a medicament for treating a tumor in a subject.

In certain preferred embodiments, the tumor is selected from colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal cancer, thyroid cancer, thyroid ductal carcinoma and bladder cancer. In certain preferred embodiments, the tumor is selected from lung cancer, esophageal cancer, ovarian cancer, endometrial cancer, pancreatic cancer, tongue cancer, kidney cancer, prostate cancer, nasopharyngeal cancer, and bladder cancer. In certain preferred embodiments, the tumor is thyroid cancer.

In certain preferred embodiments, the subject is a mammal, such as a human.

In a seventh aspect, the invention relates to a pharmaceutical composition comprising the modified CVB1 according to the fifth aspect, or the nucleic acid molecule according to the sixth aspect.

In certain preferred embodiments, the pharmaceutical composition may be in any form known in the medical art. For example, the pharmaceutical composition may be tablet, pill, suspension, emulsion, solution, gel, capsule, powder, granule, elixir, lozenge, suppository, injection (including injection liquid, lyophilized powder) and other forms.

In some embodiments, the pharmaceutical composition is an injection liquid or a lyophilized powder.

In certain preferred embodiments, the pharmaceutical composition further comprises a pharmaceutically acceptable carrier or excipient. In certain preferred embodiments, the pharmaceutical composition comprises a stabilizer.

In certain preferred embodiments, the pharmaceutical composition optionally further comprises an additional pharmaceutically active agent. In a preferred embodiment, the additional pharmaceutically active agent is a drug with anti-tumor activity, such as an additional oncolytic virus, chemotherapeutic agent or immunotherapeutic agent.

In certain preferred embodiments, the pharmaceutical composition is used for treating a tumor in a subject.

In certain preferred embodiments, the tumor is selected from colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal cancer, thyroid cancer, thyroid ductal carcinoma and bladder cancer. In certain preferred embodiments, the tumor is selected from lung cancer, esophageal cancer, ovarian cancer, endometrial cancer, pancreatic cancer, tongue cancer, kidney cancer, prostate cancer, nasopharyngeal cancer, and bladder cancer. In certain preferred embodiments, the tumor is thyroid cancer.

In certain preferred embodiments, the subject is a mammal, such as a human.

In an eighth aspect, the present invention also relates to use of the modified CVB1 according to the fifth aspect, or the nucleic acid molecule according to the sixth aspect, for treating a tumor in a subject, or for manufacture of a medicament for treating a tumor in a subject.

In certain preferred embodiments, the tumor is selected from colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal cancer, thyroid cancer, thyroid ductal carcinoma and bladder cancer. In certain preferred embodiments, the tumor is selected from lung cancer, esophageal cancer, ovarian cancer, endometrial cancer, pancreatic cancer, tongue cancer, kidney cancer, prostate cancer, nasopharyngeal cancer, and bladder cancer. In certain preferred embodiments, the tumor is thyroid cancer.

In certain preferred embodiments, the subject is a mammal, such as a human.

In a ninth aspect, the present invention also relates to a method of treating a tumor, which comprises a step of administering to a subject in need thereof an effective amount of the modified CVB1 as described in the fifth aspect, or an effective amount of the nucleic acid molecule as described in the sixth aspect.

In certain preferred embodiments, the tumor is selected from colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal cancer, thyroid cancer, thyroid ductal carcinoma and bladder cancer. In certain preferred embodiments, the tumor is selected from lung cancer, esophageal cancer, ovarian cancer, endometrial cancer, pancreatic cancer, tongue cancer, kidney cancer, prostate cancer, nasopharyngeal cancer, and bladder cancer. In certain preferred embodiments, the tumor is thyroid cancer.

In certain preferred embodiments, the subject is a mammal, such as a human.

## Beneficial effects of the invention

Compared with the prior art, the technical solution of the present invention has at least the following beneficial effects:

The inventors of the present application have found for the first time that CVB1 has a broad-spectrum tumor-killing activity. Based on this finding, the present invention further provides an oncolytic virus based on CVB1, which has higher tumor killing activity and tumor specificity, thus can be used alone in the treatment of tumors, and can also be used as an auxiliary method for traditional tumor treatment, or as a treatment method when other treatment methods are lacking.

The CVB1 or modified form thereof according to the present invention has little or no effect on normal cells, and can be safely administered to a subject (such as a human). Therefore, the CVB1 modified form thereof according to the present invention has great clinical value.

The embodiments of the present invention will be described in detail below in conjunction with the drawings and examples, but those skilled in the art will understand that the following drawings and examples are only used to illustrate the present invention, not to limit the scope of the present invention. The various objects and advantageous aspects of the invention will become apparent to those skilled in the art from the following detailed description of the drawings and preferred embodiments.

**Brief Description of the Drawings**

FIGS. 1A to ID show the micrographs of the in vitro killing experiments in Example 2 of wild type CVB1 on human pancreatic ductal epithelial cell line hTERT-HPNE, human nasopharyngeal carcinoma cell line CNE, human liver cancer cell line HepG2, human endometrial cancer cell line Ishikawa, human breast cancer cell line BT-474, human non-small cell lung cancer cell line EBC-1, human laryngeal cancer cell line HEp-2, human tongue cancer cell line SCC-25, human colorectal cancer cell line HT-29, human ovarian cancer cell line A2780, human pancreatic cancer cell line AsPC-1, and human prostate cancer cell line DU145, in which MOCK indicates cells that were not infected with the virus. The results show that after 72 hours of infection with a multiplicity of infection (MOI) of 1, CVB1 showed a significant oncolytic effect on human tumor cell lines CNE, HepG2, Ishikawa, BT-474, EBC-1, HEp-2, SCC-25, HT-29, A2780, AsPC-1 and DU145, but had no effect on human non-tumor cell hTERT-HPNE.

FIG. 2 shows an electropherogram of one sample of the wild-type CVB1 virus genomic RNA obtained by the in vitro transcription method in Example 2.

FIG. 3 shows the killing effect of the wild-type CVB1 virus genomic RNA on human cervical cancer cell line Hela in Example 2. The results show that the Hela cells transfected with the CVB 1 genomic RNA were almost completely lysed and died 48 hours after the transfection.

FIGS. 4A to 4I show the results of in vivo anti-tumor experiments of the wild-type CVB 1 against human breast cancer cell line BcaP37 (A), human non-small cell lung cancer cell line A549 (B) and SPC-A-1 (C), human Burkitt's lymphoma cell lines Raji (D), human endometrial cancer cell lines Ishikawa (E) and HEC-1-B (F), human cervical cancer cell lines Hela (G) and C-33A (H), and human glioma cell line GBM (I) in Example 3 of the present invention. The results show that in the challenge experiment groups, $10^6$ TCID50 per tumor mass of CVB1 were injected intratumorally every two days. After 5 treatments in total, the growth of the tumors formed by subcutaneous inoculation of BcaP37, A549, SPC-A-1, Raji, Ishikawa, HEC-1-B, Hela, C-33A or GBM cells in SCID mice significantly slowed down and arrested, and the tumors were even lysed and disappeared. In contrast, the tumors of the negative group (CTRL) without treatment of oncolytic virus maintained the normal growth, and their tumor volumes were significantly larger than those in the challenge groups.

FIG. 5 shows the results of in vivo anti-tumor experiments of CVB1-WT, CVB1-HRV2, CVB1-miR133&206T, CVB1-GM-CSF, and CVB1-Anti-PD-1 against human glioma cell line GBM in Example 3. The results showed that, in the challenge experimental groups, $10^6$ TCID50 per tumor mass of CVB1 or modified forms thereof were injected intratumorally every two days. After 5 treatments in total for 10 days, the growth of the tumors formed by subcutaneous inoculation of GBM cells in SCID mice arrested, and the tumors were even lysed and disappeared. In contrast, the tumors of the negative group (CTRL) without treatment of oncolytic virus maintained the normal growth, and their tumor volumes were significantly larger than those in the challenge groups.

Sequence information

Information of parts of sequences involved in the present invention is provided in Table 1 below.

Table 1: Description of the sequences

| SEQ ID NO: | Description |
|---|---|
| 1 | cDNA sequence of wild type CVB1 (CVB1-WT) |
| 2 | RNA sequence of internal ribosome entry site sequence of human rhinovirus 2 (HRV2) |
| 3 | RNA sequence of miR-133 target sequence |
| 4 | RNA sequence of miR-206 target sequence |
| 5 | RNA sequence of tandem sequence of miR-133 target sequence and miR-206 target sequence |
| 6 | DNA sequence of human granulocyte-macrophage colony stimulating factor (GM-CSF) gene |
| 7 | DNA sequence of anti-PD-1 single chain antibody (Anti-PD-1 scFv) |

(continued)

| SEQ ID NO: | Description |
|---|---|
| 8 | cDNA sequence of the modified form of CVB1 (CVB1 -HRV2) |
| 9 | cDNA sequence of the modified form of CVB1 (CVB1-miR133&206T) |
| 10 | cDNA sequence of the modified form of CVB1 (CVB1 -GM-CSF) |
| 11 | cDNA sequence of the modified form of CVB1 (CVB1-Anti-PD1) |
| 12 | Genomic sequence of wild type CVB1 (CVB1-WT) |
| 13 | Genomic sequence of the modified form of CVB1 (CVB1 -HRV2) |
| 14 | Genomic sequence of the modified form of CVB1 (CVB1-miR133&206T) |
| 15 | Genomic sequence of the modified form of CVB1 (CVB 1-GM-CSF) |
| 16 | Genomic sequence of modified form of CVB1 (CVB1-Anti-PD1) |
| 17 | DNA sequence of miR-133 target sequence |
| 18 | DNA sequence of miR-206 target sequence |
| 19 | DNA sequence of tandem sequence of miR-133 target sequence and miR-206 target sequence |
| 20 | DNA sequence of internal ribosome entry site sequence of human rhinovirus 2 (HRV2) |

## Specific Models for Carrying Out the Invention

The present invention will now be described with reference to the following examples intended to illustrate the invention (not to limit the invention).

Unless otherwise specified, the molecular biology experimental methods and immunoassays used in the present invention basically referred to J. Sambrook et al., Molecular Cloning: A Laboratory Manual, 2nd Edition, Cold Spring Harbor Laboratory Press, 1989, and FM Ausubel et al., Short Protocols in Molecular Biology, 3rd Edition, John Wiley & Sons, Inc., 1995. The use of restriction enzymes was in accordance with the conditions recommended by the product manufacturers. If no specific conditions were indicated in the examples, the conventional conditions or the conditions recommended by the manufacturers should be followed. The used reagents or instruments, of which manufacturers were not given, were all conventional products that were commercially available. Those skilled in the art know that the examples describe the present invention by way of example, and are not intended to limit the claimed scope of the invention. All publications and other references mentioned herein are incorporated by reference in their entirety.

Example 1: Acquisition and preparation of CVB1 and modified forms thereof

### 1.1 Isolation of enterovirus CVB 1 from clinical specimens of patients

(1) The pharyngeal and anal swabs of patients were from the Center for Disease Control and Prevention of Xiamen City, China; African green monkey kidney cells (Vero cells; ATCC® Number: CCL-81™) were preserved by the National Institute of Diagnostics and Vaccine Development in Infectious Diseases, Xiamen University, China, and were cultured in MEM medium supplemented with 10% fetal bovine serum, glutamine, penicillin and streptomycin.

(2) Sample processing: the pharyngeal swabs and anal swabs of patients were sufficiently agitated in a specimen preservation solution to wash off the virus and virus-containing cells adhering to the swabs, and then the specimen preservation solution was subjected to high speed centrifugation at 4000 rpm and 4°C for 30 min;

(3) Inoculation and observation:

A. Vero cells were plated in a 24-well plate with $1 \times 10^5$ cells/well. The growth medium (MEM medium, containing 10% fetal bovine serum, as well as glutamine, penicillin and streptomycin) was aspirated, and 1 mL of maintenance medium (MEM medium, containing 2% fetal calf serum, as well as glutamine, penicillin and streptomycin) was added in each well. Then except the negative control wells, each well was inoculated with 50 $\mu$L of the

sample supernatant, and cultured in an incubator at 37°C, 5% $CO_2$.

B. The cells were observed under a microscope every day for one week, and the occurrence of specific cytopathic effect (CPE) in the inoculated wells was recorded.

C. If the enterovirus-specific cytopathic effect appeared in the cells in the inoculated wells within 7 days, the cells and supernatant were collected and frozen at -80 °C; if no CPE appeared after 7 days, the cells were subjected to blind passage.

D. If CPE appeared within 6 blind passages, the cells and supernatant were collected and frozen at -80 °C; If CPE did not appear after 6 blind passages, the cells were determined as negative.

(4) Virus isolation and cloning:
The viruses isolated from the clinical specimens were identified by RT-PCR (Hou et al., Virus Res 2015, 205: 41-44) and specific antibody-based enzyme-linked immunospot assay (ELISPOT) (Yang et al. Clin Vaccine Immunol 2014, 21(3): 312- 320), and Coxsackievirus B1 positive cultures were selected and subjected to at least 3 cloning experiments. The virus clones obtained by the limiting dilution method in each experiment were also identified by RT-PCR and ELISPOT, and Coxsackievirus B1 positive clones were selected and subjected to the next round of cloning. A single strain of Coxsackievirus B1 with strong growth viability were selected as a candidate oncolytic virus strain.

1.2 Obtaining rescued strains of CVB1 and modified forms thereof based on infectious cloning and reverse genetics technology

This example used the wild-type CVB1 (SEQ ID NO: 1) as an example to show how to obtain CVB1 and modified forms thereof used in the present invention by reverse genetics technology. The specific method was as follows.

(1) Construction of viral infectious clones: The cDNA sequence of the wild-type CVB1 (named CVB1-WT) was shown in SEQ ID NO:1, and its genomic RNA sequence was shown in SEQ ID NO:12; or the gene insertion or substitution based on the cDNA of the wild-type CVB1 (SEQ ID NO:1), comprising:

Modified form 1: The internal ribosome entry site sequence of the wild-type CVB1 was replaced with the internal ribosome entry site sequence of human rhinovirus 2 (which has a DNA sequence shown in SEQ ID NO: 20), to obtain the cDNA (SEQ ID NO: 8) of a recombinant virus (named as CVB 1-HRV2), which has a genomic RNA sequence shown as SEQ ID NO: 13;

Modified form 2: The tandem sequence (which has a DNA sequence shown in SEQ ID NO: 19) of the miR-133 target sequence (which has a DNA sequence shown in SEQ ID NO: 17) and the miR-206 target sequence (which has a DNA sequence shown in SEQ ID NO: 18) was inserted between 7303-7304 bp of the 3' untranslated region of the cDNA (SEQ ID NO: 1) of the wild-type CVB1, to obtain the cDNA (SEQ ID NO : 9) of a recombinant virus (named CVB1-miR133&206T), which has a genomic RNA sequence shown as SEQ ID NO: 14;

Modified form 3: The human granulocyte-macrophage colony stimulating factor (GM-CSF) gene (SEQ ID NO: 6) was inserted between the VP1 gene and 2A gene of the cDNA (SEQ ID NO: 1) of wild-type CVB1 to obtain the cDNA (SEQ ID NO: 10) of a recombinant virus (named CVB1-GM-CSF), which has a genomic RNA sequence shown as SEQ ID NO: 15;

Modified form 4: The sequence (SEQ ID NO: 7) encoding the single chain antibody against human programmed death receptor 1 (Anti-PD-1 scFv) was inserted into the VP1 gene and the 2A gene of wild-type CVB1 to obtain the cDNA (SEQ ID NO: 11) of a recombinant virus (named CVB1-Anti-PD-1), which has a genomic RNA sequence shown as SEQ ID NO: 16.

The cDNA sequences (SEQ ID NOs: 1, 8-11) of the above five oncolytic viruses were sent to the gene synthesis company (Shanghai Shenggong Bioengineering Co., Ltd.) for full gene synthesis, and ligated into pSVA plasmids (Hou et al. Human, Virus Res 2015, 205: 41-44), thereby obtaining infectious cloning plasmids of the CVB1 or its modified forms (i.e., CVB1-WT, CVB1-HRV2, CVB1-miR133&206T, CVB1-GM-CSF and CVB1-Anti-PD-1).

(2) Plasmid mini-kit and *E coli*. DH5α competent cells were purchased from Beijing Tiangen Biochemical Technology Co., Ltd.; Hela cells (ATCC® Number: CCL-2™) and human rhabdomyosarcoma cells (RD cells; ATCC® Number:

CCL-136™) were kept by National Institute of Diagnostics and Vaccine Development in Infectious Diseases, Xiamen University, China, and were cultured with DMEM and MEM media respectively, in which 10% fetal bovine serum as well as glutamine, penicillin and streptomycin were added; transfection reagents Lipofactamine2000 and Opti-MEM were purchased from Thermo Fisher Scientific Company.

(3) The infectious cloning plasmids containing the cDNA sequences of the above five oncolytic viruses were transformed into *E coli* DH5$\alpha$ competent cells, the monoclonal strains were picked out and shaken after the outgrowth of clones, and the plasmids were extracted using the plasmid mini-kit, and then sent to the company (Shanghai Biotech Engineering Co., Ltd.) for sequencing analysis.

(4) The infectious cloning plasmids with correct sequence and the helper plasmid pAR3126 were co-transfected into the cells to rescue virus (Hou et al. Virus Res 2015, 205: 41-44). Hela cells were first transfected according to the instructions of the transfection reagent; then observed under a microscope. When CPE appeared in Hela cells, the cells and culture supernatant were harvested, and inoculated with RD cells followed by passaging and culturing. The rescued strains obtained thereby can be used as the candidate strain of oncolytic virus.

Example 2: In vitro anti-tumor experiment of CVB1 and its modified forms

2.1 Viruses and cell lines as used

(1) Viruses: In this example, the CVB1-WT (SEQ ID NO: 12), CVB1-HRV2 (SEQ ID NO: 13), CVB1-miR133&206T (SEQ ID NO: 14), CVB1-GM-CSF (SEQ ID NO: 15) and CVB1-Anti-PD-1 (SEQ ID NO: 16) as provided in Example 1 and a strain of wild-type Coxsackievirus B type 3 (hereinafter referred to as: CVB3-WT; GenBank database accession number : KY286529.1) were used.

(2) Cell lines: human rhabdomyosarcoma cell RD (ATCC® Number: CCL-136™); human colorectal cancer cell lines SW1116 (ATCC® Number: CCL-233™), SW480 (ATCC® Number: CCL-228™ ) and HT-29 (ATCC® Number: HTB-38™); human gastric cancer cell lines AGS (ATCC® Number: CRL-1739™), SGC7901 (CCTCC deposit number: GDC150), BGC823 (CCTCC deposit number: GDC151) and NCI-N87 (ATCC® Number: CRL-5822™); human esophageal cancer cell line TE-1 (purchased from the Cell Resource Center, Shanghai Institute for Biological Sciences, Chinese Academy of Sciences, No. 3131C0001000700089); human small cell lung cancer cell line DMS114 (ATCC® Number: CRL-2066™); human non-small cell lung cancer cell lines SPC-A-1 (CCTCC deposit number: GDC050), NCI-H1975 (ATCC® Number: CRL-5908™), NCI-H1299 (ATCC® Number: CRL-5803™), A549 (ATCC® Number: CCL-185™), NCI-H661 (ATCC® Number: HTB-183™), EBC-1 (Thermo Fisher Scientific, Catalog #: 11875101) and NCI-H1703 (ATCC® Number: CRL-5889™); human liver cancer cell lines C3A (ATCC® Number: CRL-10741™), HepG2 (ATCC® Number: HB-8065™), SMMC7721 (purchased from the Basic Medical Cell Center, Institute of Basic Medical Sciences, Chinese Academy of Medical Sciences, Number: 3111C0001CCC000087), BEL7402 (CCTCC deposit number: GDC035), BEL7404 (purchased from the Cell Resource Center, Shanghai Institutes for Biological Sciences, Chinese Academy of Sciences, number: 3131C0001000700064), Huh7 (CCTCC deposit number: GDC134) and PLC/PRF/5 (ATCC® Number: CRL-8024™); human ovarian cancer cell lines SKOV3 (ATCC® Number: HTB-77™) and Caov3 (ATCC® Number: HTB-75™); human endometrial cancer cell lines Hec-1-A (ATCC® Number: HTB-112™), Hec-1-B (ATCC® Number: HTB-113™) and Ishikawa (ECACC No. 99040201); human cervical cancer cell lines Hela (ATCC® Number: CCL-2™), Caski (ATCC® Number: CRL-1550™) and C-33A (ATCC® Number: HTB-31™); human melanoma cell lines SK-MEL-1 (ATCC® Number: HTB-67™) and MeWo (ATCC® Number: HTB-65™); human breast cancer cell lines BcaP37 (CCTCC deposit number: GDC206), BT-474 (ATCC® Number: HTB-20™) and MDA-MB-231 (ATCC® Number: HTB-26™); human kidney cancer cell lines A-498 (ATCC® Number: HTB-44™) and 786-O (ATCC® Number: CRL-1932™); human pancreatic cancer cell lines Capan-2 (ATCC® Number: HTB-80™), AsPC-1 (ATCC® Number: CRL-1682™), SW1990 (ATCC® Number: CRL-2172™), HPAF-2 (ATCC® Number: CRL-1997™) and CFPAC-1 (ATCC® Number: CRL-1918™); human osteosarcoma cell line U2OS (ATCC® Number: HTB-96™); human prostate cancer cell lines DU145 (ATCC® Number: HTB-81™) and LNCap (ATCC® Number: CRL-1740™); human neuroglioma cell line GBM (primary tumor cell line isolated from a patient tumor tissue); human neuroblastoma cell line SH-SY5Y (ATCC® Number: CRL-2266™); human tongue squamous carcinoma cell lines CAL27 (ATCC® Number: CRL-2095™) and SCC-25 (ATCC® Number: CRL-1628™); human nasopharyngeal carcinoma cell line CNE (purchased from the Center for Basic Medical Cells, Institute of Basic Medical Sciences, Chinese Academy of Medical Sciences, No.: 3131C0001000700013); human nasal septum squamous cell carcinoma cell line RPMI 2650 (ATCC® Number: CCL-30™); human laryngeal carcinoma cell line HEp-2 (ATCC® Number: CCL-23™); human thyroid cancer cell lines SW579 (preserved by the National Engineering Research Center for Diagnostic Reagents and Vaccines for Infectious Disease) and human thyroid ductal carcinoma cell line TT (ATCC® Number: CRL-1803™); human bladder cancer cell lines J82 (ATCC® Number: HTB-1™) and 5637 (ATCC® Number: HTB-9™); human Burkitt's lymphoma cell lines Daudi (ATCC® Number: CCL-213™) and Raji (ATCC® Number: CCL-86™); human normal cell lines including: human pancreatic ductal epithelial cell line hTERT-HPNE (ATCC® Number: CRL-4023™), human skin keratinocyte cell line HaCat (CCTCC, deposit number: GDC106), human embryonic lung fibroblast cell line MRC-5 (ATCC® Number: CCL-171™), human foreskin fibroblast cell line HFF-1 (ATCC® Number: SCRC-1041™), human prostate stromal cell line WPMY-1 (ATCC® Number: CRL-2854™), human umbilical vein endothelial cell line HUVEC (Thermo Fisher Scientific, Catalog #: C01510C) and the differentiated human liver progenitor cell line HepaRG (with the characteristics of primary hepatocytes; Thermo Fisher Scientific, Catalog #: HPRGC10). The above cells were all preserved by National Institute of Diagnostics and Vaccine Development in Infectious Diseases, Xiamen University, China. HepaRG cells were cultured in WME medium (added with 1.5% DMSO); AGS and TT were cultured in F-12K medium; SH-SY5Y was cultured in DMEM:F12 (1:1) medium; CFPAC-1 was cultured in IMDM medium; RD, C-33A, EBC-1, SK-MEL-1, J82 and DU145 were cultured in MEM medium; Raji, Daudi, 5637, 786-O, TE-1, Caski, NCI-H1299, NCI-H1703, NCI-H1975, NCI-H661, SGC7901, BGC823, SW1116, HEp-2 and LNCap were cultured in RPMI-1640 medium; and other cells were cultured in DMEM medium. These mediums were all supplemented with 10% fetal bovine serum, glutamine and penicillin-streptomycin. All the above cells were cultured under standard conditions of 37 °C and 5% $CO_2$.

2.2 Cultivation of virus

The RD cells were evenly plated on 10 cm cell culture plates, under the culturing conditions of MEM medium containing 10% fetal bovine serum, glutamine, penicillin and streptomycin, 37 °C, 5% $CO_2$, and saturated humidity. When the cell confluence reached 90% or more, the cell culture medium was replaced with serum-free MEM medium, and each plate was inoculated with $10^7$ TCID50 of CVB1-WT, CVB1-HRV2, CVB1-miR133&206T, CVB1-GM-CSF or CVB1-Anti-PD-1. After 24 hours of continuous cultivation, CVB1 or its modified forms proliferated in RD cells and caused CPE in the cells. When more than 90% of the cells turned contracted and rounded, showed increased graininess, and became detached and lysed, the cells and their culture supernatants were harvested. After freeze-thawing for three cycles, the culture supernatants were collected and centrifuged to remove cell debris, under the centrifugation conditions of 4000 rpm, 10 min, and 4°C. Finally, the supernatants were filtered with 0.22μm disposable filter (Millipore) to remove all cell debris and other impurities.

2.3 Determination of virus titer

The RD cells were coated in a 96-well plate with a cell density of $10^4$ cells/well. After the cells adhered, the virus solution obtained in Example 2.2 was subjected to a 10-fold gradient dilution starting at 10-fold with serum-free MEM medium. 50μl of the diluted virus was added to the wells with cells. After 7 days, the wells where CPE appeared were monitored and recorded, followed by calculation using Karber method, in which the calculation formula was $lg^{TCID50} = L - D (S-0.5)$, L: logarithm of the highest dilution, D: difference between logarithms of dilutions, S: sum of proportions of positive wells. The unit of TCID50 thereby calculated was TCID50/50μl, which should be converted into TCID50/ml.

2.4 In vitro anti-tumor experiments of viruses

The human tumor cells and normal cells were inoculated into 96-well plates at $10^4$ per well. After the cells adhered, the medium in each well was replaced with corresponding cell culture medium without serum, and viruses were inoculated at MOIs of 10, 1, 0.1 and 0.01, respectively. Then, CPE of the cells were monitored daily by a microscope.

FIGS. 1A to ID showed the micrographs of the human pancreatic ductal epithelial cell line hTERT-HPNE, human nasopharyngeal carcinoma cell line CNE, human liver cancer cell line HepG2, human endometrial cancer cell line Ishikawa, human breast cancer cell line BT-474, human non-small cell lung cancer cell line EBC-1, human laryngeal cancer cell line HEp-2, human tongue cancer cell line SCC-25, human colorectal cancer cell line HT-29, human ovarian cancer cell line A2780, human pancreatic cancer cell line AsPC-1 and human prostate cancer cell line DU145, which were not infected with viruses (negative control group, Mock) or which were treated with CVB1 -WT at MOI = 1 for 72 hours. The results showed that after 72 hours of infection with a multiplicity of infection (MOI) of 1, a significant reduction in the number of the tumor cells, marked shrinking and lysis and the like, were detected in the virus-infected groups; while as compared to the non-tumor cells in the Mock group, the non-tumor cells infected with the viruses showed almost no change in cell morphology. The above results indicated that CVB1 showed significant oncolytic effects on human nasopharyngeal cancer cell line CNE, human liver cancer cell line HepG2, human endometrial cancer cell line Ishikawa, human breast cancer cell line BT-474, human non-small cell lung cancer cell line EBC-1, human laryngeal cancer cell line HEp-2, human tongue cancer cell line SCC-25, human colorectal cancer cell line HT-29, human ovarian cancer cell line A2780, human pancreatic cancer cell line AsPC-1 and human prostate cancer cell line DU145, but had no effect on the non-tumor cells such as human pancreatic ductal epithelial cell line hTERT-HPNE.

Cell Counting Kit-8 (CCK-8 kit; Shanghai Biyuntian Biotechnology Co., Ltd.) was used to detect cell survival rate after 72 hours of virus infection and culture. The specific methods were as follows:

For adherent cells, the original medium in a 96-well cell culture plate was directly discarded;

for suspension cells, the original medium in a 96-well cell culture plate was carefully discarded after centrifugation; and then 100 µl of fresh serum-free medium was added per well. 10 µl of CCK-8 solution was added to each of the wells inoculated with cells, and an equal amount of CCK-8 solution was also added to the blank culture medium as a negative control, followed by incubation at 37 °C in a cell culture incubator for 0.5-3 hours. The absorbance was detected at 450 nm using a microplate reader at 0.5, 1, 2, 3 hours, respectively, and the time point where the absorbance was within a suitable range was selected as a reference for cell survival rate. The CCK-8 test results of CVB1-WT for each kind of cells were shown in Table 2, where "-" indicated that the cell survival rate after virus treatment was not significantly different from that of the MOCK group; "+" indicated that after virus treatment, the cell number was reduced, the survival rate was still greater than 50% but was significantly different from that of the MOCK group; "++" indicated that the cell survival rate after virus treatment was less than 50%, and was significantly different from that of the MOCK group.

The calculation of cell survival rate is:

$$Cell_survival_rate(\%) = \frac{(reading_of_test_group - reading_of_negative_group)}{(reading_of_positive_group - reading_of_negative_group)} \times 100\%$$

Table 2: Results of in vitro anti-tumor test of CVB1-WT

| Cell lines \ MOI | 10 | 1 | 0.1 | 0.01 |
|---|---|---|---|---|
| RD | ++ | ++ | ++ | + |
| SW1116 | ++ | ++ | ++ | + |
| SW480 | ++ | ++ | ++ | ++ |
| HT29 | ++ | ++ | ++ | ++ |
| AGS | ++ | ++ | ++ | ++ |
| SGC7901 | ++ | ++ | ++ | + |
| BGC823 | ++ | ++ | ++ | ++ |
| NCI-N87 | ++ | ++ | ++ | - |
| TE-1 | ++ | ++ | ++ | + |
| DMS114 | ++ | ++ | ++ | ++ |
| SPC-A-1 | ++ | ++ | ++ | ++ |
| NCI-H1975 | ++ | ++ | ++ | + |
| NCI-H1299 | ++ | ++ | ++ | ++ |
| A549 | ++ | ++ | ++ | + |

| | | | | |
|---|---|---|---|---|
| NCI-H661 | ++ | ++ | ++ | ++ |
| EBC-1 | ++ | ++ | ++ | ++ |
| NCI-H1703 | ++ | ++ | ++ | + |
| C3A | ++ | ++ | ++ | ++ |
| HepG2 | ++ | ++ | ++ | ++ |
| SMMC7721 | ++ | ++ | ++ | ++ |
| BEL7404 | ++ | ++ | ++ | ++ |
| BEL7402 | ++ | ++ | ++ | ++ |
| Huh7 | ++ | ++ | ++ | ++ |
| PLC/PRF/5 | ++ | ++ | ++ | ++ |
| SKOV3 | ++ | ++ | + | - |
| CaOV3 | ++ | ++ | ++ | - |
| HEC-1-A | ++ | ++ | ++ | + |
| HEC-1-B | ++ | ++ | ++ | ++ |
| Ishikawa | ++ | ++ | ++ | ++ |
| Hela | ++ | ++ | ++ | ++ |
| CaSki | ++ | ++ | ++ | ++ |
| C-33A | ++ | ++ | ++ | ++ |
| SK-MEL-1 | ++ | ++ | ++ | + |
| MeWo | ++ | ++ | + | - |
| BcaP37 | ++ | ++ | ++ | ++ |
| BT-474 | ++ | ++ | ++ | ++ |
| MDA-MB-231 | ++ | ++ | + | - |
| A498 | ++ | ++ | ++ | + |
| 786-O | ++ | ++ | + | - |
| Capan-2 | ++ | ++ | + | - |
| HPAF-2 | ++ | ++ | + | + |
| AsPC-1 | ++ | ++ | ++ | ++ |
| SW1990 | ++ | ++ | ++ | + |
| CFPAC-1 | ++ | ++ | ++ | + |
| U2OS | ++ | + | - | - |
| DU145 | ++ | ++ | ++ | ++ |
| LNCap | ++ | ++ | ++ | + |
| GBM | ++ | ++ | ++ | ++ |
| SH-SY5Y | ++ | ++ | ++ | ++ |
| CAL27 | ++ | ++ | ++ | + |
| SCC-25 | ++ | ++ | ++ | ++ |
| CNE | ++ | ++ | ++ | + |
| RPMI 2650 | ++ | ++ | + | + |
| HEp-2 | ++ | ++ | ++ | ++ |
| TT | ++ | ++ | ++ | - |

| | | | | |
|---|---|---|---|---|
| SW579 | ++ | + | - | - |
| J82 | ++ | + | - | - |
| 5637 | ++ | ++ | ++ | ++ |
| Daudi | ++ | ++ | + | - |
| Raji | ++ | ++ | + | - |
| hTERT-HPNE | + | - | - | - |
| differentiated RG | + | - | - | - |
| Hacat | - | - | - | - |
| MRC-5 | - | - | - | - |
| HFF-1 | - | - | - | - |
| wpmy-1 | - | - | - | - |
| HUVEC | - | - | - | - |

Note: "-" indicated that there was no significant difference in cell survival rate between virus treatment group and MOCK group; "+" indicated that after virus treatment, the number of cells was reduced, the survival rate was greater than 50% but was significantly different from that of MOCK group; "++" indicated that the cell survival rate after virus treatment was less than 50%, and was significantly different from that of the MOCK group.

It can be seen from Table 2 that CVB1-WT had killing effect on most of the detected tumor cells. In particular, the virus had significant killing effects on colorectal cancer cell lines, gastric cancer cell lines, lung cancer cell lines, liver cancer cell lines, cervical cancer cell lines, endometrial cancer cell lines, pancreatic cancer cell lines, prostate cancer cell lines, nasopharyngeal cancer cell lines, tongue cancer cell lines, laryngeal cancer cell lines, glioma cell lines and neuroblastoma cell lines. On the other hand, the virus was substantially non-toxic to the non-tumor cell lines tested, including the human embryonic lung fibroblast cell line MRC-5, human foreskin fibroblast cell line HFF-1, human skin keratinocyte cell line HaCat, human prostate stromal cell line WPMY-1, and human umbilical vein endothelial cell line HUVEC, except that it had certain toxicity to human normal pancreatic ductal epithelial cell line hTERT-HPNE and the differentiated human liver progenitor cell line HepaRG at MOI=10.

It is particularly worth noting that although CVB1-WT and the wild-type Coxsackievirus B type 3 strain (CVB3-WT; GenBank database accession number: KY286529.1) reported to have certain killing activity on specific tumors, belong to Coxsackie viruses, the genome-wide nucleotide homology between the two was only 72.8%, and the nucleotide homology of coding region was only 71%, that was, they were two completely different viruses. In particular, the inventors found that CVB1 had a significantly superior tumor killing effect, and had killing activities on most of the detected tumor cells at least tens of times, or even hundreds of times, as compared with CVB3, by comparing the killing efficacies of CVB1-WT and CVB3-WT on different types of tumor cells (Table 3). It can be seen from this that CVB1 of the present invention can produce more potent anti-tumor activity at a relatively lower dose, which could greatly improve the safety of administration while ensuring the therapeutic efficacy, and thus is particularly suitable for anti-tumor treatment.

Table 3: Comparison of results of in vitro anti-tumor tests of CVB1-WT and CVB3-WT

| Cell lines | | CVB1-WT MOI 1 | CVB1-WT MOI 0.1 | CVB1-WT MOI 0.01 | CVB1-WT EC50 (MOI) | CVB3-WT MOI 1 | CVB3-WT MOI 0.1 | CVB3-WT MOI 0.01 | CVB3-WT EC50 (MOI) | Potency multiple (EC50 ratio) |
|---|---|---|---|---|---|---|---|---|---|---|
| Lung cancer cell line | NCI-H1975 | ++ | ++ | + | 0.02 | ++ | - | - | 0.97 | 48.5 |
| | A549 | ++ | ++ | + | 0.02 | ++ | + | + | 0.82 | 41 |
| | EBC-1 | ++ | ++ | ++ | <0.01 | ++ | ++ | + | 0.08 | >8 |
| | NCI-H1703 | ++ | ++ | + | 0.03 | ++ | + | + | 0.89 | 29.6 |
| Esophageal cancer cell line | TE-1 | ++ | ++ | + | 0.04 | + | - | - | 15.23 | 380.75 |
| Ovarian cancer cell line | CaOV3 | ++ | ++ | - | 0.03 | ++ | + | - | 0.78 | 26 |
| Endometrial cancer cell line | HEC-1-A | ++ | ++ | + | 0.05 | + | - | - | 13.34 | 266.8 |
| Pancreatic cancer cell line | Capan-2 | ++ | + | - | 0.08 | - | - | - | >10 | >125 |
| | AsPC-1 | ++ | ++ | ++ | <0.01 | ++ | + | - | 0.96 | >96 |
| | SW1990 | ++ | ++ | + | 0.02 | ++ | + | - | 0.82 | 41 |
| | CFPAC-1 | ++ | ++ | + | 0.03 | + | - | - | 25.71 | 857 |
| Tongue cancer cell line | CAL27 | ++ | ++ | + | 0.03 | + | - | - | 17.20 | 573.3 |
| | SCC-25 | ++ | ++ | ++ | <0.01 | ++ | + | - | 0.78 | >78 |
| Renal cancer cell line | 786-O | ++ | + | - | 0.05 | + | - | - | 17.84 | 356.8 |
| Prostate cancer cell line | DU145 | ++ | ++ | ++ | <0.01 | ++ | ++ | + | 0.05 | >5 |

| | | | | | | | | | | |
|---|---|---|---|---|---|---|---|---|---|---|
| Nasopharyngeal cancer cell line | CNE | ++ | ++ | + | 0.03 | ++ | + | - | 0.88 | 29.33 |
| Bladder cancer cell line | 5637 | ++ | ++ | ++ | <0.01 | ++ | + | - | 0.53 | 53 |

Note: "-" indicated that there was no significant difference in cell survival rate between virus treatment group and MOCK group; "+" indicated that after virus treatment, the number of cells was reduced, the survival rate was greater than 50% but was significantly different from that of MOCK group; "++" indicated that the cell survival rate after virus treatment was less than 50%, and was significantly different from that of the MOCK group; EC50, half of effective dose, referring to herein as the MOI of viruses by which the cell survival rate drops to 50%; potency multiple, referring to herein as the multiple of oncolytic efficacy of CVB1 and CVB3 on specific cells, that is, the EC50 ratio.

In addition, the results of in vitro anti-tumor experiments of CVB1-miR133&206T, CVB1-GM-CSF and CVB1-Anti-PD-1 showed that the above-mentioned modified forms of CVB1 all retained the killing effect of the parental strain of wild-type CVB1 on the detected tumor cells, and the significant killing effects of the parental strain of wild-type CVB1 on colorectal cancer cell lines, gastric cancer cell lines, lung cancer cell lines, liver cancer cell lines, cervical cancer cell lines, endometrial cancer cell lines, pancreatic cancer cell lines, prostate cancer cell lines, nasopharyngeal cancer cell lines, tongue cancer cell lines, laryngeal cancer cell lines, glioma cell lines, and neuroblastoma cell lines, in which the results of CCK-8 detection of the oncolytic activities to human colorectal cancer cell line SW480, human gastric cancer cell line AGS, human endometrial cancer cell line Ishikawa line and human glioma cell line GBM were shown in Table 4.

It is particularly worth noting that CVB 1-HRV2 has significant killing activity to some tumor cells to which CVB1-WT showed weak killing activity, and brings a significant beneficial technical effect; in which the results of CCK-8 detection of the oncolytic activity to human thyroid cancer cell line SW579 were shown in Table 5.

Table 4: Results of in vitro anti-tumor tests of CVB1-miR133&206T, CVB1-GM-CSF and CVB1-Anti-PD-1

| Cell lines \ MOI | | 10 | 1 | 0.1 | 0.01 |
|---|---|---|---|---|---|
| CVB1-miR133&206T | SW480 | ++ | ++ | ++ | ++ |
| | AGS | ++ | ++ | ++ | ++ |
| | Ishikawa | ++ | ++ | ++ | ++ |
| | GBM | ++ | ++ | ++ | ++ |
| CVB1-GM-CSF | SW480 | ++ | ++ | ++ | ++ |
| | AGS | ++ | ++ | ++ | ++ |
| | Ishikawa | ++ | ++ | ++ | ++ |
| | GBM | ++ | ++ | ++ | ++ |
| CVB1-Anti-PD-1 | SW480 | ++ | ++ | ++ | ++ |
| | AGS | ++ | ++ | ++ | ++ |
| | Ishikawa | ++ | ++ | ++ | ++ |
| | GBM | ++ | ++ | ++ | ++ |

Note: "-" indicated that there was no significant difference in cell survival rate between virus treatment group and MOCK group; "+" indicated that after virus treatment, the number of cells was reduced, the survival rate was greater than 50% but was significantly different from that of MOCK group; "++" indicated that the cell survival rate after virus treatment was less than 50%, and was significantly different from that of the MOCK group.

Table 5: Comparison of results of in vitro oncolytic tests of CVB1-WT and CVB1-HRV2 on human thyroid cancer cell line SW579

| Cell lines \ MOI | 10 | 1 | 0.1 | 0.01 |
|---|---|---|---|---|
| CVB1-WT | ++ | + | - | - |
| CVB1-HRV2 | ++ | ++ | ++ | + |

Note: "-" indicated that there was no significant difference in cell survival rate between virus treatment group and MOCK group; "+" indicated that after virus treatment, the number of cells was reduced, the survival rate was greater than 50% but was significantly different from that of MOCK group; "++" indicated that the cell survival rate after virus treatment was less than 50%, and was significantly different from that of the MOCK group.

2.5 Serial passaging of CVB1 for adaptation

In this example, CVB1 was serially passaged for adaptation in a certain tumor cell to obtain a strain with enhanced killing activity on the tumor cell.

The wild-type enterovirus CVB1 was serially passaged for adaptation in human osteosarcoma cell line U2OS, human thyroid cancer cell line SW579 and human bladder cancer cell line J82, on which the oncolytic effect of CVB1 was not very significant. The specific methods were as follows:

One kind of the above tumor cells was evenly plated on a 10 cm cell culture plate, and the culture conditions were a corresponding cell culture medium containing 10% fetal bovine serum, glutamine, penicillin and streptomycin, 37 °C, 5% $CO_2$, saturated humidity. When the cell confluence reached 90% or more, the cell culture medium was replaced with serum-free cell culture medium, each plate was inoculated with $10^7$ TCID50 of CVB1 virus, and the culture environment was changed to 33 °C, 5% $CO_2$, saturated humidity. When CVB1 proliferated in tumor cells and caused CPE in the cells (after infection for up to 3 day), the cells and their culture supernatant were harvested. After freeze-thawing for three cycles, centrifugation was performed at 4000 rpm for 10 min at 4°C. The centrifugal supernatant was taken and added onto new tumor cells with a confluence of more than 90%, to complete one round of virus passage. The passage was repeated for more than 10 times in this way, and a part of the virus solution of each round of passage was taken out for titration of virus in RD cells, and the specific method referred to Example 2.3. Generally, the virus replication capacity would increase with the increase of generations, and when a relatively high infectious titer was reached and the virus replication was stable in the tumor cell, the adapted strain of CVB1 for the tumor cells was obtained.

Subsequently, the human tumor cells U2OS, SW579 or J82 were inoculated to 96-well plates at $10^4$ cells/well by the method of the in vitro anti-tumor experiment described in Example 2.4. After the cells adhered, the medium in each well was replaced with the corresponding cell culture medium free of serum, followed by incubation at 37°C for 30 min, and then the serially passaged CVB1 strains adapted for each of the above kinds of cells were inoculated at MOIs of 10, 1, 0.1, and 0.01 (the viral titers were detected on RD cells), respectively. Subsequently, CPE of the cells were monitored daily by a microscope, and the cell survival rate was detected using CCK-8 method 72 hours after the infection and culture of viruses.

The results were shown in Table 6. After serial passaging of the wild-type CVB1 in a kind of tumor cells on which CVB1 had poor oncolytic effect, its killing activity on the tumor cell was significantly enhanced, indicating that the CVB1 adapted strain with enhanced oncolytic effect on the tumor cells could be obtained by the above-mentioned serial passaging method.

Table 6: Results of in vitro killing experiment of CVB1 on a tumor cell after serial passaging for adaptation in the tumor cell

| MOI / Cell lines | 10 | 1 | 0.1 | 0.01 |
|---|---|---|---|---|
| U2OS | ++ | ++ | ++ | - |
| SW579 | ++ | ++ | + | + |
| J82 | ++ | ++ | ++ | + |

Note: "-" indicated that there was no significant difference in cell survival rate between virus treatment group and MOCK group; "+" indicated that after virus treatment, the number of cells was reduced, the survival rate was greater than 50% but was significantly different from that of MOCK group; "++" indicated that the cell survival rate after virus treatment was less than 50%, and was significantly different from that of the MOCK group.

2.6 Evaluation of oncolytic effect of genomic RNA of CVB1

In this example, a large amount of infectious live viruses of CVB1 could be produced by transfecting the purified genomic RNA of CVB1 into a certain kind of tumor cells, and thus kill the tumor cells.

The viral genomic RNA was first obtained by in vitro transcription, and this method could be found in, for example, Hadac E M, Kelly E J and Russell S J. Mol Ther, 2011, 19(6): 1041-1047. Specifically, the infectious cloning plasmid of wild-type CVB1 obtained in Example 1 was linearized, and the linearized plasmid was used as a template for in vitro transcription using MEGAscript™ T7 Transcription Kit (Thermo Fisher Scientific, AM1333) so as to produce a large amount of viral RNA. And the obtained viral RNA was purified using MEGAclear™ Transcription Clean-Up Kit (Thermo Fisher Scientific, AM1908) for next use. The RNA electropherogram of one sample was shown in FIG. 2.

Subsequently, according to the method of the in vitro anti-tumor experiment described in Example 2.4, the human cervical cancer tumor cell line Hela was inoculated to a 24-well plate at $10^5$ cells/well. After the cells adhered, the medium in each well was replaced with a corresponding cell culture medium free of serum, followed by incubation at 37 °C for 30 min. Then Hela cells were transfected with purified virus RNA at 1 μg per well using transfection reagent Lipofectamine® 2000 (Thermo Fisher Scientific, 11668019), and the negative control group was transfected with irrelevant RNA nucleic acid molecules. Subsequently, CPE of the cells were monitored daily by a microscope.

The results showed that CPE began to appear in the Hela cells transfected with genomic RNA of CVB1 about 8 hours after transfection, and then the cytopathy gradually increased. After 48 hours, the survival rate was measured using the CCK8 method, the Hela cells had almost all died and lysed. And the micrographs of Hela cells at 0 and 48 hours after infection were shown in FIG. 3. The culture supernatant was inoculated into new Hela cells and CPE was quickly produced. The results indicated that the direct administration with the nucleic acid of CVB1 also had good killing activity and could be used to treat tumors.

Example 3: In vivo anti-tumor experiment of CVB1 and modified forms thereof

<u>3.1 Viruses, cell lines and laboratory animals</u>

(1) Viruses: In this example, the CVB1-WT (SEQ ID NO: 12), CVB1-HRV2 (SEQ ID NO: 13), CVB1-miR133&206T (SEQ ID NO: 14), CVB1-GM-CSF (SEQ ID NO: 15) and CVB1-Anti-PD-1 (SEQ ID NO: 16) provided in Example 1 were used. For the virus culture and virus titer determination methods, see Examples 2.2 and 2.3, respectively.

(2) Cell lines: human breast cancer cell line BcaP37 (CCTCC deposit number: GDC206), human non-small cell lung cancer cell lines A549 (ATCC® Number: CCL-185™) and SPC-A-1 (CCTCC deposit number: GDC050), human Burkitt's lymphoma cell line Raji (ATCC® Number: CCL-86™), human endometrial cancer cell lines Ishikawa (ECACC No. 99040201) and HEC-1-B (ATCC® Number: HTB-113™), human cervical cancer cell lines Hela (ATCC® Number: CCL-2™) and C-33A (ATCC® Number: HTB-31™), and human glioma cell line GBM (primary tumor cell line isolated from patient tumor tissue). Except that Raji was cultured using RPMI-1640 medium and C-33A was cultured using MEM medium, the above cells were cultured using DMEM medium, and the above mediums were added with 10% fetal bovine serum, glutamine and penicillin-streptomycin. All the above cells were cultured under standard conditions of 37 °C and 5% $CO_2$.

(3) Laboratory animals: 6-8-week-old female C.B17 SCID mice were from Shanghai Silaike Experimental Animal Co., Ltd.; according to the protocol approved by Experimental Animal Center and Ethics Committee, Xiamen University, the mice were raised under SPF conditions.

<u>3.2 In vivo anti-tumor experiments of viruses</u>

The tumor cells used for subcutaneous tumor formation in SCID mice were digested with 0.01% trypsin, and then resuspended into a single cell suspension using cell culture medium containing 10% fetal bovine serum. The cell density of the suspension was counted. The cells were precipitated by centrifugation under 1000 g for 3 min, and then the cells were resuspended with an appropriate volume of PBS to reach a concentration of about $10^6$-$10^7$ cells/100 μl PBS. The tumor cells were subcutaneously inoculated in the back of SCID mice at $10^6$-$10^7$ cells/100 μl PBS/site with a syringe. When the tumor cells formed a tumor mass of approximately 100 $mm^3$ under the skin of SCID mice after about 14-21 days, the tumor-bearing SCID mice were randomly divided into experimental groups (administrated with CVB1-WT, CVB1-HRV2, CVB1-miR133&206T, CVB1-GM-CSF or CVB1-Anti-PD-1) and negative control group, with 4 animals per group (n=4). Oncolytic virus (CVB1-WT, CVB1-HRV2, CVB1-miR133&206T, CVB1-GM-CSF or CVB1-Anti-PD-1) at $10^6$ TCID50/100μl serum-free medium/tumor mass or equivalent amount of serum-free medium were intratumorally injected every two days, for a total of 5 treatments. The tumor size was measured with a vernier caliper and recorded every two days, and the method for calculating the tumor size was:

$$\text{Tumor size (mm}^3\text{)} = \text{tumor length value} \times (\text{tumor width value})^2/2.$$

The treatment results of CVB1-WT on the above six tumors were shown in FIGS. 4A to 4I, respectively. The results showed that after the challenge of CVB1-WT, the growth of the detected tumors of BcaP37 (A), A549 (B), SPC-A-1 (C), Raji (D), Ishikawa (E), HEC-1-B(F) , Hela (G), C-33A (H) and GBM (I) gradually slowed down and arrested, and the tumors were even lysed and disappeared; in contrast, the tumors in the negative group (CTRL) maintained normal growth, and the tumor sizes were significantly larger than those in the experimental groups.

FIG. 5 showed the results obtained after a treatment of the GBM tumor model with CVB1-WT, CVB1-HRV2, CVB1-miR133&206T, CVB1-GM-CSF, or CVB1-Anti-PD-1 for 10 days. The results showed that, as compared with the negative control group without oncolytic virus treatment, the tumors were significantly reduced in volume and even almost disappeared after being treated with CVB1-WT, CVB1-HRV2, CVB1-miR133&206T, CVB1-GM-CSF and CVB1-Anti-PD-1 respectively, and the reduction extents in tumor volume after treatment with the five oncolytic viruses were similar. The above results indicated that CVB 1-WT, CVB1-HRV2, CVB1-miR133&206T, CVB1-GM-CSF and CVB1-Anti-PD-1 all exhibited remarkable and favorable anti-tumor activity in vivo.

Although the specific embodiments of the present invention have been described in detail, those skilled in the art will understand that various modifications and changes can be made to the details based on all the teachings that have been published, and these changes are within the scope of the present invention. The entirety of the invention is given by the appended claims and any equivalents thereof.

## Claims

1. Use of a Coxsackievirus B1 (CVB1) or a modified form thereof or a nucleic acid molecule for treating a tumor in a subject, or for manufacture of a medicament for treating a tumor in a subject; wherein the nucleic acid molecule comprises a sequence selected from the following:

   (1) a genomic sequence or cDNA sequence of the CVB1 or modified form thereof; and
   (2) a complementary sequence of the genomic sequence or cDNA sequence.

2. Use according to claim 1, wherein the CVB1 is a wild-type CVB1;
   preferably, the CVB1 is a clinical isolate isolated from an individual infected with Coxsackievirus B 1;
   preferably, the genomic sequence of the CVB1 or modified form thereof has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 12; more preferably, the genomic sequence of the CVB1 or modified form thereof is a nucleotide sequence as shown in SEQ ID NO: 12;
preferably, the cDNA sequence of the CVB1 or modified form thereof has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 1; more preferably, the cDNA sequence of the CVB1 or modified form thereof is a nucleotide sequence as shown in SEQ ID NO: 1.

**3.** Use according to claim 1 or 2, wherein the modified form is a modified CVB1 which has a substitution, insertion or deletion of one or more nucleotides in its genome as compared to a wild-type CVB1;
preferably, compared to the wild-type CVB1, the modified CVB1 has one or more modifications selected from the following:

(1) one or more mutations in an untranslated region (e.g. 5'UTR or 3'UTR);
(2) an insertion of one or more exogenous nucleic acids;
(3) a deletion or mutation of one or more endogenous genes; and
(4) any combination of the above three items.

**4.** Use according to claim 3, wherein the modified CVB1 comprises one or more mutations in a 5' untranslated region (5' UTR);
preferably, the modified CVB1 has a substitution of all or part of the 5'UTR sequence;
preferably, an internal ribosome entry site (IRES) sequence in the 5'UTR of the modified CVB1 is replaced with an exogenous IRES sequence, such as an internal ribosome entry site sequence of human rhinovirus 2 (HRV2);
preferably, the internal ribosome entry site sequence of human rhinovirus 2 (HRV2) is shown in SEQ ID NO: 2.

**5.** Use according to claim 3 or 4, wherein the modified CVB1 comprises an exogenous nucleic acid;
preferably, the exogenous nucleic acid encodes a cytokine (e.g., GM-CSF, preferably human GM-CSF), or an anti-tumor protein or polypeptide (e.g., scFV against PD-1 or PD-L1, preferably scFv against human PD- 1 or PD-L1);
preferably, the exogenous nucleic acid is inserted between 5'UTR and VP4 gene, or between VP1 gene and 2A gene of a genome of the modified CVB1;
preferably, the exogenous nucleic acid comprises a target sequence of one or more (e.g., 2, 3, or 4) microRNA;
preferably, the target sequence of microRNA is inserted in a 3' untranslated region (3'UTR) of a genome of the modified CVB1;
preferably, the exogenous nucleic acid comprises a target sequence of miR-133 and/or miR-206;
preferably, the target sequence of miR-133 is shown in SEQ ID NO: 3;
preferably, the target sequence of miR-206 is shown in SEQ ID NO:4.

**6.** Use according to any one of claims 3 to 5, wherein the modified CVB1 comprises at least one insertion of an exogenous nucleic acid as defined in claim 5 and/or at least one mutation in the untranslated region as defined in claim 4.

**7.** Use according to any one of claims 3 to 6, wherein the modified CVB1 has one of the following characteristics:

(1) the genomic sequence of the modified CVB1 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from the following: the nucleotide sequences as shown in SEQ ID NOs: 13-16; preferably the genomic sequence of the modified CVB1 is any one selected from the nucleotide sequences as shown in SEQ ID NOs: 13-16;
2) the cDNA sequence of the modified CVB1 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from the following: the nucleotide sequences as shown in SEQ ID NOs: 8-11; preferably, the cDNA sequence of the modified CVB1 is any one selected from the nucleotide sequences as shown in SEQ ID NOs: 8-11.

**8.** Use according to any one of claims 1 to 7, wherein the medicament comprises one or several of the CVB1 and modified form thereof.

**9.** Use according to any one of claims 1 to 8, wherein the nucleic acid molecule consists of the genomic sequence or cDNA sequence of the CVB1 or modified form thereof, or a complementary sequence of the genomic sequence or cDNA sequence;
preferably, the nucleic acid molecule has the genomic sequence of the CVB 1 or modified form thereof;
preferably, the nucleic acid molecule has a nucleotide sequence as shown in any one of SEQ ID NOs: 12-16.

**10.** Use according to any one of claims 1 to 8, wherein the nucleic acid molecule is a vector (e.g., a cloning vector or expression vector) comprising a genomic sequence or cDNA sequence of the CVB1 or modified form thereof, or a complementary sequence of the genomic sequence or cDNA sequence;
preferably, the nucleic acid molecule is a vector (e.g., a cloning vector or expression vector) comprising the cDNA sequence of the CVB1 or modified form thereof, or the complementary sequence of the cDNA sequence;
preferably, the nucleic acid molecule is a vector comprising a nucleotide sequence as shown in any one of SEQ ID NOs: 1, 8-11, or a complementary sequence thereof.

**11.** Use according to any one of claims 1 to 10, wherein the medicament further comprises an additional pharmaceutically active agent having anti-tumor activity, such as an additional oncolytic virus, chemotherapeutic agent or immunotherapeutic agent;
preferably, the additional oncolytic virus is selected from the group consisting of herpes virus, adenovirus, parvovirus, reovirus, Newcastle disease virus, vesicular stomatitis virus, measles virus or any combination thereof;
preferably, the chemotherapeutic agent is selected from the group consisting of 5-fluorouracil, mitomycin, methotrexate, hydroxyurea, cyclophosphamide, dacarbazine, mitoxantrone, anthracyclines (e.g., epirubicin or doxorubicin), etoposide, platinum compounds (e.g., carboplatin or cisplatin), taxanes (e.g., paclitaxel or docetaxel), or any combination thereof;
preferably, the immunotherapeutic agent is selected from the group consisting of immune checkpoint inhibitors (e.g., PD-L1/PD-1 inhibitors or CTLA-4 inhibitors), tumor-specific targeting antibodies (e.g., rituximab or herceptin), or any combination thereof.

**12.** Use according to any one of claims 1 to 11, which has at least one of the following characteristics:

(1) the tumor is selected from the group consisting of colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal cancer, thyroid cancer, thyroid ductal carcinoma and bladder cancer;
(2) the subject is a mammal, such as a human.

**13.** A method for treating a tumor, comprising a step of administering to a subject in need thereof an effective amount of a CVB1 or a modified form thereof, or an effective amount of a nucleic acid molecule; wherein the nucleic acid molecule comprises a sequence selected from the following:

(1) a genomic sequence or cDNA sequence of the CVB1 or modified form thereof; and
(2) a complementary sequence of the genomic sequence or cDNA sequence;
preferably, the CVB1 or modified form thereof, or the nucleic acid molecule is defined as in any one of claims 1 to 12;
preferably, one or more of the CVB1 and modified form thereof is administered to the subject;
preferably, the tumor is selected from the group consisting of colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal cancer, thyroid cancer, thyroid ductal carcinoma and bladder cancer;
preferably, the subject is a mammal, such as a human.

**14.** A pharmaceutical composition, comprising a CVB1 or a modified form thereof, or a nucleic acid molecule; and, a pharmaceutically acceptable carrier or excipient; wherein the nucleic acid molecule comprises a sequence selected from the following:

(1) a genomic sequence or cDNA sequence of the CVB1 or modified form thereof; and
(2) a complementary sequence of the genomic sequence or cDNA sequence;
preferably, the CVB1 or modified form thereof, or the nucleic acid molecule is defined as in any one of claims 1 to 12;
preferably, the pharmaceutical composition further comprises an additional pharmaceutically active agent having anti-tumor activity, such as an additional oncolytic virus, chemotherapeutic agent or immunotherapeutic agent;
preferably, the pharmaceutical composition is used for treating a tumor in a subject;
preferably, the tumor is selected from the group consisting of colorectal cancer, gastric cancer, lung cancer, liver cancer, ovarian cancer, endometrial cancer, cervical cancer, melanoma, breast cancer, kidney cancer, pancreatic cancer, lymphoma, osteogenic sarcoma, prostate cancer, glioma, neuroblastoma, tongue cancer, nasopharyngeal cancer, squamous cell carcinoma of nasal septum, pharyngeal squamous cell carcinoma, squamous cell carcinoma of submandibular gland, laryngeal cancer, thyroid cancer, thyroid ductal carcinoma and bladder cancer;
preferably, the subject is a mammal, such as a human.

**15.** A modified CVB1, which has a substitution of an internal ribosome entry site (IRES) sequence in a 5'UTR with an internal ribosome entry site sequence of human rhinovirus 2 (HRV2) as compared to a wild-type CVB1.

**16.** The modified CVB1 according to claim 15, wherein the modified CVB1 has at least one of the following characteristics:

1) the internal ribosome entry site sequence of human rhinovirus 2 (HRV2) is shown in SEQ ID NO: 2;
2) the wild-type CVB1 has a genomic sequence with a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 12; preferably, the genomic sequence of the wild-type CVB1 is a nucleotide sequence as shown in SEQ ID NO: 12;
3) the wild type CVB1 has a cDNA sequence with a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 1; preferably, the cDNA sequence of the wild-type CVB1 is a nucleotide sequence as shown in SEQ ID NO: 1;

**17.** The modified CVB1 according to claim 15 or 16, wherein the modified CVB1 further comprises an exogenous nucleic acid;
preferably, the exogenous nucleic acid encodes a cytokine (e.g., GM-CSF, preferably human GM-CSF), or an anti-tumor protein or polypeptide (e.g., scFv against PD-1 or PD-L1, preferably scFv against human PD- 1 or PD-L1);
preferably, the exogenous nucleic acid is inserted between 5'UTR and VP4 gene, or between VP1 gene and 2A gene of a genome of the modified CVB1;
preferably, the exogenous nucleic acid comprises a target sequence of one or more (e.g., 2, 3 or 4) microRNA;
preferably, the target sequence of microRNA is inserted in a 3' untranslated region (3'UTR) of a genome of the modified CVB1;
preferably, the exogenous nucleic acid comprises a target sequence of miR-133 and/or miR-206;
preferably, the target sequence of miR-133 is shown in SEQ ID NO: 3;
preferably, the target sequence of miR-206 is shown in SEQ ID NO:4.

**18.** The modified CVB1 according to any one of claims 15 to 17, wherein the modified CVB 1 has one of the following characteristics:

1) the modified CVB1 has a genomic sequence with a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 13; preferably the genomic sequence of the modified CVB1 is a nucleotide sequence as shown in SEQ ID NO: 13;
2) the modified CVB1 has a cDNA sequence with a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 8; preferably the cDNA sequence of the modified CVB1 is a nucleotide sequence as shown in SEQ ID NO: 8.

**19.** A nucleic acid molecule, comprising a sequence selected from the following:

(1) a genomic sequence or cDNA sequence of the modified CVB1 according to any one of claims 15 to 18; and
(2) a complementary sequence of the genomic sequence or cDNA sequence.

**20.** The nucleic acid molecule according to claim 19, wherein the nucleic acid molecule consists of the genomic sequence or cDNA sequence of the modified CVB1, or the complementary sequence of the genomic sequence or cDNA sequence;
preferably, the nucleic acid molecule has the genomic sequence of the modified CVB1;
preferably, the nucleic acid molecule has a nucleotide sequence as shown in SEQ ID NO: 13.

**21.** The nucleic acid molecule according to claim 19, wherein the nucleic acid molecule is a vector (e.g., a cloning vector or expression vector) comprising the genomic sequence or cDNA sequence of the modified CVB1, or the complement of the genomic sequence or cDNA sequence;
preferably, the nucleic acid molecule is a vector (e.g., a cloning vector or an expression vector) comprising the cDNA sequence of the modified CVB1, or the complementary sequence of the cDNA sequence;
preferably, the nucleic acid molecule is a vector comprising a nucleotide sequence as shown in SEQ ID NO: 8 or complementary sequence thereof.

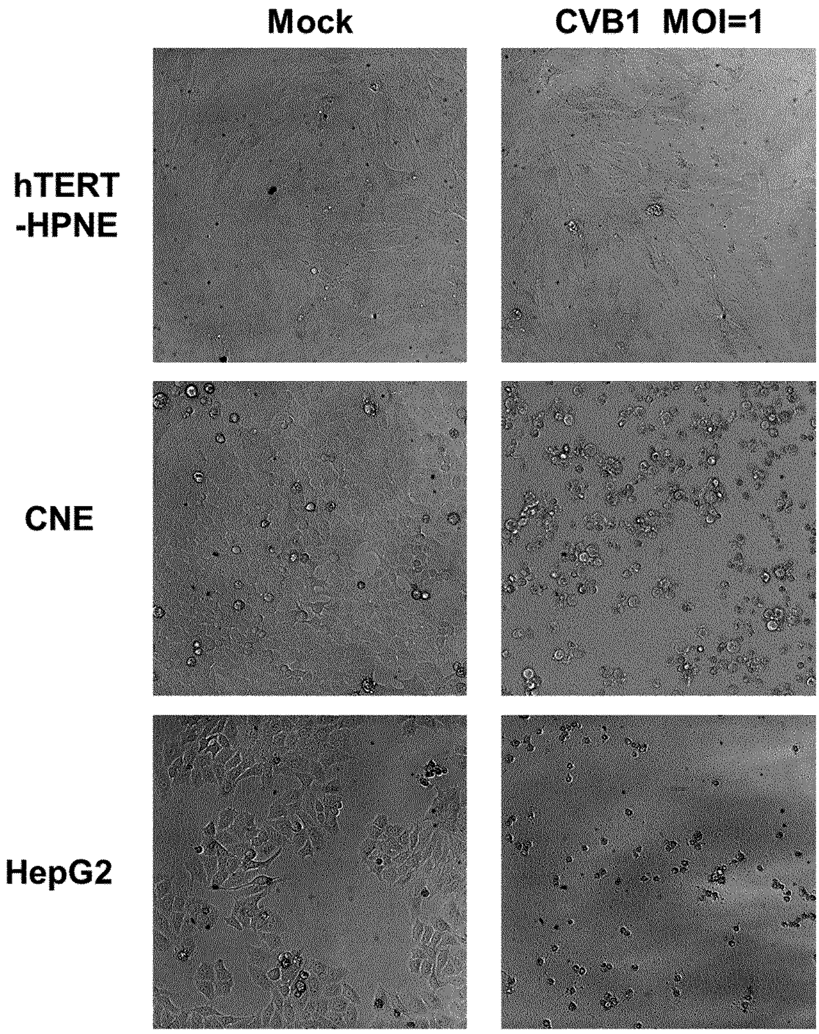


FIG. 1A

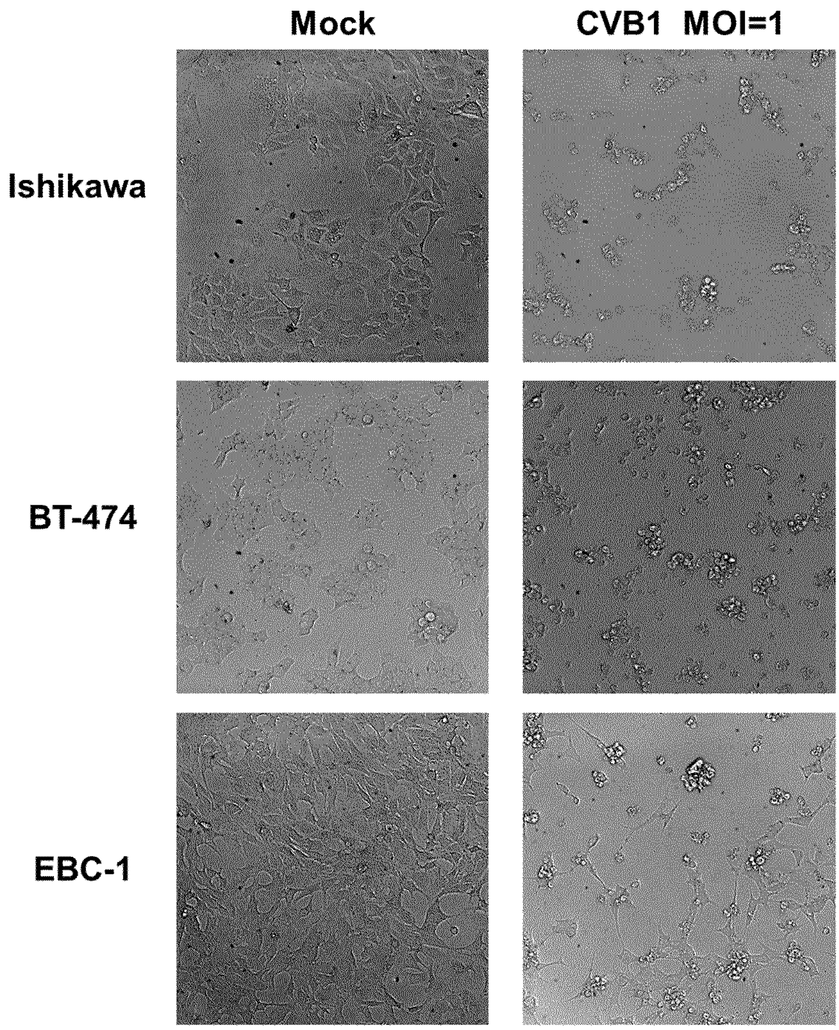


FIG. 1B

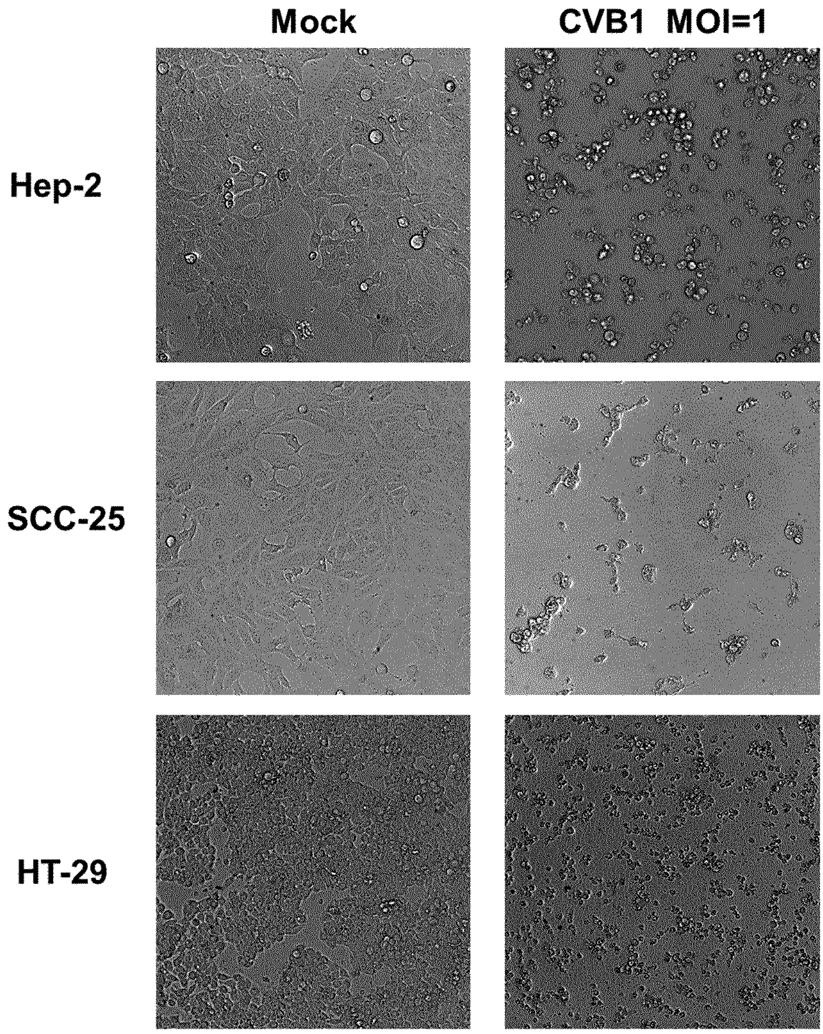


FIG. 1C

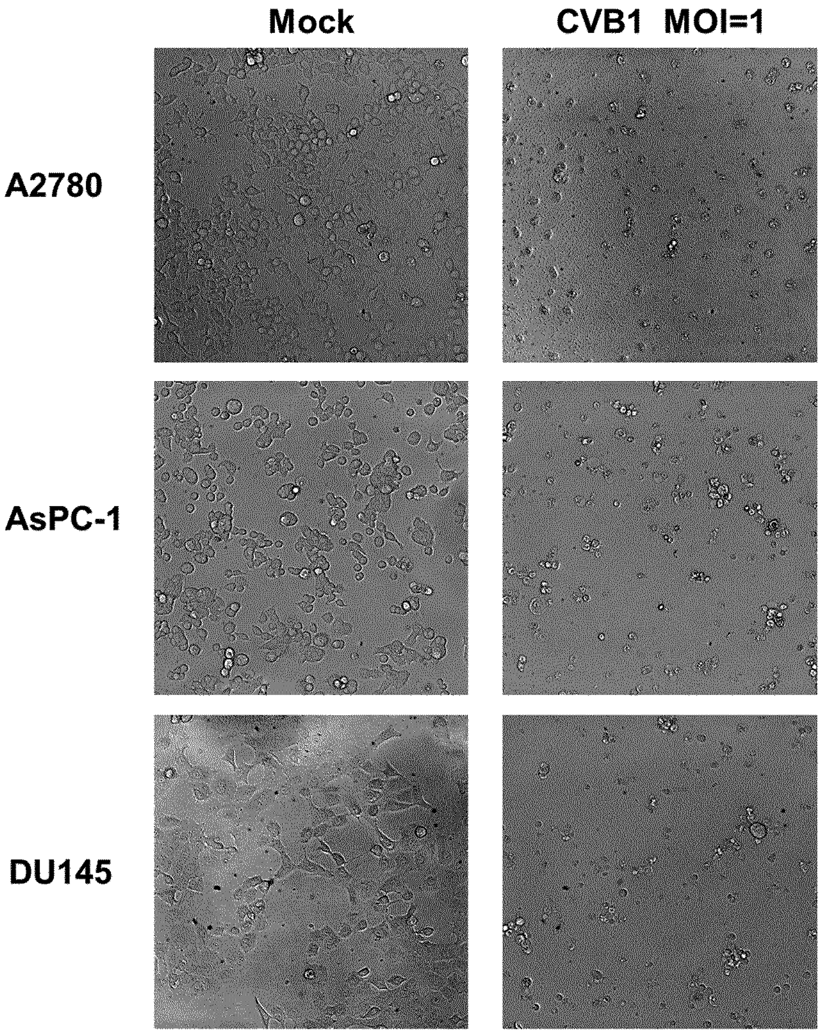


FIG. 1D

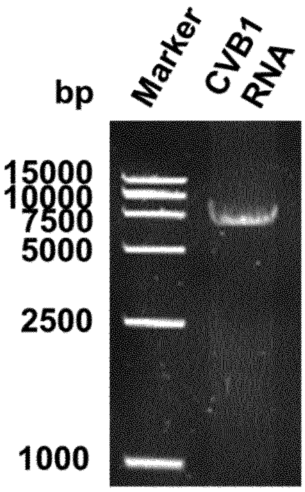
bp
Marker
CVB1 RNA
15000
10000
7500
5000
2500
1000

FIG. 2

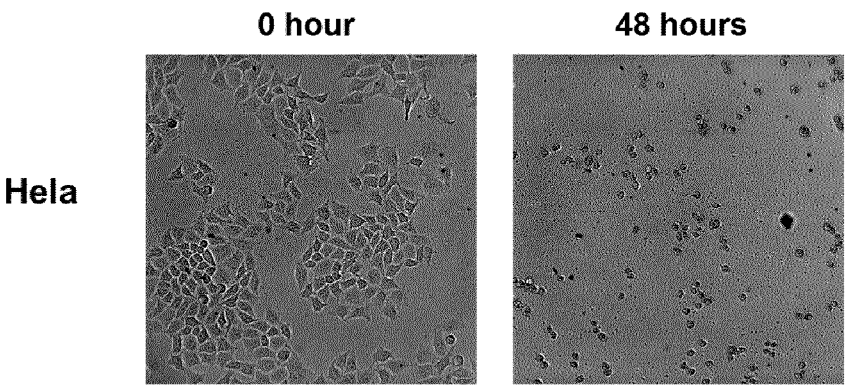
0 hour
48 hours
Hela

FIG. 3

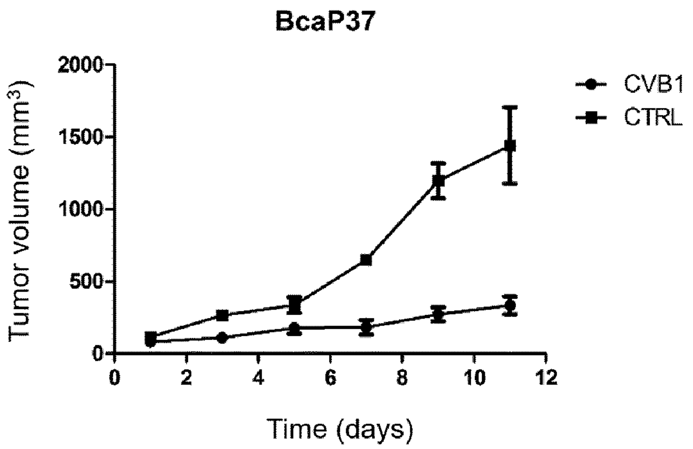


FIG. 4A

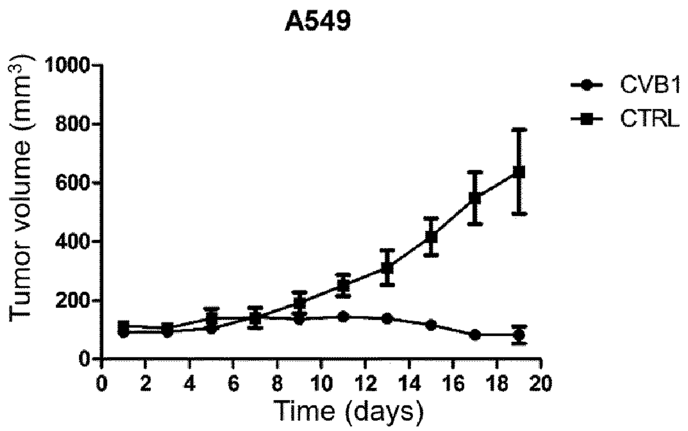


FIG. 4B

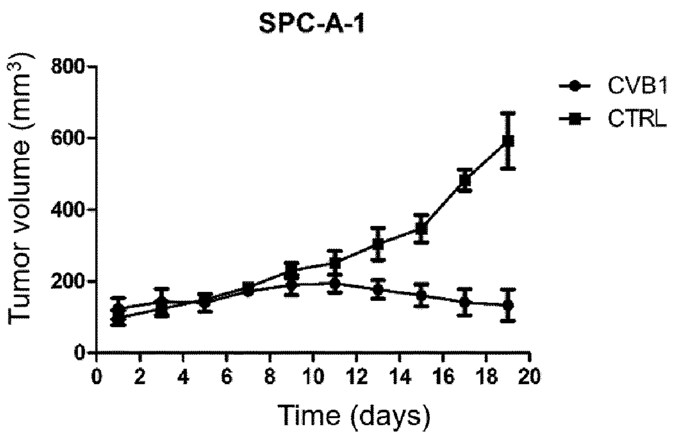


FIG. 4C

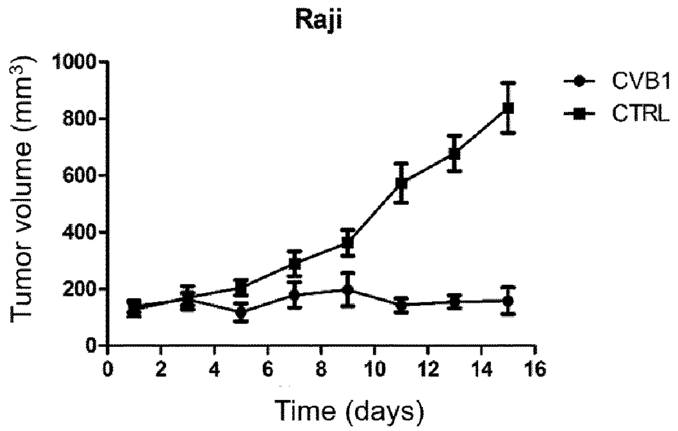


FIG. 4D

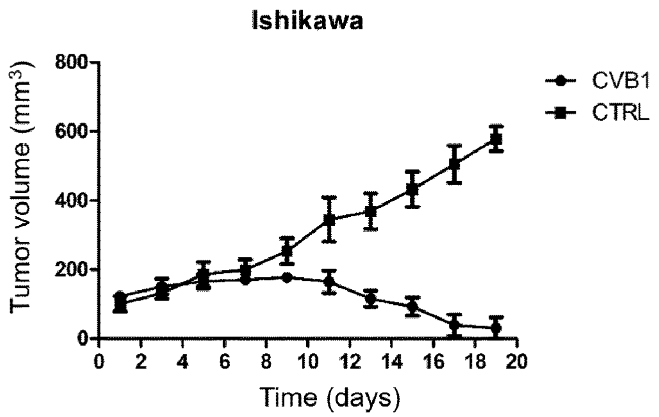


FIG. 4E

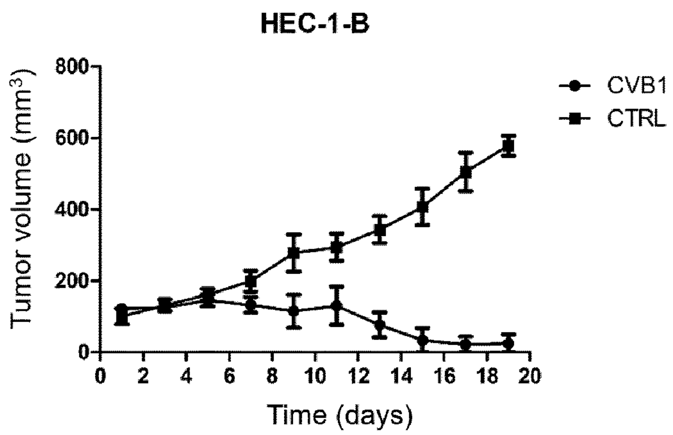


FIG. 4F

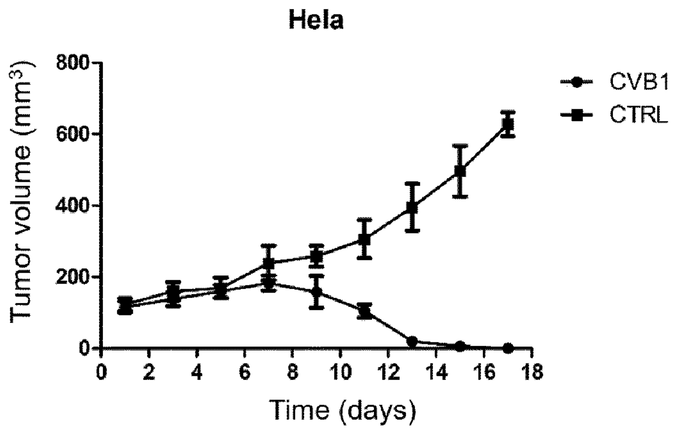


FIG. 4G

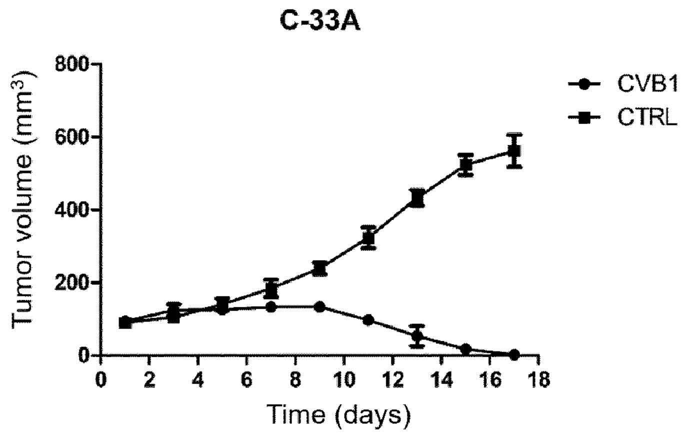


FIG. 4H

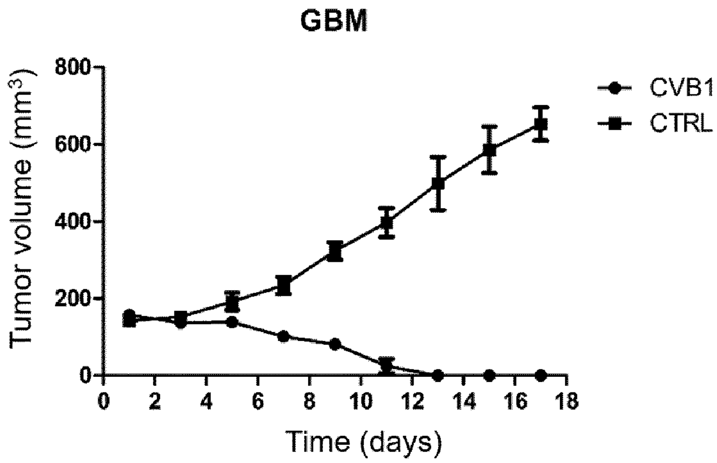


FIG. 4I

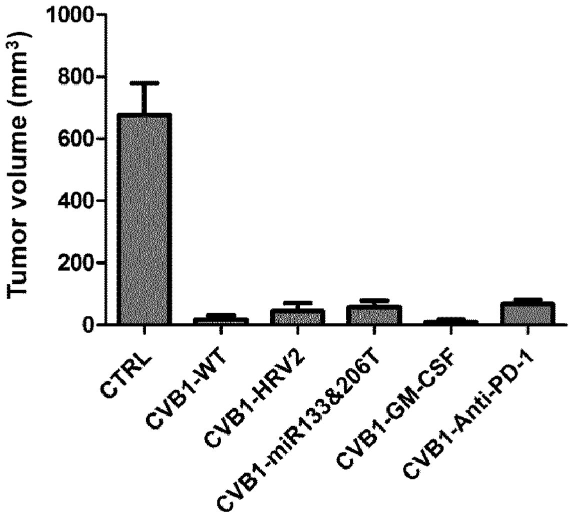
Tumor volume (mm3)
1000
800
600
400
200
0
CTRL
CVB1-WT
CVB1-HRV2
CVB1-miR133&206T
CVB1-GM-CSF
CVB1-Anti-PD-1

FIG. 5

## INTERNATIONAL SEARCH REPORT

International application No.
**PCT/CN2019/082608**

**A. CLASSIFICATION OF SUBJECT MATTER**

C12N 7/01(2006.01)i; A61P 35/00(2006.01)i; A61P 35/02(2006.01)i; A61P 35/04(2006.01)i

According to International Patent Classification (IPC) or to both national classification and IPC

**B. FIELDS SEARCHED**

Minimum documentation searched (classification system followed by classification symbols)

C12N; A61P

Documentation searched other than minimum documentation to the extent that such documents are included in the fields searched

Electronic data base consulted during the international search (name of data base and, where practicable, search terms used)

CNABS; CPRSABS; CNTXT; VEN; EPTXT; USTXT; WOTXT; CNKI; PUBMED; 百度学术, BAIDU XUESHU; GENBANK/EMBL/DDBJ; 中国专利序列数据库, China Patent Sequence Database: 柯萨奇病毒, 溶瘤病毒, 融瘤病毒, 肿瘤, 癌, Coxsackievirus, oncolytic, B1, 序列1和12

**C. DOCUMENTS CONSIDERED TO BE RELEVANT**

| Category* | Citation of document, with indication, where appropriate, of the relevant passages | Relevant to claim No. |
|---|---|---|
| PX | CN 109568350 A (XIAMEN UNIVERSITY; YANG SHENG TANG COMPANY LTD.) 05 April 2019 (2019-04-05)<br>see entire document | 1-12 (all in part), 14-21 |
| X | US 2018085411 A1 (SATOR THERAPEUTICS LLC) 29 March 2018 (2018-03-29)<br>see the abstract, description, paragraphs [0064]-[0070], and figures 1 and 2 | 1-12 (all in part), 14-21 |
| A | CN 103981152 A (WUHAN BOWEID BIOTECHNOLOGY CO., LTD.) 13 August 2014 (2014-08-13)<br>see entire document | 1-12 (all in part), 14-21 |
| A | CN 101065144 A (VIRALYTICS LIMITED) 31 October 2007 (2007-10-31)<br>see entire document | 1-12 (all in part), 14-21 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 July 2019** | **24 July 2019** |
| Name and mailing address of the ISA/CN<br>**National Intellectual Property Administration, PRC (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No. **PCT/CN2019/082608**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2019/082608**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-12**（均为部分）、**13**
because they relate to subject matter not required to be searched by this Authority, namely:

[1] Claims 1-12 (all in part) set forth the use of treating tumors in subjects, claim 13 sets forth a method of treating humor. The above subject matter relates to a method of treatment of the human or animal body by therapy and therefore does not warrant an international search according to the criteria set out in PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2019/082608**

| Patent document cited in search report | | | Publication date (day/month/year) | Patent family member(s) | | | Publication date (day/month/year) |
|---|---|---|---|---|---|---|---|
| CN | 109568350 | A | 05 April 2019 | None | | | |
| US | 2018085411 | A1 | 29 March 2018 | AU | 2017335673 | A1 | 11 April 2019 |
| | | | | CA | 3037573 | A1 | 05 April 2018 |
| | | | | WO | 2018064134 | A1 | 05 April 2018 |
| CN | 103981152 | A | 13 August 2014 | CN | 103981152 | B | 21 January 2015 |
| CN | 101065144 | A | 31 October 2007 | ZA | 200702269 | A | 25 September 2008 |
| | | | | ZA | 200702269 | B | 25 September 2008 |
| | | | | CN | 101065144 | B | 13 June 2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008103755 A1
- US 20160143969 A1


### Non-patent literature cited in the description


- **DOBRIKOVA et al.** *Mol Ther,* 2008, vol. 16 (11), 1865-1872
- **SHAFREN et al.** *Int J Cancer,* 2005, vol. 115 (2), 320-328
- **HALEY et al.** *J Mol Med (Berl),* 2009, vol. 87 (4), 385-399
- **RINEHART et al.** *J Virol,* 1997, vol. 71 (5), 3986-3991
- **RUIZ AJ ; RUSSELL S J.** MicroRNAs and oncolytic viruses. *J]. Curr Opin Virol,* 2015, vol. 13, 40-48
- **ARDOLINO M ; HSU J ; RAULET D H.** Cytokine treatment in cancer immunotherapy. *J]. Oncotarget,* 2015, vol. 6 (23), 19346-19347
- **CANDOLFI M ; KING GD ; MUHAMMAD AG et al.** Evaluation of proapototic transgenes to use in combination with Flt3L in an immune-stimulatory gene therapy approach for Glioblastoma multiforme (GBM). *J]. FASEB J,* 2008, vol. 22, 1077
- **NOLAN E ; SAVAS P ; POLICHENI AN et al.** Combined immune checkpoint blockade as a therapeutic strategy for BRCA1-mutated breast cancer. *J]. Science Trans Med,* 2017, vol. 9, eaal4922
- **ROSCA EV ; KOSKIMAKI JE ; RIVERA CG et al.** Anti-angiogenic peptides for cancer therapeutics. *J]. Curr Pharm Biotechnol,* 2011, vol. 12 (8), 1101-1116
- **BIRD et al.** *Science,* 1988, vol. 242, 423-426
- **HUSTON et al.** *Proc. Natl. Acad. Sci. USA,* 1988, vol. 85, 5879-5883
- **PLUCKTHUN.** The Pharmacology of Monoclonal Antibodies. Springer-Verlag, 1994, vol. 113, 269-315
- **NEEDLEMAN et al.** *J. Mol. Biol.,* 1970, vol. 48, 443-453
- **E. MEYERS ; W. MILLER.** *Comput. Appl. Biosci.,* 1988, vol. 4, 11-17
- **NEEDLEMAN ; WUNSCH.** *J. Mol. Biol.,* 1970, vol. 48, 444-453
- Remington's Pharmaceutical Sciences. Mack Publishing Company, 1995
- **MERRILL et al.** *J Virol,* 2006, vol. 80 (7), 3147-3156
- **MERRILL ; GROMEIER.** *J Virol,* 2006, vol. 80 (14), 6936-6942
- **NEPLIOUEVA et al.** *PLoS One,* 2010, vol. 5 (7), e11710
- **DOBRIKOV et al.** *Mol Cell Biol,* 2011, vol. 31 (14), 2947-2959
- **DOBRIKOV et al.** *Mol Cell Biol,* 2013, vol. 33 (5), 937-946
- **BAOHONG ZHANG et al.** *Developmental Biology,* 01 February 2007, vol. 302 (1), 1-12
- **YANG LS ; LI SX ; LIU YJ et al.** *Virus Res,* 2015, vol. 210, 165-168
- **HOU WH ; YANG LS ; LI SX et al.** *Virus Res,* 2015, vol. 205, 41-44
- **YIN H et al.** *Nat Rev Genet.,* August 2014, vol. 15 (8), 541-55
- **RILEY MK.** *Vermerris W. Nanomaterials (Basel),* 28 April 2017, vol. 7 (5), E94
- **J. SAMBROOK et al.** Molecular Cloning: A Laboratory Manual. Cold Spring Harbor Laboratory Press, 1989
- **FM AUSUBEL et al.** Short Protocols in Molecular Biology. John Wiley & Sons, Inc, 1995
- **HOU et al.** *Virus Res,* 2015, vol. 205, 41-44
- **YANG et al.** *Clin Vaccine Immunol,* 2014, vol. 21 (3), 312-320
- **HOU et al.** *Human, Virus Res,* 2015, vol. 205, 41-44
- **HADAC E M ; KELLY E J ; RUSSELL S.** *J. Mol Ther,* 2011, vol. 19 (6), 1041-1047